# EUROPEAN PATENT APPLICATION

(11) **EP 3 012 269 A1**
(43) Date of publication of application: **27.04.2016**
(21) Application number: 14189520.1
(22) Date of filing: 20.10.2014
(51) Int. Cl.: C07K 14/78

(54) **Cytokine-like 1 (CYTL1) protein and uses thereof**

(71) Applicant: Hofer, Erhard, 1090 Vienna (AT)
(72) Inventor: HOFER, Erhard, 1230 Vienna (AT); HOFER-WARBINEK, Renate, 1230 Vienna (AT); SCHNELLER, Doris, 69126 Heidelberg (DE); STURTZEL, Caterina, 1170 Vienna (AT)
(74) Representative: Sonn & Partner Patentanwälte

(57) **Abstract**

The present invention provides an isolated CYTL1 protein,
wherein the protein sequence comprises two consecutive threonine residues (possibly replaced by other glycosylatable amino-acid residues), and wherein the first of said residues is glycosylated and/or wherein the second of said residues is glycosylated, especially both; and
wherein the protein induces sprouting in a spheroid-based in-vitro angiogenesis assay compared to an uninduced control.

This protein is especially used in therapy, preferably therapy of myocardial infarction or prevention or therapy of ischemia or tissue damage, and as a marker for endothelial colony forming cells (ECFCs). Furthermore, the present invention provides anti-CYTL1 antibodies for use in cancer therapy.

SEQUENCE LISTING

<110> Hofer Erhard

<120> Cytokine-like 1 and uses thereof

<130> R65765

<160> 83

<170> BiSSAP 1.3

<210> 1
<211> 136
<212> PRT
<213> Homo sapiens

<400> 1
Met Arg Thr Pro Gly Pro Leu Pro Val Leu Leu Leu Leu Leu Ala Gly
1               5                   10                  15
Ala Pro Ala Ala Arg Pro Thr Pro Pro Thr Cys Tyr Ser Arg Met Arg
            20                  25                  30
Ala Leu Ser Gln Glu Ile Thr Arg Asp Phe Asn Leu Leu Gln Val Ser
        35                  40                  45
Glu Pro Ser Glu Pro Cys Val Arg Tyr Leu Pro Arg Leu Tyr Leu Asp
    50                  55                  60
Ile His Asn Tyr Cys Val Leu Asp Lys Leu Arg Asp Phe Val Ala Ser
65                  70                  75                  80
Pro Pro Cys Trp Lys Val Ala Gln Val Asp Ser Leu Lys Asp Lys Ala
                85                  90                  95
Arg Lys Leu Tyr Thr Ile Met Asn Ser Phe Cys Arg Arg Asp Leu Val
            100                 105                 110
Phe Leu Leu Asp Asp Cys Asn Ala Leu Glu Tyr Pro Ile Pro Val Thr
        115                 120                 125
Thr Val Leu Pro Asp Arg Gln Arg
    130                 135

<210> 2
<211> 136
<212> PRT
<213> Nomascus leucogenys

<400> 2
Met Arg Thr Pro Gly Pro Leu Pro Val Leu Leu Leu Leu Leu Ala Gly
1               5                   10                  15
Ala Pro Ala Val Arg Pro Ala Pro Pro Thr Cys Tyr Ser Arg Met Arg
            20                  25                  30
Gly Leu Ser Gln Glu Ile Thr Arg Asp Phe Gln Leu Leu Gln Val Ser
        35                  40                  45
Glu Pro Ser Glu Pro Cys Val Arg Tyr Leu Pro Arg Leu Tyr Leu Asp
    50                  55                  60
Ile His Asn Tyr Cys Val Leu Asp Lys Leu Gln Asp Phe Val Ala Ser
65                  70                  75                  80
Pro Gln Cys Trp Lys Val Ala Gln Val Asp Ser Leu Lys Asp Lys Ala
                85                  90                  95
Arg Lys Leu Tyr Thr Ile Met Asn Ser Phe Cys Arg Arg Asp Leu Val
            100                 105                 110
Phe Leu Leu Asp Asp Cys Asn Ala Leu Glu Tyr Pro Ile Pro Val Thr
        115                 120                 125
Thr Val Leu Pro Asp His Gln Arg
    130                 135

<210> 3

<211> 136
<212> PRT
<213> Macaca mulatta

<400> 3
Met Arg Thr Pro Gly Pro Leu Pro Val Leu Leu Leu Leu Leu Ala Gly
1               5                   10                  15
Ile Pro Ala Ala Arg Pro Thr Pro Pro Thr Cys Tyr Ser Arg Met Arg
            20                  25                  30
Ala Leu Ser Gln Glu Ile Thr Arg Asp Phe Gln Leu Leu Gln Ser Ser
        35                  40                  45
Glu Pro Ser Glu Pro Cys Val Arg Tyr Leu Pro Arg Leu Tyr Leu Asp
    50                  55                  60
Ile His Asn Tyr Cys Val Leu Asp Lys Leu Arg Asp Phe Val Ala Ser
65                  70                  75                  80
Pro Gln Cys Trp Lys Ala Ala Gln Val Asp Ser Leu Lys Asp Lys Ala
                85                  90                  95
Arg Lys Leu Tyr Thr Ile Met Asn Ser Phe Cys Arg Arg Asp Leu Val
            100                 105                 110
Phe Leu Leu Asp Asp Cys Asn Ala Leu Glu Tyr Pro Ile Pro Val Thr
        115                 120                 125
Thr Val Leu Pro Asp His Gln Arg
    130                 135

<210> 4
<211> 136
<212> PRT
<213> Pan troglodytes

<400> 4
Met Arg Met Pro Gly Pro Leu Pro Val Leu Leu Leu Leu Leu Ala Gly
1               5                   10                  15
Ala Pro Ala Ala Arg Pro Thr Pro Pro Thr Cys Tyr Ser Arg Met Arg
            20                  25                  30
Ala Leu Ser Gln Glu Ile Thr Arg Asp Phe Gln Leu Leu Gln Val Ser
        35                  40                  45
Glu Pro Ser Glu Pro Cys Val Arg Tyr Leu Pro Arg Leu Tyr Leu Asp
    50                  55                  60
Ile His Asn Tyr Cys Val Leu Asp Lys Leu Arg Asp Phe Val Ala Ser
65                  70                  75                  80
Pro Pro Cys Trp Lys Val Ala Gln Val Asp Ser Leu Lys Asp Lys Ala
                85                  90                  95
Arg Lys Leu Tyr Thr Ile Met Asn Ser Phe Cys Arg Arg Asp Leu Val
            100                 105                 110
Phe Leu Leu Asp Asp Cys Asn Ala Leu Glu Tyr Pro Ile Pro Val Thr
        115                 120                 125
Thr Val Leu Pro Asp Arg Gln Arg
    130                 135

<210> 5
<211> 136
<212> PRT
<213> Gorilla gorilla gorilla

<400> 5
Met Arg Thr Pro Gly Pro Leu Pro Val Leu Leu Leu Leu Leu Ala Gly
1               5                   10                  15
Ala Pro Ala Ala Arg Pro Thr Pro Pro Thr Cys Tyr Ser Arg Met Arg
            20                  25                  30
Ala Leu Ser Gln Glu Ile Thr Arg Asp Phe Gln Leu Leu Gln Val Ser
        35                  40                  45
Glu Pro Ser Glu Pro Cys Val Arg Tyr Leu Pro Arg Leu Tyr Leu Asp
    50                  55                  60

Ile His Asn Tyr Cys Val Leu Asp Lys Leu Arg Asp Phe Val Ala Ser
65                  70                  75                  80
Pro Pro Cys Trp Lys Val Ala Gln Val Asp Ser Leu Lys Asp Lys Ala
                85                  90                  95
Arg Lys Leu Tyr Thr Ile Met Asn Ser Phe Cys Arg Arg Asp Leu Val
            100                 105                 110
Phe Leu Leu Asp Asp Cys Asn Ala Leu Glu Tyr Pro Ile Pro Val Thr
        115                 120                 125
Thr Val Leu Pro Asp Arg Gln Arg
    130                 135

<210> 6
<211> 136
<212> PRT
<213> Pongo abelii

<400> 6
Met Arg Thr Pro Gly Pro Leu Pro Val Leu Leu Leu Leu Leu Ala Gly
1               5                   10                  15
Ile Pro Ala Ala Arg Pro Thr Pro Pro Thr Cys Tyr Ser Arg Met Arg
            20                  25                  30
Ala Leu Ser Gln Glu Ile Thr Arg Asp Phe Gln Leu Leu Gln Val Ser
        35                  40                  45
Glu Pro Ser Glu Pro Cys Val Arg Tyr Leu Pro Arg Leu Tyr Leu Asp
    50                  55                  60
Ile His Asn Tyr Cys Val Leu Asp Lys Leu Arg Asp Phe Val Ala Ser
65                  70                  75                  80
Pro Pro Cys Trp Lys Val Ala Gln Val Asp Ser Leu Lys Asp Lys Ala
                85                  90                  95
Arg Lys Leu Tyr Thr Ile Met Asn Ser Phe Cys Arg Arg Asp Leu Val
            100                 105                 110
Phe Leu Leu Asp Asp Cys Asn Ala Leu Glu Tyr Pro Ile Pro Val Thr
        115                 120                 125
Thr Val Leu Pro Asp Arg Gln Arg
    130                 135

<210> 7
<211> 136
<212> PRT
<213> Macaca mulatta

<400> 7
Met Arg Thr Pro Gly Pro Leu Pro Val Leu Leu Leu Leu Leu Ala Gly
1               5                   10                  15
Ile Pro Ala Ala Arg Pro Thr Pro Pro Thr Cys Tyr Ser Arg Met Arg
            20                  25                  30
Ala Leu Ser Gln Glu Ile Thr Arg Asp Phe Gln Leu Leu Gln Ser Ser
        35                  40                  45
Glu Pro Ser Glu Pro Cys Val Arg Tyr Leu Pro Arg Leu Tyr Leu Asp
    50                  55                  60
Ile His Asn Tyr Cys Val Leu Asp Lys Leu Arg Asp Phe Val Ala Ser
65                  70                  75                  80
Pro Gln Cys Trp Lys Val Ala Gln Val Asp Ser Leu Lys Asp Lys Ala
                85                  90                  95
Arg Lys Leu Tyr Thr Ile Met Asn Ser Phe Cys Arg Arg Asp Leu Val
            100                 105                 110
Phe Leu Leu Asp Asp Cys Asn Ala Leu Glu Tyr Pro Ile Pro Val Thr
        115                 120                 125
Thr Val Leu Pro Asp His Gln Arg
    130                 135

<210> 8
<211> 136

<212> PRT
<213> Macaca fascicularis

<400> 8
Met Arg Thr Pro Gly Pro Leu Pro Val Leu Leu Leu Leu Leu Ala Gly
1               5                   10                  15
Ile Pro Ala Ala Arg Pro Thr Pro Pro Thr Cys Tyr Ser Arg Met Arg
            20                  25                  30
Ala Leu Ser Gln Glu Ile Thr Arg Asp Phe Gln Leu Leu Gln Ser Ser
        35                  40                  45
Glu Pro Ser Glu Pro Cys Val Arg Tyr Leu Pro Arg Leu Tyr Leu Asp
    50                  55                  60
Ile His Asn Tyr Cys Val Leu Asp Lys Leu Arg Asp Phe Val Ala Ser
65                  70                  75                  80
Pro Gln Cys Trp Lys Val Ala Gln Val Asp Ser Leu Lys Asp Lys Ala
                85                  90                  95
Arg Lys Leu Tyr Thr Ile Met Asn Ser Phe Cys Arg Arg Asp Leu Val
            100                 105                 110
Phe Leu Leu Asp Asp Cys Asn Ala Leu Glu Tyr Pro Ile Pro Val Thr
        115                 120                 125
Thr Val Leu Pro Asp His Gln Arg
    130                 135

<210> 9
<211> 136
<212> PRT
<213> Myotis brandtii

<400> 9
Met Trp Pro Gln Asp Lys Ala Val Leu Cys Val Gly Ile Lys Ala Ala
1               5                   10                  15
Gly Thr Trp Ala Gly Leu Ala Arg Glu Gly Trp Gln Gly Ala Cys Gly
            20                  25                  30
Asp Arg Ala Glu Gly Val Ser Arg Gly His His Ser Asp Ala Ser Pro
        35                  40                  45
Phe Leu Gln Glu Leu Cys Val Met Tyr Leu Pro Arg Leu Tyr Leu Asp
    50                  55                  60
Ile His Asn Tyr Cys Val Leu Ala Lys Leu Arg Asp Phe Val Ala Ser
65                  70                  75                  80
Pro His Cys Trp Arg Val Ala Pro Val Asp Ala Leu Lys Asp Lys Val
                85                  90                  95
Arg Lys Leu Tyr Thr Ile Met Asn Ser Phe Cys Arg Arg Asp Leu Val
            100                 105                 110
Phe Leu Ser Asp Asp Cys Ser Ala Leu Asp Tyr Pro Ile Pro Ala Thr
        115                 120                 125
Thr Val Pro Pro Asp Pro Gln Gly
    130                 135

<210> 10
<211> 140
<212> PRT
<213> Sarcophilus harrisii

<400> 10
Ser Ser Asp Lys Arg Arg Thr Met Lys Leu Tyr Phe Ala Leu Met Val
1               5                   10                  15
Leu Leu Ser Ile Ser Leu Leu Ala Thr Ser Ala Pro Pro Thr Cys Tyr
            20                  25                  30
Ser Arg Met Leu Asn Leu Ser Lys Glu Ile Met Asp Ser Phe Asn Ala
        35                  40                  45
Leu Gln Asn Glu Glu Pro Leu Glu Pro Cys Val Glu Gly Leu Pro Lys
    50                  55                  60
Leu Tyr Leu Asp Ile His Asn Tyr Cys Val Leu Ala Lys Leu Arg Asp

65                  70                  75                  80
Phe Val Ala Ser Pro Lys Cys Arg Lys Val Ala Arg Val Gly Ala Leu
                85                  90                  95
Lys Asp Gln Val Arg Lys Leu Tyr Thr Ile Met Asn Ser Phe Cys Arg
            100                 105                 110
Arg Asp Leu Val Phe Leu Ser Asp Asp Cys Glu Val Leu Glu Asn Pro
        115                 120                 125
Leu Gln Ala Pro Thr Thr Val Arg Thr Gln Val Arg
    130                 135                 140

<210> 11
<211> 137
<212> PRT
<213> Rattus norvegicus

<400> 11
Met Ala Pro Lys Thr Leu Pro Leu Leu Leu Ser Leu Leu Leu Val Val
1               5                   10                  15
Trp Pro Leu Thr Val Gln Pro Ala Pro Pro Thr Cys Tyr Ser Arg Met
            20                  25                  30
Leu Thr Leu Ser Arg Glu Ile Met Ala Asp Phe Gln Ser Leu Gln Ala
        35                  40                  45
Ser Glu Pro Glu Asp Ser Cys Val Arg Tyr Leu Pro Arg Leu Tyr Leu
    50                  55                  60
Asp Ile His Asn Tyr Cys Val Leu Thr Lys Leu Arg Asp Phe Val Ala
65                  70                  75                  80
Ser Pro Gln Cys Trp Lys Met Ala Glu Val Asp Met Leu Lys Asp Arg
                85                  90                  95
Val Arg Lys Leu Tyr Thr Ile Met Asn Ser Phe Cys Arg Arg Asp Leu
            100                 105                 110
Val Phe Leu Ser Asp Asp Cys Ser Val Leu Glu His Pro Ile Pro Glu
        115                 120                 125
Thr Thr Asp Pro Pro Asp Trp Gln Ser
    130                 135

<210> 12
<211> 125
<212> PRT
<213> Neovison vison

<400> 12
Leu Leu Leu Ala Gly Pro Pro Ala Ala Gln Pro Ala Pro Pro Thr Cys
1               5                   10                  15
Tyr Ser Arg Met Leu Ser Leu Ser Arg Glu Val Thr Gly Asp Phe Gln
            20                  25                  30
Ser Leu Gln Ala Val Glu Pro Ser Glu Pro Cys Val Arg Tyr Leu Pro
        35                  40                  45
Thr Leu Tyr Leu Asp Ile His Asn Tyr Cys Val Leu Ala Lys Leu Arg
    50                  55                  60
Asp Phe Val Ala Ser Pro Gln Cys Trp Lys Val Ala Gln Val Asp Ala
65                  70                  75                  80
Leu Lys Asp Lys Val Arg Lys Leu Tyr Ile Ile Met Asn Ser Phe Cys
                85                  90                  95
Arg Arg Asp Leu Val Phe Leu Ser Asp Asp Cys Asn Ala Leu Glu Tyr
            100                 105                 110
Pro Ile Pro Val Thr Thr Val Leu Pro Asp Gly Gln Arg
        115                 120                 125

<210> 13
<211> 138
<212> PRT
<213> Loxodonta africana

<400> 13
Leu Ser Thr Met Thr Leu Trp Pro Leu Ser Leu Leu Leu Leu Leu Leu
1               5                   10                  15
Val Gly Pro Pro Ala Thr Gln Pro Thr Pro Pro Thr Cys Tyr Ser Arg
            20                  25                  30
Met Leu Ser Leu Ser Arg Glu Ile Val Gly Asp Phe Gln Ser Leu Gln
        35                  40                  45
Ala Thr Glu Pro Ser Glu Pro Cys Val Arg Tyr Leu Pro Arg Leu Tyr
    50                  55                  60
Leu Asp Ile His Asn Tyr Cys Val Leu Ala Lys Leu Arg Asp Phe Val
65                  70                  75                  80
Ala Ser Pro Gln Cys Trp Lys Val Ala Gln Val Asp Ala Leu Lys Asp
                85                  90                  95
Lys Val Arg Lys Leu Tyr Thr Ile Met Asn Ser Phe Cys Arg Arg Asp
            100                 105                 110
Leu Val Phe Leu Ser Asp Asp Cys Asn Ala Leu Glu Tyr Pro Ile Pro
        115                 120                 125
Val Thr Thr Ala Leu Pro Asp Arg Gln Met
    130                 135

<210> 14
<211> 135
<212> PRT
<213> Canis lupus familiaris

<400> 14
Met Met Pro Arg Leu Leu Cys Leu Leu Leu Leu Leu Leu Ala Gly Ser
1               5                   10                  15
Pro Thr Ala Arg Pro Ala Pro Pro Thr Cys Tyr Ser Arg Met Leu Ser
            20                  25                  30
Leu Ser Arg Glu Val Thr Gly Asp Phe Gln Asn Leu Gln Ala Thr Glu
        35                  40                  45
Pro Ser Glu Pro Cys Val Arg Tyr Leu Pro Arg Leu Tyr Leu Asp Ile
    50                  55                  60
His Asn His Cys Val Leu Ala Lys Leu Arg Asp Phe Val Ala Ser Pro
65                  70                  75                  80
Gln Cys Trp Lys Val Ala Gln Val Asp Gly Leu Lys Asp Lys Val Arg
                85                  90                  95
Lys Leu Tyr Thr Ile Met Asn Ser Phe Cys Arg Arg Asp Leu Val Phe
            100                 105                 110
Leu Ser Asp Asp Cys Asn Ala Leu Glu Tyr Pro Ile Pro Val Thr Thr
        115                 120                 125
Ile Leu Pro Asp Arg Gln Arg
    130                 135

<210> 15
<211> 134
<212> PRT
<213> Otolemur garnettii

<400> 15
Val Arg Thr Val Met Leu Pro Leu Leu Leu Leu Leu Leu Ala Val Pro
1               5                   10                  15
Pro Ala Trp Pro Ala Pro Pro Thr Cys Tyr Ser Arg Met Leu Ser Leu
            20                  25                  30
Ser Arg Glu Val Thr Asn Asp Phe Gln Arg Leu Leu Ala Ala Glu Leu
        35                  40                  45
Gln Glu Pro Cys Val Arg His Leu Pro Arg Leu Tyr Leu Asp Ile His
    50                  55                  60
Asn Tyr Cys Val Leu Ala Lys Leu Arg Asp Phe Val Ala Ser Pro Gln
65                  70                  75                  80
Cys Trp Lys Met Ala Gln Ala Asp Ala Leu Lys Glu Lys Val Arg Lys
                85                  90                  95

Leu Tyr Thr Ile Thr Asn Val Tyr Cys Arg Arg Asp Leu Val Phe Leu
            100                 105                 110
Ser Asp Asp Cys Ser Ala Leu Glu Tyr Pro Ile Pro Val Thr Thr Ile
        115                 120                 125
Leu Pro Asp Arg Gln Arg
    130

<210> 16
<211> 135
<212> PRT
<213> Myotis davidii

<400> 16
Met Arg Pro Trp Leu Leu Pro Leu Leu Leu Leu Leu Leu Ala Gly Pro
1               5                   10                  15
Pro Thr Val Trp Pro Ala Pro Pro Thr Cys Tyr Ser Arg Met Leu Gly
            20                  25                  30
Leu Ser Leu Glu Ile Ser Arg Asp Phe Gln Arg Leu Gln Ala Met Glu
        35                  40                  45
Pro Ser Glu Leu Cys Val Thr Tyr Leu Pro Arg Leu Tyr Leu Asp Ile
    50                  55                  60
His Asn Tyr Cys Val Leu Ala Lys Leu Arg Asp Phe Val Ala Ser Pro
65                  70                  75                  80
His Cys Trp Arg Val Ala Pro Val Asp Ala Leu Lys Asp Lys Val Arg
                85                  90                  95
Lys Leu Tyr Thr Ile Met Asn Ser Phe Cys Arg Arg Asp Leu Val Phe
            100                 105                 110
Leu Ser Asp Asp Cys Ser Ala Leu Asp Tyr Pro Ile Pro Ala Thr Thr
        115                 120                 125
Val Pro Pro Asp Pro Gln Gly
    130                 135

<210> 17
<211> 137
<212> PRT
<213> Ovis aries

<400> 17
Thr Met Thr Pro Gln Leu Phe Pro Leu Leu Leu Leu Leu Leu Leu Ala
1               5                   10                  15
Gly Leu Pro Ala Thr Arg Pro Ala Pro Pro Thr Cys Tyr Ser Arg Met
            20                  25                  30
Leu Ala Leu Ser Arg Glu Ile Thr Ser Asp Phe Arg Ser Leu Gln Ala
        35                  40                  45
Thr Glu Pro Ser Glu Pro Cys Val Arg Tyr Leu Pro Arg Leu Tyr Leu
    50                  55                  60
Asp Ile His Asn Tyr Cys Val Leu Ala Lys Leu Arg Asp Phe Val Ala
65                  70                  75                  80
Ser Pro Gln Cys Trp Lys Val Ala Gln Val Asp Ala Leu Lys Asp Lys
                85                  90                  95
Val Arg Lys Leu Tyr Thr Ile Met Asn Ser Phe Cys Arg Arg Asp Leu
            100                 105                 110
Val Phe Leu Ser Asp Asp Cys Asn Ala Leu Glu Tyr Pro Ile Leu Val
        115                 120                 125
Thr Thr Val Leu Pro Asp His Gln Ser
    130                 135

<210> 18
<211> 137
<212> PRT
<213> Bos taurus

<400> 18

Met Thr Ala Gln Val Phe Pro Leu Leu Leu Leu Leu Leu Leu Leu Ala
1               5                   10                  15
Gly Leu Pro Thr Thr Arg Pro Thr Pro Pro Thr Cys Tyr Ser Arg Met
            20                  25                  30
Leu Ala Leu Ser Arg Glu Ile Thr Ser Asp Phe Gln Ser Leu Gln Ala
        35                  40                  45
Thr Glu Pro Ser Glu Ala Cys Val Arg Tyr Leu Pro Arg Leu Tyr Leu
    50                  55                  60
Asp Ile His Asn Tyr Cys Val Leu Ala Lys Leu Arg Asp Phe Val Ala
65                  70                  75                  80
Ser Pro His Cys Trp Lys Leu Ala Gln Val Asp Ala Leu Lys Asp Lys
                85                  90                  95
Val Arg Lys Leu Tyr Thr Ile Met Asn Ser Phe Cys Arg Arg Asp Leu
            100                 105                 110
Val Phe Leu Thr Asp Asp Cys Asn Ala Leu Glu Tyr Pro Val Leu Val
        115                 120                 125
Thr Thr Val Leu Pro Asp His Gln Ser
    130                 135

<210> 19
<211> 137
<212> PRT
<213> Bos mutus

<400> 19
Met Thr Pro Gln Val Phe Pro Leu Leu Leu Leu Leu Leu Leu Leu Ala
1               5                   10                  15
Gly Leu Pro Thr Thr Arg Pro Thr Pro Pro Thr Cys Tyr Ser Arg Met
            20                  25                  30
Leu Ala Leu Ser Arg Glu Ile Thr Ser Asp Phe Gln Ser Leu Gln Ala
        35                  40                  45
Thr Glu Pro Ser Glu Ala Cys Val Arg Tyr Leu Pro Arg Leu Tyr Leu
    50                  55                  60
Asp Ile His Asn Tyr Cys Val Leu Ala Lys Leu Arg Asp Phe Val Ala
65                  70                  75                  80
Ser Pro His Cys Trp Lys Leu Ala Gln Val Asp Ala Leu Lys Asp Lys
                85                  90                  95
Val Arg Lys Leu Tyr Thr Ile Met Asn Ser Phe Cys Arg Arg Asp Leu
            100                 105                 110
Val Phe Leu Thr Asp Asp Cys Asn Ala Leu Glu Tyr Pro Val Leu Val
        115                 120                 125
Thr Thr Val Leu Pro Asp His Gln Ser
    130                 135

<210> 20
<211> 123
<212> PRT
<213> Cricetulus griseus

<400> 20
Met Ala Pro Arg Ala Leu Pro Leu Leu Leu Pro Leu Leu Met Val Val
1               5                   10                  15
Leu Trp Pro Leu Val Val Glu Pro Ala Pro Pro Thr Cys Tyr Ser Arg
            20                  25                  30
Met Leu Thr Leu Ser His Glu Ile Met Ala Asp Phe Gln Ser Leu Gln
        35                  40                  45
Ala Ser Glu Pro Glu Asn Tyr Cys Val Leu Ala Lys Leu Arg Asp Phe
    50                  55                  60
Val Ala Ser Pro Gln Cys Trp Lys Met Ala Glu Val Asp Thr Leu Lys
65                  70                  75                  80
Asp Arg Val Arg Lys Leu Tyr Thr Ile Met Asn Ser Phe Cys Arg Arg
                85                  90                  95
Asp Leu Val Phe Leu Ser Asp Asp Cys Asn Ala Leu Glu Tyr Pro Ile

100                 105                 110
Pro Glu Thr Thr Gly Pro Pro Asp Trp Gln Ser
        115                 120

<210> 21
<211> 136
<212> PRT
<213> Felis catus

<400> 21
Thr Met Thr Pro Gly Leu Leu Pro Leu Leu Leu Leu Leu Leu Ala Gly
1               5                   10                  15
Pro Pro Ala Ala Gln Pro Ala Pro Pro Thr Cys Tyr Ser Arg Met Leu
            20                  25                  30
Ser Leu Ser Arg Glu Val Thr Gly Asp Ala Gln Arg Leu Gln Ala Met
        35                  40                  45
Glu Pro Ser Glu Ala Cys Val Arg Tyr Leu Pro Arg Leu His Leu Asp
    50                  55                  60
Ile His Asn Tyr Cys Val Leu Ala Lys Leu Arg Asp Phe Val Ala Ser
65                  70                  75                  80
Pro Gln Cys Trp Lys Val Ala Gln Val Asp Ala Leu Lys Asp Lys Val
                85                  90                  95
Arg Lys Leu Tyr Thr Ile Met Asn Ser Phe Cys Arg Arg Asp Leu Val
            100                 105                 110
Phe Leu Ser Asp Asp Cys Asp Ala Leu Glu Tyr Pro Val Pro Val Thr
        115                 120                 125
Thr Val Leu Pro Gly Arg Gln Arg
    130                 135

<210> 22
<211> 135
<212> PRT
<213> Mustela putorius furo

<400> 22
Met Arg Ser Arg Leu Leu Pro Val Leu Leu Leu Leu Leu Ala Gly Pro
1               5                   10                  15
Pro Thr Ala Gln Pro Ala Pro Pro Thr Cys Tyr Ser Arg Met Leu Ser
            20                  25                  30
Leu Ser Arg Glu Val Thr Gly Asp Phe Gln Ser Leu Gln Ala Val Glu
        35                  40                  45
Pro Ser Glu Pro Cys Val Arg Tyr Leu Pro Thr Leu Tyr Leu Asp Ile
    50                  55                  60
His Asn Tyr Cys Val Leu Ala Lys Leu Arg Asp Phe Val Ala Ser Thr
65                  70                  75                  80
Gln Cys Trp Lys Val Ala Gln Val Asp Ala Leu Lys Asp Lys Val Arg
                85                  90                  95
Lys Leu Tyr Ile Ile Met Asn Ser Phe Cys Arg Arg Asp Leu Val Phe
            100                 105                 110
Leu Ser Asp Asp Cys Asn Ala Leu Glu Tyr Pro Ile Pro Val Thr Thr
        115                 120                 125
Val Leu Pro Asp Gly Gln Arg
    130                 135

<210> 23
<211> 134
<212> PRT
<213> Ailuropoda melanoleuca

<400> 23
Thr Pro Arg Leu Leu Pro Leu Leu Leu Leu Leu Leu Ala Gly Pro Pro
1               5                   10                  15
Ser Ala Gln Pro Ala Pro Pro Thr Cys Tyr Ser Arg Met Leu Thr Leu

                20                  25                  30
Ser Arg Glu Val Ala Gly Asp Phe Gln Ser Leu Gln Ala Ala Glu Pro
        35                  40                  45
Ser Glu Pro Cys Val Arg Tyr Leu Pro Thr Leu Tyr Leu Asp Ile His
    50                  55                  60
Asn Tyr Cys Val Leu Ala Arg Leu Arg Asp Phe Val Ala Ser Pro Gln
65                  70                  75                  80
Cys Trp Lys Val Ala Gln Val Asp Ala Leu Lys Asp Lys Val Arg Lys
                85                  90                  95
Leu Tyr Thr Ile Met Asn Ser Phe Cys Arg Arg Asp Leu Val Phe Leu
            100                 105                 110
Ser Asp Asp Cys Asn Ala Leu Glu Tyr Pro Ile Pro Val Thr Thr Val
        115                 120                 125
Leu Pro Asp Gly Gln Arg
    130

<210> 24
<211> 136
<212> PRT
<213> Ailuropoda melanoleuca

<400> 24
Ala Met Thr Pro Arg Leu Leu Pro Leu Leu Leu Leu Leu Leu Ala Gly
1               5                   10                  15
Pro Pro Ser Ala Gln Pro Ala Pro Pro Thr Cys Tyr Ser Arg Met Leu
            20                  25                  30
Thr Leu Ser Arg Glu Val Ala Gly Asp Phe Gln Ser Leu Gln Ala Ala
        35                  40                  45
Glu Pro Ser Glu Pro Cys Val Arg Tyr Leu Pro Thr Leu Tyr Leu Asp
    50                  55                  60
Ile His Asn Tyr Cys Val Leu Ala Arg Leu Arg Asp Phe Val Ala Ser
65                  70                  75                  80
Pro Gln Cys Trp Lys Val Ala Gln Val Asp Ala Leu Lys Asp Lys Val
                85                  90                  95
Arg Lys Leu Tyr Thr Ile Met Asn Ser Phe Cys Arg Arg Asp Leu Val
            100                 105                 110
Phe Leu Ser Asp Asp Cys Asn Ala Leu Glu Tyr Pro Ile Pro Val Thr
        115                 120                 125
Thr Val Leu Pro Asp Gly Gln Arg
    130                 135

<210> 25
<211> 137
<212> PRT
<213> Oryctolagus cuniculus

<400> 25
Met Thr Arg Trp Pro Leu Pro Leu Leu Leu Leu Leu Leu Ala Gly Pro
1               5                   10                  15
Pro Pro Ala Ala Pro Ala Pro Pro Thr Cys Tyr Ser Arg Leu Arg Ala
            20                  25                  30
Leu Ser Arg Glu Leu Thr Arg Asp Phe Gln Ser Leu Gln Asp Glu Glu
        35                  40                  45
Pro Ser Glu Pro Cys Val Lys Tyr Leu Pro Ala Leu Tyr Leu Asp Ile
    50                  55                  60
His Asn Tyr Cys Val Leu Ala Lys Leu Arg Asp Phe Val Ala Ser Pro
65                  70                  75                  80
Gln Cys Trp His Val Ala Arg Val Asp Ala Leu Lys Asp Gln Val Arg
                85                  90                  95
Lys Leu Tyr Thr Leu Met Asn Ser Phe Cys Arg Arg Asp Leu Val Phe
            100                 105                 110
Leu Ser Asp Asp Cys Ser Ala Leu Glu Tyr Pro Ser Pro Gly Thr Thr
        115                 120                 125

Asp Leu Pro Gly Leu His Ala Arg Glu
    130                 135

<210> 26
<211> 106
<212> PRT
<213> Heterocephalus glaber

<400> 26
Met Leu Gly Leu Ser Arg Glu Ile Thr Gly Asp Phe Gln Leu Leu Gln
1               5                   10                  15
Ala Ala Glu Pro Ala Glu Ala Cys Val Arg Tyr Leu Pro Arg Leu Tyr
            20                  25                  30
Leu Asp Ile His Asn Tyr Cys Val Leu Ala Gln Leu Arg Asp Phe Val
        35                  40                  45
Ala Ser Pro Gln Cys Trp Lys Val Ala Gln Val Asp Ala Leu Arg Asp
    50                  55                  60
Lys Val Arg Lys Leu Tyr Thr Ile Met Asn Ser Phe Cys Arg Arg Asp
65                  70                  75                  80
Leu Val Phe Leu Ser Asp Asp Cys Thr Ala Leu Glu Tyr Pro Ile Pro
                85                  90                  95
Val Thr Thr Ser Leu Pro Asp His Gln Ser
            100                 105

<210> 27
<211> 134
<212> PRT
<213> Ictidomys tridecemlineatus

<400> 27
Met Met Pro Trp Thr Ser Leu Leu Leu Leu Leu Leu Ala Gly Leu Pro
1               5                   10                  15
Ala Ala Arg Pro Ala Pro Pro Thr Cys Tyr Ser Arg Met Leu Thr Leu
            20                  25                  30
Ser Arg Glu Ile Thr Ser Glu Phe Gln Gly Leu Gln Ala Ala Glu Pro
        35                  40                  45
Gly Glu Pro Cys Val Arg Tyr Leu Pro Gly Leu His Leu Asp Ile His
    50                  55                  60
Asn Tyr Cys Val Leu Ala Lys Leu Arg Asp Phe Val Ala Ser Pro His
65                  70                  75                  80
Cys Trp Lys Met Ala Gln Val Asp Ala Leu Lys Asp Lys Val Arg Lys
                85                  90                  95
Leu Tyr Thr Ile Met Asn Ser Phe Cys Arg Arg Asp Leu Val Phe Leu
            100                 105                 110
Ser Asp Asp Cys Ser Ala Leu Glu His Pro Ile Pro Val Thr Thr Gly
        115                 120                 125
Pro Pro Glu Gly Gln Ser
    130

<210> 28
<211> 114
<212> PRT
<213> Homo sapiens

<400> 28
Thr Pro Pro Thr Cys Tyr Ser Arg Met Arg Ala Leu Ser Gln Glu Ile
1               5                   10                  15
Thr Arg Asp Phe Asn Leu Leu Gln Val Ser Glu Pro Ser Glu Pro Cys
            20                  25                  30
Val Arg Tyr Leu Pro Arg Leu Tyr Leu Asp Ile His Asn Tyr Cys Val
        35                  40                  45
Leu Asp Lys Leu Arg Asp Phe Val Ala Ser Pro Pro Cys Trp Lys Val
    50                  55                  60

Ala Gln Val Asp Ser Leu Lys Asp Lys Ala Arg Lys Leu Tyr Thr Ile
65                  70                  75                  80
Met Asn Ser Phe Cys Arg Arg Asp Leu Val Phe Leu Leu Asp Asp Cys
                85                  90                  95
Asn Ala Leu Glu Tyr Pro Ile Pro Val Thr Thr Val Leu Pro Asp Arg
            100                 105                 110
Gln Arg



<210> 29
<211> 114
<212> PRT
<213> Nomascus leucogenys

<400> 29
Ala Pro Pro Thr Cys Tyr Ser Arg Met Arg Gly Leu Ser Gln Glu Ile
1               5                   10                  15
Thr Arg Asp Phe Gln Leu Leu Gln Val Ser Glu Pro Ser Glu Pro Cys
            20                  25                  30
Val Arg Tyr Leu Pro Arg Leu Tyr Leu Asp Ile His Asn Tyr Cys Val
        35                  40                  45
Leu Asp Lys Leu Gln Asp Phe Val Ala Ser Pro Gln Cys Trp Lys Val
    50                  55                  60
Ala Gln Val Asp Ser Leu Lys Asp Lys Ala Arg Lys Leu Tyr Thr Ile
65                  70                  75                  80
Met Asn Ser Phe Cys Arg Arg Asp Leu Val Phe Leu Leu Asp Asp Cys
                85                  90                  95
Asn Ala Leu Glu Tyr Pro Ile Pro Val Thr Thr Val Leu Pro Asp His
            100                 105                 110
Gln Arg



<210> 30
<211> 114
<212> PRT
<213> Macaca mulatta

<400> 30
Thr Pro Pro Thr Cys Tyr Ser Arg Met Arg Ala Leu Ser Gln Glu Ile
1               5                   10                  15
Thr Arg Asp Phe Gln Leu Leu Gln Ser Ser Glu Pro Ser Glu Pro Cys
            20                  25                  30
Val Arg Tyr Leu Pro Arg Leu Tyr Leu Asp Ile His Asn Tyr Cys Val
        35                  40                  45
Leu Asp Lys Leu Arg Asp Phe Val Ala Ser Pro Gln Cys Trp Lys Ala
    50                  55                  60
Ala Gln Val Asp Ser Leu Lys Asp Lys Ala Arg Lys Leu Tyr Thr Ile
65                  70                  75                  80
Met Asn Ser Phe Cys Arg Arg Asp Leu Val Phe Leu Leu Asp Asp Cys
                85                  90                  95
Asn Ala Leu Glu Tyr Pro Ile Pro Val Thr Thr Val Leu Pro Asp His
            100                 105                 110
Gln Arg



<210> 31
<211> 114
<212> PRT
<213> Pan troglodytes

<400> 31
Thr Pro Pro Thr Cys Tyr Ser Arg Met Arg Ala Leu Ser Gln Glu Ile
1               5                   10                  15

Thr Arg Asp Phe Gln Leu Leu Gln Val Ser Glu Pro Ser Glu Pro Cys
            20                  25                  30
Val Arg Tyr Leu Pro Arg Leu Tyr Leu Asp Ile His Asn Tyr Cys Val
        35                  40                  45
Leu Asp Lys Leu Arg Asp Phe Val Ala Ser Pro Pro Cys Trp Lys Val
    50                  55                  60
Ala Gln Val Asp Ser Leu Lys Asp Lys Ala Arg Lys Leu Tyr Thr Ile
65                  70                  75                  80
Met Asn Ser Phe Cys Arg Arg Asp Leu Val Phe Leu Leu Asp Asp Cys
                85                  90                  95
Asn Ala Leu Glu Tyr Pro Ile Pro Val Thr Thr Val Leu Pro Asp Arg
            100                 105                 110
Gln Arg

<210> 32
<211> 114
<212> PRT
<213> Gorilla gorilla gorilla

<400> 32
Thr Pro Pro Thr Cys Tyr Ser Arg Met Arg Ala Leu Ser Gln Glu Ile
1               5                   10                  15
Thr Arg Asp Phe Gln Leu Leu Gln Val Ser Glu Pro Ser Glu Pro Cys
            20                  25                  30
Val Arg Tyr Leu Pro Arg Leu Tyr Leu Asp Ile His Asn Tyr Cys Val
        35                  40                  45
Leu Asp Lys Leu Arg Asp Phe Val Ala Ser Pro Pro Cys Trp Lys Val
    50                  55                  60
Ala Gln Val Asp Ser Leu Lys Asp Lys Ala Arg Lys Leu Tyr Thr Ile
65                  70                  75                  80
Met Asn Ser Phe Cys Arg Arg Asp Leu Val Phe Leu Leu Asp Asp Cys
                85                  90                  95
Asn Ala Leu Glu Tyr Pro Ile Pro Val Thr Thr Val Leu Pro Asp Arg
            100                 105                 110
Gln Arg

<210> 33
<211> 114
<212> PRT
<213> Pongo abelii

<400> 33
Thr Pro Pro Thr Cys Tyr Ser Arg Met Arg Ala Leu Ser Gln Glu Ile
1               5                   10                  15
Thr Arg Asp Phe Gln Leu Leu Gln Val Ser Glu Pro Ser Glu Pro Cys
            20                  25                  30
Val Arg Tyr Leu Pro Arg Leu Tyr Leu Asp Ile His Asn Tyr Cys Val
        35                  40                  45
Leu Asp Lys Leu Arg Asp Phe Val Ala Ser Pro Pro Cys Trp Lys Val
    50                  55                  60
Ala Gln Val Asp Ser Leu Lys Asp Lys Ala Arg Lys Leu Tyr Thr Ile
65                  70                  75                  80
Met Asn Ser Phe Cys Arg Arg Asp Leu Val Phe Leu Leu Asp Asp Cys
                85                  90                  95
Asn Ala Leu Glu Tyr Pro Ile Pro Val Thr Thr Val Leu Pro Asp Arg
            100                 105                 110
Gln Arg

<210> 34
<211> 114

<212> PRT
<213> Macaca mulatta

<400> 34
Thr Pro Pro Thr Cys Tyr Ser Arg Met Arg Ala Leu Ser Gln Glu Ile
1               5                   10                  15
Thr Arg Asp Phe Gln Leu Leu Gln Ser Ser Glu Pro Ser Glu Pro Cys
            20                  25                  30
Val Arg Tyr Leu Pro Arg Leu Tyr Leu Asp Ile His Asn Tyr Cys Val
        35                  40                  45
Leu Asp Lys Leu Arg Asp Phe Val Ala Ser Pro Gln Cys Trp Lys Val
    50                  55                  60
Ala Gln Val Asp Ser Leu Lys Asp Lys Ala Arg Lys Leu Tyr Thr Ile
65                  70                  75                  80
Met Asn Ser Phe Cys Arg Arg Asp Leu Val Phe Leu Leu Asp Asp Cys
                85                  90                  95
Asn Ala Leu Glu Tyr Pro Ile Pro Val Thr Thr Val Leu Pro Asp His
            100                 105                 110
Gln Arg


<210> 35
<211> 114
<212> PRT
<213> Macaca fascicularis

<400> 35
Thr Pro Pro Thr Cys Tyr Ser Arg Met Arg Ala Leu Ser Gln Glu Ile
1               5                   10                  15
Thr Arg Asp Phe Gln Leu Leu Gln Ser Ser Glu Pro Ser Glu Pro Cys
            20                  25                  30
Val Arg Tyr Leu Pro Arg Leu Tyr Leu Asp Ile His Asn Tyr Cys Val
        35                  40                  45
Leu Asp Lys Leu Arg Asp Phe Val Ala Ser Pro Gln Cys Trp Lys Val
    50                  55                  60
Ala Gln Val Asp Ser Leu Lys Asp Lys Ala Arg Lys Leu Tyr Thr Ile
65                  70                  75                  80
Met Asn Ser Phe Cys Arg Arg Asp Leu Val Phe Leu Leu Asp Asp Cys
                85                  90                  95
Asn Ala Leu Glu Tyr Pro Ile Pro Val Thr Thr Val Leu Pro Asp His
            100                 105                 110
Gln Arg


<210> 36
<211> 110
<212> PRT
<213> Myotis brandtii

<400> 36
Trp Gln Gly Ala Cys Gly Asp Arg Ala Glu Gly Val Ser Arg Gly His
1               5                   10                  15
His Ser Asp Ala Ser Pro Phe Leu Gln Glu Leu Cys Val Met Tyr Leu
            20                  25                  30
Pro Arg Leu Tyr Leu Asp Ile His Asn Tyr Cys Val Leu Ala Lys Leu
        35                  40                  45
Arg Asp Phe Val Ala Ser Pro His Cys Trp Arg Val Ala Pro Val Asp
    50                  55                  60
Ala Leu Lys Asp Lys Val Arg Lys Leu Tyr Thr Ile Met Asn Ser Phe
65                  70                  75                  80
Cys Arg Arg Asp Leu Val Phe Leu Ser Asp Asp Cys Ser Ala Leu Asp
                85                  90                  95
Tyr Pro Ile Pro Ala Thr Thr Val Pro Pro Asp Pro Gln Gly

            100                 105                 110

<210> 37
<211> 114
<212> PRT
<213> Sarcophilus harrisii

<400> 37
Ala Pro Pro Thr Cys Tyr Ser Arg Met Leu Asn Leu Ser Lys Glu Ile
1               5                   10                  15
Met Asp Ser Phe Asn Ala Leu Gln Asn Glu Glu Pro Leu Glu Pro Cys
            20                  25                  30
Val Glu Gly Leu Pro Lys Leu Tyr Leu Asp Ile His Asn Tyr Cys Val
        35                  40                  45
Leu Ala Lys Leu Arg Asp Phe Val Ala Ser Pro Lys Cys Arg Lys Val
    50                  55                  60
Ala Arg Val Gly Ala Leu Lys Asp Gln Val Arg Lys Leu Tyr Thr Ile
65                  70                  75                  80
Met Asn Ser Phe Cys Arg Arg Asp Leu Val Phe Leu Ser Asp Asp Cys
                85                  90                  95
Glu Val Leu Glu Asn Pro Leu Gln Ala Pro Thr Thr Val Arg Thr Gln
            100                 105                 110
Val Arg

<210> 38
<211> 114
<212> PRT
<213> Rattus norvegicus

<400> 38
Ala Pro Pro Thr Cys Tyr Ser Arg Met Leu Thr Leu Ser Arg Glu Ile
1               5                   10                  15
Met Ala Asp Phe Gln Ser Leu Gln Ala Ser Glu Pro Glu Asp Ser Cys
            20                  25                  30
Val Arg Tyr Leu Pro Arg Leu Tyr Leu Asp Ile His Asn Tyr Cys Val
        35                  40                  45
Leu Thr Lys Leu Arg Asp Phe Val Ala Ser Pro Gln Cys Trp Lys Met
    50                  55                  60
Ala Glu Val Asp Met Leu Lys Asp Arg Val Arg Lys Leu Tyr Thr Ile
65                  70                  75                  80
Met Asn Ser Phe Cys Arg Arg Asp Leu Val Phe Leu Ser Asp Asp Cys
                85                  90                  95
Ser Val Leu Glu His Pro Ile Pro Glu Thr Thr Asp Pro Pro Asp Trp
            100                 105                 110
Gln Ser

<210> 39
<211> 114
<212> PRT
<213> Neovison vison

<400> 39
Ala Pro Pro Thr Cys Tyr Ser Arg Met Leu Ser Leu Ser Arg Glu Val
1               5                   10                  15
Thr Gly Asp Phe Gln Ser Leu Gln Ala Val Glu Pro Ser Glu Pro Cys
            20                  25                  30
Val Arg Tyr Leu Pro Thr Leu Tyr Leu Asp Ile His Asn Tyr Cys Val
        35                  40                  45
Leu Ala Lys Leu Arg Asp Phe Val Ala Ser Pro Gln Cys Trp Lys Val
    50                  55                  60
Ala Gln Val Asp Ala Leu Lys Asp Lys Val Arg Lys Leu Tyr Ile Ile

65                  70                  75                  80
Met Asn Ser Phe Cys Arg Arg Asp Leu Val Phe Leu Ser Asp Asp Cys
                85                  90                  95
Asn Ala Leu Glu Tyr Pro Ile Pro Val Thr Thr Val Leu Pro Asp Gly
            100                 105                 110
Gln Arg



<210> 40
<211> 114
<212> PRT
<213> Loxodonta africana

<400> 40
Thr Pro Pro Thr Cys Tyr Ser Arg Met Leu Ser Leu Ser Arg Glu Ile
1               5                   10                  15
Val Gly Asp Phe Gln Ser Leu Gln Ala Thr Glu Pro Ser Glu Pro Cys
            20                  25                  30
Val Arg Tyr Leu Pro Arg Leu Tyr Leu Asp Ile His Asn Tyr Cys Val
        35                  40                  45
Leu Ala Lys Leu Arg Asp Phe Val Ala Ser Pro Gln Cys Trp Lys Val
    50                  55                  60
Ala Gln Val Asp Ala Leu Lys Asp Lys Val Arg Lys Leu Tyr Thr Ile
65                  70                  75                  80
Met Asn Ser Phe Cys Arg Arg Asp Leu Val Phe Leu Ser Asp Asp Cys
                85                  90                  95
Asn Ala Leu Glu Tyr Pro Ile Pro Val Thr Thr Ala Leu Pro Asp Arg
            100                 105                 110
Gln Met



<210> 41
<211> 114
<212> PRT
<213> Canis lupus familiaris

<400> 41
Ala Pro Pro Thr Cys Tyr Ser Arg Met Leu Ser Leu Ser Arg Glu Val
1               5                   10                  15
Thr Gly Asp Phe Gln Asn Leu Gln Ala Thr Glu Pro Ser Glu Pro Cys
            20                  25                  30
Val Arg Tyr Leu Pro Arg Leu Tyr Leu Asp Ile His Asn His Cys Val
        35                  40                  45
Leu Ala Lys Leu Arg Asp Phe Val Ala Ser Pro Gln Cys Trp Lys Val
    50                  55                  60
Ala Gln Val Asp Gly Leu Lys Asp Lys Val Arg Lys Leu Tyr Thr Ile
65                  70                  75                  80
Met Asn Ser Phe Cys Arg Arg Asp Leu Val Phe Leu Ser Asp Asp Cys
                85                  90                  95
Asn Ala Leu Glu Tyr Pro Ile Pro Val Thr Thr Ile Leu Pro Asp Arg
            100                 105                 110
Gln Arg



<210> 42
<211> 114
<212> PRT
<213> Otolemur garnettii

<400> 42
Ala Pro Pro Thr Cys Tyr Ser Arg Met Leu Ser Leu Ser Arg Glu Val
1               5                   10                  15
Thr Asn Asp Phe Gln Arg Leu Leu Ala Ala Glu Leu Gln Glu Pro Cys

                20                  25                  30
Val Arg His Leu Pro Arg Leu Tyr Leu Asp Ile His Asn Tyr Cys Val
        35                  40                  45
Leu Ala Lys Leu Arg Asp Phe Val Ala Ser Pro Gln Cys Trp Lys Met
    50                  55                  60
Ala Gln Ala Asp Ala Leu Lys Glu Lys Val Arg Lys Leu Tyr Thr Ile
65                  70                  75                  80
Thr Asn Val Tyr Cys Arg Arg Asp Leu Val Phe Leu Ser Asp Asp Cys
                85                  90                  95
Ser Ala Leu Glu Tyr Pro Ile Pro Val Thr Thr Ile Leu Pro Asp Arg
            100                 105                 110
Gln Arg


<210> 43
<211> 114
<212> PRT
<213> Myotis davidii

<400> 43
Ala Pro Pro Thr Cys Tyr Ser Arg Met Leu Gly Leu Ser Leu Glu Ile
1               5                   10                  15
Ser Arg Asp Phe Gln Arg Leu Gln Ala Met Glu Pro Ser Glu Leu Cys
            20                  25                  30
Val Thr Tyr Leu Pro Arg Leu Tyr Leu Asp Ile His Asn Tyr Cys Val
        35                  40                  45
Leu Ala Lys Leu Arg Asp Phe Val Ala Ser Pro His Cys Trp Arg Val
    50                  55                  60
Ala Pro Val Asp Ala Leu Lys Asp Lys Val Arg Lys Leu Tyr Thr Ile
65                  70                  75                  80
Met Asn Ser Phe Cys Arg Arg Asp Leu Val Phe Leu Ser Asp Asp Cys
                85                  90                  95
Ser Ala Leu Asp Tyr Pro Ile Pro Ala Thr Thr Val Pro Pro Asp Pro
            100                 105                 110
Gln Gly


<210> 44
<211> 114
<212> PRT
<213> Ovis aries

<400> 44
Ala Pro Pro Thr Cys Tyr Ser Arg Met Leu Ala Leu Ser Arg Glu Ile
1               5                   10                  15
Thr Ser Asp Phe Arg Ser Leu Gln Ala Thr Glu Pro Ser Glu Pro Cys
            20                  25                  30
Val Arg Tyr Leu Pro Arg Leu Tyr Leu Asp Ile His Asn Tyr Cys Val
        35                  40                  45
Leu Ala Lys Leu Arg Asp Phe Val Ala Ser Pro Gln Cys Trp Lys Val
    50                  55                  60
Ala Gln Val Asp Ala Leu Lys Asp Lys Val Arg Lys Leu Tyr Thr Ile
65                  70                  75                  80
Met Asn Ser Phe Cys Arg Arg Asp Leu Val Phe Leu Ser Asp Asp Cys
                85                  90                  95
Asn Ala Leu Glu Tyr Pro Ile Leu Val Thr Thr Val Leu Pro Asp His
            100                 105                 110
Gln Ser


<210> 45
<211> 114
<212> PRT

<213> Bos taurus

<400> 45
Thr Pro Pro Thr Cys Tyr Ser Arg Met Leu Ala Leu Ser Arg Glu Ile
1               5                   10                  15
Thr Ser Asp Phe Gln Ser Leu Gln Ala Thr Glu Pro Ser Glu Ala Cys
            20                  25                  30
Val Arg Tyr Leu Pro Arg Leu Tyr Leu Asp Ile His Asn Tyr Cys Val
        35                  40                  45
Leu Ala Lys Leu Arg Asp Phe Val Ala Ser Pro His Cys Trp Lys Leu
    50                  55                  60
Ala Gln Val Asp Ala Leu Lys Asp Lys Val Arg Lys Leu Tyr Thr Ile
65                  70                  75                  80
Met Asn Ser Phe Cys Arg Arg Asp Leu Val Phe Leu Thr Asp Asp Cys
                85                  90                  95
Asn Ala Leu Glu Tyr Pro Val Leu Val Thr Thr Val Leu Pro Asp His
            100                 105                 110
Gln Ser


<210> 46
<211> 114
<212> PRT
<213> Bos mutus

<400> 46
Thr Pro Pro Thr Cys Tyr Ser Arg Met Leu Ala Leu Ser Arg Glu Ile
1               5                   10                  15
Thr Ser Asp Phe Gln Ser Leu Gln Ala Thr Glu Pro Ser Glu Ala Cys
            20                  25                  30
Val Arg Tyr Leu Pro Arg Leu Tyr Leu Asp Ile His Asn Tyr Cys Val
        35                  40                  45
Leu Ala Lys Leu Arg Asp Phe Val Ala Ser Pro His Cys Trp Lys Leu
    50                  55                  60
Ala Gln Val Asp Ala Leu Lys Asp Lys Val Arg Lys Leu Tyr Thr Ile
65                  70                  75                  80
Met Asn Ser Phe Cys Arg Arg Asp Leu Val Phe Leu Thr Asp Asp Cys
                85                  90                  95
Asn Ala Leu Glu Tyr Pro Val Leu Val Thr Thr Val Leu Pro Asp His
            100                 105                 110
Gln Ser


<210> 47
<211> 99
<212> PRT
<213> Cricetulus griseus

<400> 47
Ala Pro Pro Thr Cys Tyr Ser Arg Met Leu Thr Leu Ser His Glu Ile
1               5                   10                  15
Met Ala Asp Phe Gln Ser Leu Gln Ala Ser Glu Pro Glu Asn Tyr Cys
            20                  25                  30
Val Leu Ala Lys Leu Arg Asp Phe Val Ala Ser Pro Gln Cys Trp Lys
        35                  40                  45
Met Ala Glu Val Asp Thr Leu Lys Asp Arg Val Arg Lys Leu Tyr Thr
    50                  55                  60
Ile Met Asn Ser Phe Cys Arg Arg Asp Leu Val Phe Leu Ser Asp Asp
65                  70                  75                  80
Cys Asn Ala Leu Glu Tyr Pro Ile Pro Glu Thr Thr Gly Pro Pro Asp
                85                  90                  95
Trp Gln Ser

<210> 48
<211> 114
<212> PRT
<213> Felis catus

<400> 48
Ala Pro Pro Thr Cys Tyr Ser Arg Met Leu Ser Leu Ser Arg Glu Val
1               5                   10                  15
Thr Gly Asp Ala Gln Arg Leu Gln Ala Met Glu Pro Ser Glu Ala Cys
            20                  25                  30
Val Arg Tyr Leu Pro Arg Leu His Leu Asp Ile His Asn Tyr Cys Val
        35                  40                  45
Leu Ala Lys Leu Arg Asp Phe Val Ala Ser Pro Gln Cys Trp Lys Val
    50                  55                  60
Ala Gln Val Asp Ala Leu Lys Asp Lys Val Arg Lys Leu Tyr Thr Ile
65                  70                  75                  80
Met Asn Ser Phe Cys Arg Arg Asp Leu Val Phe Leu Ser Asp Asp Cys
                85                  90                  95
Asp Ala Leu Glu Tyr Pro Val Pro Val Thr Thr Val Leu Pro Gly Arg
            100                 105                 110
Gln Arg


<210> 49
<211> 114
<212> PRT
<213> Mustela putorius furo

<400> 49
Ala Pro Pro Thr Cys Tyr Ser Arg Met Leu Ser Leu Ser Arg Glu Val
1               5                   10                  15
Thr Gly Asp Phe Gln Ser Leu Gln Ala Val Glu Pro Ser Glu Pro Cys
            20                  25                  30
Val Arg Tyr Leu Pro Thr Leu Tyr Leu Asp Ile His Asn Tyr Cys Val
        35                  40                  45
Leu Ala Lys Leu Arg Asp Phe Val Ala Ser Thr Gln Cys Trp Lys Val
    50                  55                  60
Ala Gln Val Asp Ala Leu Lys Asp Lys Val Arg Lys Leu Tyr Ile Ile
65                  70                  75                  80
Met Asn Ser Phe Cys Arg Arg Asp Leu Val Phe Leu Ser Asp Asp Cys
                85                  90                  95
Asn Ala Leu Glu Tyr Pro Ile Pro Val Thr Thr Val Leu Pro Asp Gly
            100                 105                 110
Gln Arg


<210> 50
<211> 114
<212> PRT
<213> Ailuropoda melanoleuca

<400> 50
Ala Pro Pro Thr Cys Tyr Ser Arg Met Leu Thr Leu Ser Arg Glu Val
1               5                   10                  15
Ala Gly Asp Phe Gln Ser Leu Gln Ala Ala Glu Pro Ser Glu Pro Cys
            20                  25                  30
Val Arg Tyr Leu Pro Thr Leu Tyr Leu Asp Ile His Asn Tyr Cys Val
        35                  40                  45
Leu Ala Arg Leu Arg Asp Phe Val Ala Ser Pro Gln Cys Trp Lys Val
    50                  55                  60
Ala Gln Val Asp Ala Leu Lys Asp Lys Val Arg Lys Leu Tyr Thr Ile
65                  70                  75                  80

Met Asn Ser Phe Cys Arg Arg Asp Leu Val Phe Leu Ser Asp Asp Cys
                85                  90                  95
Asn Ala Leu Glu Tyr Pro Ile Pro Val Thr Thr Val Leu Pro Asp Gly
            100                 105                 110
Gln Arg

<210> 51
<211> 114
<212> PRT
<213> Ailuropoda melanoleuca

<400> 51
Ala Pro Pro Thr Cys Tyr Ser Arg Met Leu Thr Leu Ser Arg Glu Val
1               5                   10                  15
Ala Gly Asp Phe Gln Ser Leu Gln Ala Ala Glu Pro Ser Glu Pro Cys
            20                  25                  30
Val Arg Tyr Leu Pro Thr Leu Tyr Leu Asp Ile His Asn Tyr Cys Val
        35                  40                  45
Leu Ala Arg Leu Arg Asp Phe Val Ala Ser Pro Gln Cys Trp Lys Val
    50                  55                  60
Ala Gln Val Asp Ala Leu Lys Asp Lys Val Arg Lys Leu Tyr Thr Ile
65                  70                  75                  80
Met Asn Ser Phe Cys Arg Arg Asp Leu Val Phe Leu Ser Asp Asp Cys
                85                  90                  95
Asn Ala Leu Glu Tyr Pro Ile Pro Val Thr Thr Val Leu Pro Asp Gly
            100                 105                 110
Gln Arg

<210> 52
<211> 116
<212> PRT
<213> Oryctolagus cuniculus

<400> 52
Ala Pro Pro Thr Cys Tyr Ser Arg Leu Arg Ala Leu Ser Arg Glu Leu
1               5                   10                  15
Thr Arg Asp Phe Gln Ser Leu Gln Asp Glu Glu Pro Ser Glu Pro Cys
            20                  25                  30
Val Lys Tyr Leu Pro Ala Leu Tyr Leu Asp Ile His Asn Tyr Cys Val
        35                  40                  45
Leu Ala Lys Leu Arg Asp Phe Val Ala Ser Pro Gln Cys Trp His Val
    50                  55                  60
Ala Arg Val Asp Ala Leu Lys Asp Gln Val Arg Lys Leu Tyr Thr Leu
65                  70                  75                  80
Met Asn Ser Phe Cys Arg Arg Asp Leu Val Phe Leu Ser Asp Asp Cys
                85                  90                  95
Ser Ala Leu Glu Tyr Pro Ser Pro Gly Thr Thr Asp Leu Pro Gly Leu
            100                 105                 110
His Ala Arg Glu
        115

<210> 53
<211> 114
<212> PRT
<213> Ictidomys tridecemlineatus

<400> 53
Ala Pro Pro Thr Cys Tyr Ser Arg Met Leu Thr Leu Ser Arg Glu Ile
1               5                   10                  15
Thr Ser Glu Phe Gln Gly Leu Gln Ala Ala Glu Pro Gly Glu Pro Cys
            20                  25                  30

Val Arg Tyr Leu Pro Gly Leu His Leu Asp Ile His Asn Tyr Cys Val
        35                  40                  45
Leu Ala Lys Leu Arg Asp Phe Val Ala Ser Pro His Cys Trp Lys Met
    50                  55                  60
Ala Gln Val Asp Ala Leu Lys Asp Lys Val Arg Lys Leu Tyr Thr Ile
65                  70                  75                  80
Met Asn Ser Phe Cys Arg Arg Asp Leu Val Phe Leu Ser Asp Asp Cys
                85                  90                  95
Ser Ala Leu Glu His Pro Ile Pro Val Thr Thr Gly Pro Pro Glu Gly
            100                 105                 110
Gln Ser

<210> 54
<211> 24
<212> DNA
<213> Artificial Sequence

<220>
<223> primer

<400> 54
tttgaatgat gagccttcgt cccc                                              24

<210> 55
<211> 26
<212> DNA
<213> Artificial Sequence

<220>
<223> primer

<400> 55
ggtctcaagt cagtgtacag gtaagc                                            26

<210> 56
<211> 16
<212> DNA
<213> Artificial Sequence

<220>
<223> primer

<400> 56
tggaccgcgc tttgct                                                       16

<210> 57
<211> 21
<212> DNA
<213> Artificial Sequence

<220>
<223> primer

<400> 57
ccctgggtag gtaactctgg g                                                 21

<210> 58
<211> 23

<212> DNA
<213> Artificial Sequence

<220>
<223> primer

<400> 58
attcccagcc catgagtcct tga                                               23


<210> 59
<211> 22
<212> DNA
<213> Artificial Sequence

<220>
<223> primer

<400> 59
acacgtggaa caccaacatc ct                                                22


<210> 60
<211> 20
<212> DNA
<213> Artificial Sequence

<220>
<223> primer

<400> 60
actgtgtgct ggacaagctg                                                   20


<210> 61
<211> 20
<212> DNA
<213> Artificial Sequence

<220>
<223> primer

<400> 61
cattgcagtc atccaacagg                                                   20


<210> 62
<211> 19
<212> DNA
<213> Artificial Sequence

<220>
<223> primer

<400> 62
tagctccctg attgcctgt                                                    19


<210> 63
<211> 20
<212> DNA
<213> Artificial Sequence

<220>
<223> primer

<400> 63
ggagaagggg ttgctgaagt                                                    20


<210> 64
<211> 20
<212> DNA
<213> Artificial Sequence

<220>
<223> primer

<400> 64
gacaatctgt accccgagga                                                    20


<210> 65
<211> 20
<212> DNA
<213> Artificial Sequence

<220>
<223> primer

<400> 65
tcccaagtag gtcacggtct                                                    20


<210> 66
<211> 26
<212> DNA
<213> Artificial Sequence

<220>
<223> primer

<400> 66
cctctactcc agtaaacctg attggg                                             26


<210> 67
<211> 24
<212> DNA
<213> Artificial Sequence

<220>
<223> primer

<400> 67
tgttcccagc atttcacact atgg                                               24


<210> 68
<211> 20
<212> DNA
<213> Artificial Sequence

<220>
<223> primer

<400> 68
gatgccagga caacctttct                                                    20


<210> 69
<211> 20
<212> DNA
<213> Artificial Sequence

<220>
<223> primer

<400> 69
agcaaatggg tcaaggtggt                                                    20


<210> 70
<211> 20
<212> DNA
<213> Artificial Sequence

<220>
<223> primer

<400> 70
tgtgtctgca aagggacgtt                                                    20


<210> 71
<211> 20
<212> DNA
<213> Artificial Sequence

<220>
<223> primer

<400> 71
ccaggttgtg cagcttgttc                                                    20


<210> 72
<211> 20
<212> DNA
<213> Artificial Sequence

<220>
<223> primer

<400> 72
gcttttcatc tgtcccgctg                                                    20


<210> 73
<211> 20
<212> DNA
<213> Artificial Sequence

<220>
<223> primer

<400> 73
agttggggtc catttcgagg                                                    20

<210> 74
<211> 20
<212> DNA
<213> Artificial Sequence

<220>
<223> primer

<400> 74
tttctgcctt ctcctgcatt                                                    20


<210> 75
<211> 20
<212> DNA
<213> Artificial Sequence

<220>
<223> primer

<400> 75
gtggcaccaa gtcttccaat                                                    20


<210> 76
<211> 20
<212> DNA
<213> Artificial Sequence

<220>
<223> primer

<400> 76
cttctctgcc tcacctggtc                                                    20


<210> 77
<211> 20
<212> DNA
<213> Artificial Sequence

<220>
<223> primer

<400> 77
tctccaaggt gtgtttgcag                                                    20


<210> 78
<211> 20
<212> DNA
<213> Artificial Sequence

<220>
<223> primer

<400> 78
aaaagcggag acaggagaca                                                    20


<210> 79
<211> 20

<212> DNA
<213> Artificial Sequence

<220>
<223> primer

<400> 79
cccttcatgt tggcttttct                                                    20


<210> 80
<211> 20
<212> DNA
<213> Artificial Sequence

<220>
<223> primer

<400> 80
ctacctccac catgccaagt                                                    20


<210> 81
<211> 20
<212> DNA
<213> Artificial Sequence

<220>
<223> primer

<400> 81
gcagtagctg cgctgataga                                                    20


<210> 82
<211> 13
<212> PRT
<213> Artificial Sequence

<220>
<223> Peptide

<400> 82
Glu Tyr Pro Ile Pro Val Thr Thr Val Leu Pro Asp Arg
1               5                   10

<210> 83
<211> 8
<212> PRT
<213> Artificial Sequence

<220>
<223> Peptide

<400> 83
Thr Pro Pro Thr Cys Tyr Ser Arg
1               5

## Description

The present invention relates to a protein, or a pharmaceutical composition thereof, and its use in the treatment or prevention of medical conditions such as ischemia, especially ischemic cardiomyopathy preferably related to myocardial infarction. The present invention further relates to a method for manufacturing said protein. In addition, the present invention relates to a method to identify, or to purify, endothelial colony-forming cells (ECFCs).

Ischemic heart disease, including myocardial infarction, was the leading cause of human death worldwide in 2011, according to the World Health Organisation. Limitation of blood flow to the heart causes ischemia of the myocardial tissue.

In treatment of myocardial infarction (and ischemic heart disease in general), it is of critical importance for the survival of the patient to prevent as much myocardial tissue damage (and eventually necrosis) as possible. This is mainly achieved through revascularisation (restoration of blood flow) by reperfusion therapy, comprising thrombolytic therapy, percutaneous coronary intervention or bypass surgery. In the very common case of ST-elevation myocardial infarction (STEMI), standard of care for management of the condition is given in O'Gara et al. (O'Gara et al., Circulation 2013, Pubmed-ID (PMID): 23247304).

However, improvement in prevention and therapy is still very much in need: Among patients with STEMI, in-hospital mortality is about 6% and 1-year mortality is 7% to 18% in the United States of America (O'Gara et al.). Moreover, myocardial cardiomyopathies, which frequently develop in the aftermath of heart infarcts, constitute a huge number of cases in need of therapies with the potential to prevent tissue loss and to trigger regeneration of functional vessels and heart muscle.

The administration of endothelial progenitor cells to patients having myocardial infarction (or ischemic disease in general) could be used in successful treatment, by promoting revascularisation of the ischemic tissue. At least in humans, a specific type of a rare true endothelial progenitor can be isolated (Martin-Ramirez et al., Nature Protocols 2012). These cells have been termed endothelial colony forming cells (ECFCs). However, the protocol for isolating sufficient numbers of these cells from peripheral blood for therapy is time-consuming, taking over 21 days on average (Hofmann et al., J Vis Exp 2009, PMID: 19861942, abstract). Moreover, another problem in regard to successful isolation is that a unique marker specific for ECFCs has not yet been described.

Thus an object of the present invention is to provide a compound or cell, in particular a novel compound, especially for use in treatment or prevention of tissue damage or ischemic disease, especially myocardial infarction. Another object of the present invention is to find whether said compound (or an antibody or inhibitor/competitor thereto) or cell is suitable for treatment of any other condition or disease such as cancer and provide said compound or cell for use in treatment of said disease. In addition, an object of the present invention is to provide a method for manufacturing said compound or cell.

Another object of the present invention is to provide a method for identifying - or purifying or enriching for - ECFCs. Furthermore, an object of the present invention is to provide the product of said methods for use in treatment of a disease or condition, preferably ischemia, especially myocardial infarction.

Therefore, the present invention discloses a novel glycosylated form of the Cytokine-like 1 (CYTL1) protein, as well as said protein for use in treatment or prevention of a disease or condition, preferably tissue damage or ischemic disease, especially myocardial infarction. Surprisingly, specifically glycosylated CYTL1 strongly induces functional vessel formation in vivo, whereas the activity of the unglycosylated form is strongly reduced. In the course of the present invention, in a rat acute infarct model, specifically glycosylated CYTL1 turned out to attenuate scar formation and functional impairment of heart muscle. It was previously unknown that CYTL1 is suitable for therapeutic use in tissue damage or ischemic disease, especially myocardial infarction.

Also in the course of the present invention, CYTL1 turned out to be a reliable marker for ECFCs. Consequently, the present invention also discloses a method for identifying - or purifying or enriching for - ECFCs by using CYTL1 as a marker, as well as the product of said methods for use in treatment of a disease or condition, preferably tissue damage or ischemia, especially myocardial infarction. Further embodiments are also disclosed below.

In an aspect,the present invention provides an isolated CYTL1 protein,
wherein the protein sequence is more than 80%, preferably more than 90%, more preferably more than 95%, even more preferably more than 98%, especially completely identical to the entire amino-acid sequence of an entry selected from the group of the following: UniProt entries Q9NRR1, G1S206, F7CJ05, H2QP59, G3QV27, H2PCW0, G7MSJ5, G7P588, S7MTB0, G3WH39, B5DFG7, U6D578, G3TD71, F1P7S3, H0WPN0, L5LQ03, W5PBC3, E1BN89, L8IQ55, G3GWE1, M3W6F7, M3Y970, D2HLS4, G1M521, G1T5G9, G5B313, I3LW41 (SEQ ID NOS: 1-27) and their respective processed proteins (SEQ ID NOS: 28-53); and
wherein the protein sequence comprises two consecutive threonine residues, and wherein the first of said residues is glycosylated and/or wherein the second of said residues is glycosylated; and
wherein the protein induces sprouting in a spheroid-based in-vitro angiogenesis assay compared to an uninduced control.

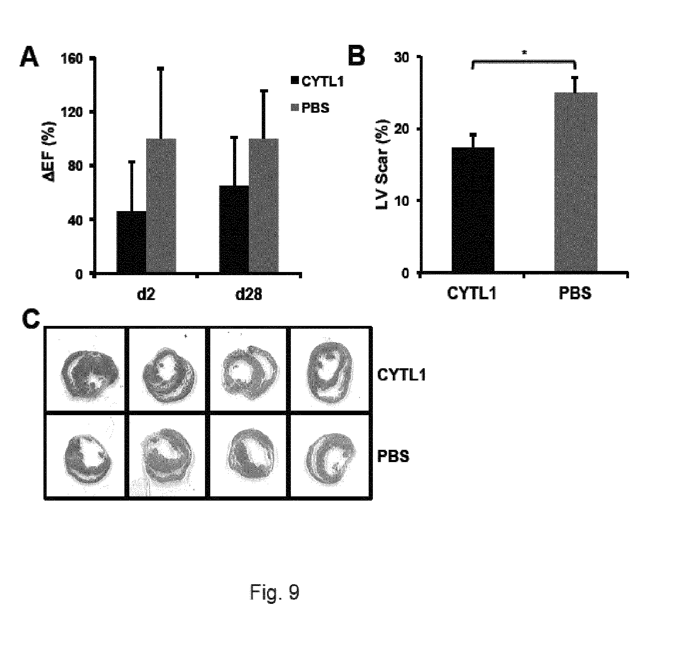

This protein is especially suitable for use in therapy, in particular for use in treatment of myocardial infarction (see also Fig. 9 and Example 11).

The integrity and functioning of all tissues is intimately linked with the vascular system which ensures appropriate supply of oxygen and nutrients as well as controls immune cell diapedesis, inflammation and, in case of damage, regeneration. A general overview about angiogenesis is given by Risau (Risau, Nature 1997, PMID: 9109485).

The vascular system is initially formed in the embryo by vasculogenesis, a process of generation and tubular assembly of endothelial cells from angioblast progenitors. Later on during growth and in the adult, new vessels are formed primarily by sprouting angiogenesis, i.e. the sprouting of endothelial cells from endothelial cells present in the vessel wall of capillaries (Carmeliet, Nature 2005, PMID: 16355210; Chung & Ferrara, Annu Rev Cell Dev Biol 2011, PMID: 21756109). The primary initiator of vasculogenesis as well as blood vessel angiogenesis is vascular endothelial growth factor-A, which can be produced by all cell types upon hypoxia, and activates endothelial progenitors or vessel wall resident endothelial cells mainly via VEGF receptor-2 (VEGFR-2) (Hofer & Schweighofer, Thromb Haemost 2007, PMID: 17334501; Shibuya & Claesson-Welsh, Exp Cell Res 2006, PMID: 16336962).

Based on the early work of Asahara et al. (Asahara et al, Science 1997, PMID: 9020076) a new field of research on endothelial progenitor cells in the adult was triggered, which was based on the description of circulating cells with properties of endothelial progenitors. This led to a concept of postnatal vasculogenesis contributing to neovessel formation in the adult (Bautch, Nature Medicine 2011, PMID: 22064433). However, following a decade of research it became clear that the majority of the cells described as potential endothelial progenitor cells were rather proangiogenic hematopoietic cells (PACs), which are recruited to sites of injury contributing to angiogenesis by paracrine factors, but not stably integrating into the vessel wall. However, at least in humans, a specific type of a rare true endothelial progenitor can be isolated from the mononuclear fraction of blood by plating onto collagen I-coated plates in a defined endothelial cell growth medium containing VEGF-A (Basile & Yoder, J Cell Physiol 2014, PMID: 23794280; Yoder et al, Blood 2007, PMID: 17053059). These cells have been termed endothelial colony forming cells (ECFCs) or late blood outgrowth endothelial cells (BOECs) (Martin-Ramirez et al, 2012).

ECFCs have been shown to home into hypoxic tumor tissue (Lucas et al, Stem cells 2009, PMID: 19522014) and to promote revascularization in the hindlimb ischemia (Yoon et al, Circulation 2005, PMID: 16145003) and heart infarction models (Dubois et al, J Am Coll Cardiol 2010, PMID: 20466204) accompanied by stable integration into the endothelial cell layer of newly formed vessels. These cells therefore are true endothelial progenitors and, in distinction to PACs, are thought to be not of hematopoietic origin but are rather derived from an endothelium resident pool of progenitors shed into circulation (Basile & Yoder, 2014). Irrespective of their debated origin and normal physiological role, these cells hold great promise for cell therapies of ischemic diseases (Dubois et al, 2010) and as a source of cells for various forms of engineering of vascularized tissues (Tulloch et al, Circ Res 2011, PMID: 21597009). ECFCs have been shown to have excellent proliferative and ischemic tissue homing capacities.

One problem in the field is that a unique marker specific for ECFCs has not yet been described. Generally, ECFCs express endothelial surface markers very similar to mature endothelial cells isolated from the vessel wall, including VEGFR-2, CD31 and VE-cadherin (Yoder et al, 2007).

In the course of the present invention, gene profiling to establish a unique transcriptomic profile of ECFCs, in comparison to mature endothelial cells of the vessel wall, was hence performed. It was found that one distinction of ECFCs is the high expression of a little investigated cytokine, termed CYTL1 (and also known as C17), which is much less expressed in mature endothelial cells. CYTL1 has originally been detected in CD34+ cells from bone marrow and cord blood (Liu et al, Genomics 2000, PMID: 10857752). Meanwhile, it was further described in articular chondrocytes (Minogue et al, Arthritis Rheum 2010, PMID: 20722018) and to regulate chondrogenesis of mesenchymal stem cells (Kim et al, JBC 2007, PMID: 17644814). It was shown to be required for cartilage homeostasis and its loss to be associated with osteoarthritis (Chao et al, PloS One 2011, PMID: 21799806; Jeon et al, JBC 2011, PMID: 21652695; Stenberg et al, Proteome Science 2013, PMID: 24090399). Furthermore, it has been linked to proliferation and metastasis of neuroblastoma cells (Wen et al, Int J Onco 2012, PMID: 22797702).

CYTL1 is also known from patent applications WO 2000/63382 A9, WO 2002/089727 A2 and WO 2009/055613 A2, unrelated to the present invention, however.

Herein it is shown that CYTL1 is a bona fide pro-angiogenic factor produced by ECFCs, which mediates high angiogenic sprouting of ECFCs and vessel wall endothelial cells by autocrine and paracrine stimulation, respectively. It is uniquely O-glycosylated and this modification is required for the stimulation of sprouting in vitro and vessel formation in vivo. Herein it is further shown that CYTL1 acts independently of VEGF-A and can partly be induced by hypoxia, but its mechanism of action is distinct and does not include the inflammatory component observed for VEGF-A. It strongly upregulates certain anti-apoptotic and anti-inflammatory genes and causes in a heart infarct model a surprising reduction of functional impairment and scar formation.

It is likely that part of the beneficial effects of CYTL1 in a rat acute infarct model (see Examples) is due to increased revascularization stimulated by CYTL1 which would allow improved regeneration of the heart muscle. However, and most surprisingly, a strong reduction in the impairment of heart function (EF as measured by sonography) was already detected two days after ligation (Figure 9A). Such a short-term beneficial effect is unlikely to be a result of neovessel formation.

This shows, most surprisingly, that CYTL1, *independent* of its neovascularization triggering function, has a protective role in tissue damage (and therefore also in ischemia and myocardial infarction and related diseases). It is likely that CYTL1 protects from stress-induced cell death and destructive inflammatory reactions, thereby diminishing the loss of functional vessels and cardiomyocytes and facilitating consecutive regeneration of the heart muscle. It is therefore even more suitable for therapy than ECFCs themselves, in addition to the easier production, handling, and administration of a protein compared to cells.

Functional neovessel formation is a prerequisite for wound repair and the regeneration of any damaged tissue. Despite the last two decades have seen enormous progress in the understanding of regulatory mechanisms leading to physiological and pathological vascularization and specific compounds to inhibit tumor angiogenesis have been developed (Carmeliet, 2005; Chung & Ferrara, 2011), the triggering of functional vessel formation in ischemic diseases is still an unresolved issue. Application of individual angiogenic growth factors and of a variety of stem and progenitor cells in animal models has so far only resulted in limited effects.

Furthermore, increasing attempts are directed to generate a variety of vascularized tissues ex vivo for transplantation to replace damaged tissue parts (Tulloch et al, 2011). Also in these cases a correct temporal-spatial triggering with growth factor or cytokines in combination with the delivery of appropriate stem and progenitor cells is likely required for functional vessel formation. In this regard the better understanding of the function of adult endothelial progenitors is of high importance.

Whereas a number of progenitor cells have been described to promote angiogenesis, ECFCs are the only cell type that can be isolated from human blood, which has been shown to improve vessel formation as well as to stably integrate into the newly formed vessels (Yoder et al, 2007). It has been proposed that these cells are shed from a pool of vessel resident progenitor cells such as from microvessels of the lung (Basile & Yoder, 2014). They circulate in low numbers and possess the capacity to home into ischemic tissues and contribute to neovessel formation (Dubois et al, 2010; Yoon et al, 2005). Data suggesting the presence of endothelial progenitors in the vessel wall have also been obtained from mice (Naito et al, EMBO J 2012, PMID: 22179698), however due to their scarcity the isolation from murine blood has not been routinely feasible and prevented their thorough investigation in the mouse model (Basile & Yoder, 2014).

Irrespective of their debated normal physiological role, the availability of a circulating cell that can be isolated from peripheral blood, grown to large quantities and used to trigger vascularization in vivo or to form vascular networks ex vivo in tissue patches has high therapeutic potential. It is conceivable that these cells can be used in an autologous way when isolated from ischemia patients or in an allogeneic way when prepared from HLA-matched cord blood samples. There is increasing interest especially in cord blood derived cells given the steady increase of available cord blood samples in corresponding cord blood banks, the increased number of autologous samples stored for individual persons at birth and the high proliferative capacity of cord blood derived cells.

As the nature and distinction of ECFCs from mature endothelial cells of the vessel wall remained unknown in the course of the present invention gene profiling using ECFCs from cord and adult peripheral was performed and compared the transcriptome to HUVEC and adult HVEC. It was striking that in both samples CYTL1 turned out to be the highest differentially expressed gene encoding a secreted protein (Table 1).

Due to the fact that only limited data have been published so far on CYTL1, the relevance of this finding was initially unclear. CYTL1 has been described to be expressed in hematopoietic CD34+ cells and in chondrocytes (Liu et al, 2000; Minogue et al, 2010). It was proposed to be distantly related in structure to MCP1 (CCL2) and to possibly bind to CCR2 (Tomczak & Pisabarro, 2011), however this was based on computer modeling on basis of the primary sequence only and confirming experimental data are missing. Furthermore, it has been implicated in promoting chondrogenesis, cartilage homeostasis and seems to reduce damage in an osteoarthritis model (Chao et al, 2011; Jeon et al, 2011; Kim et al, 2007). In neuroblastoma cells it has been linked to proliferation and metastasis (Wen et al, 2012).

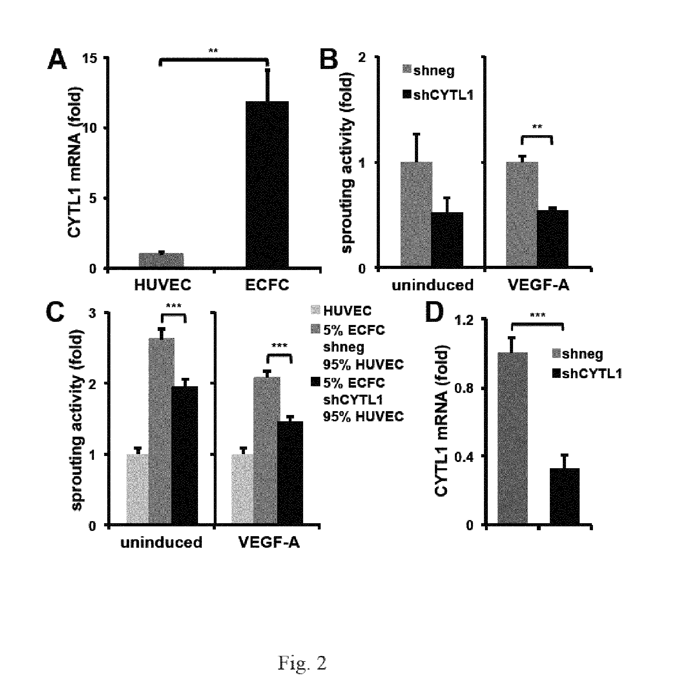

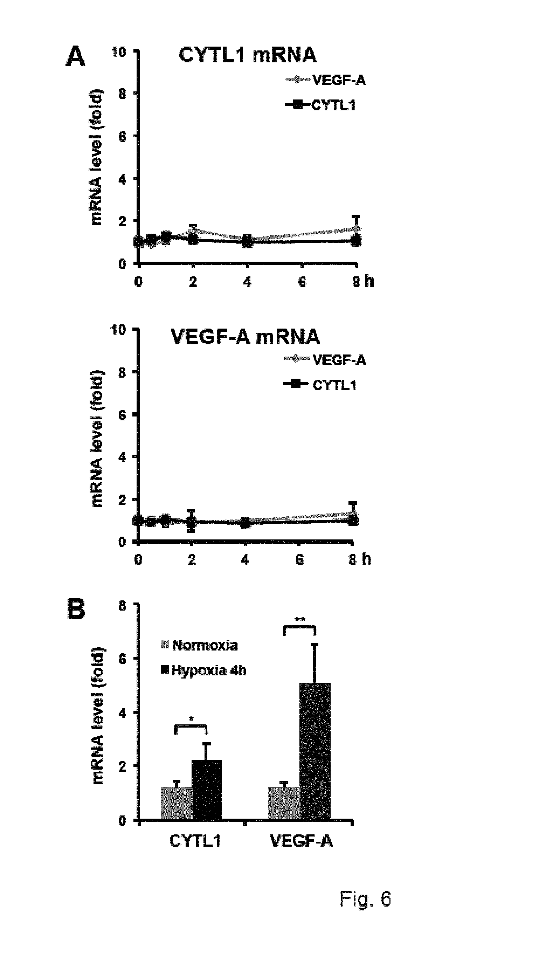

However, our data displays another important function for CYTL1 in the context of vessel formation. Herein it is shown that CYTL1 is highly expressed by ECFCs and mediates their high sprouting capacity. Moreover, it can act in a paracrine way to stimulate mature endothelial cells of the vessel wall (Figure 2). Thus CYTL1 provides a clue to the role of ECFCs in regard of stimulation of vessel formation. It is conceivable that ECFCs migrate to hypoxic areas, integrate into the vessel wall and stimulate sprouting by secretion of CYTL1. This could be either achieved by stimulating neighboring endothelial cells to become tip cells or by forming a tip cell itself. That CYTL1 plays a role in vessel formation is further corroborated by the finding that CYTL1 expression can be induced by hypoxia, the normal physiological trigger of angiogenesis (Figure 6B).

The possibility that CYTL1 may not be directly angiogenic by itself, but could induce VEGF-A and thus indirectly angiogenic sprouting, was considered. However, several lines of evidence show that this is not the case. First, it was shown that downmodulation of CYTL1 can reduce sprouting of ECFCs in the presence of excess of VEGF-A, showing that CYTL1 and VEGF-A work additively (Figure 2B). Second, it was directly shown that neither can CYTL1 induce VEGF-A mRNA nor VEGF-A CYTL1 mRNA (Figure 6A). Finally, blocking VEGFR-2 has no effect on CYTL1 activity and blocking CYTL1 does not affect VEGF-A stimulation of sprouting (Figure 7). This clearly demonstrates that both factors are working independently of each other. Most importantly, CYTL1 promotes vessel formation in vivo comparable to a mixture of VEGF-A and bFGF, showing its high efficacy (Figure 8).

Secreted type I proteins such as CYTL1 possess a signal peptide at their N-terminus directing the ribosome to endoplasmatic membranes at which the growing peptide chain is transclocated into endosomes following cleavage of the signal peptide. In addition, secreted proteins are frequently co- or post-translationally modified, the vast majority very often by N-linked glycosylation. Usually N-glycosylation takes place at Asn residues within certain consensus sites. This type of glycosylation sequence is not present in CYTL1. Another more variform modification is O-glycosylation at Ser or Thr residues for which only empirical predictions for likelihood of glycosylation of a particular sequence are available (for instance by the Hirst Glycosylation Predictor).

In the majority of cases glycosylation of growth factors and cytokines does not directly affect receptor binding and bioactivities, the modifications rather being involved in more subtle functions such as stability of the factors in vivo. Therefore many of the factors used in research or even in the clinic are produced in bacteria, yeast or insect cells, which cannot modify proteins with the authentic carbohydrate residues as done in human cells: This is what made the findings during the present invention in regard to bioactivity of the glycosylated form most surprising.

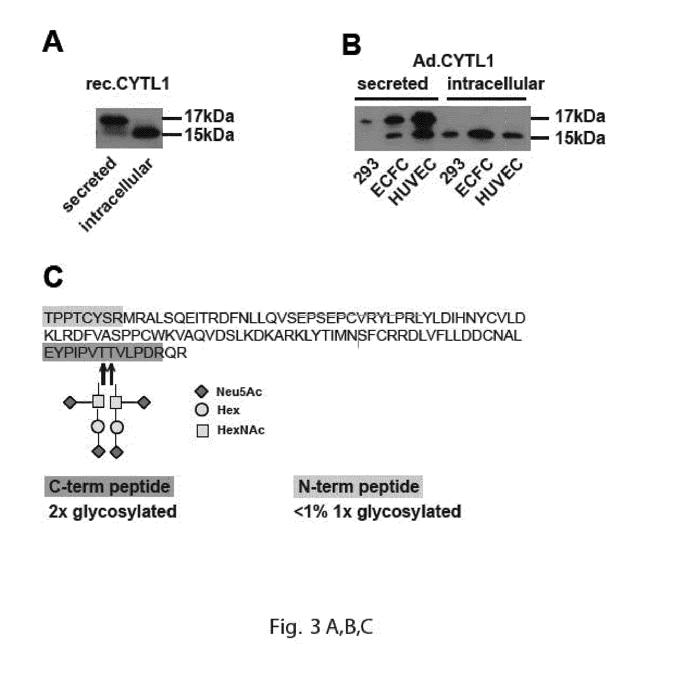

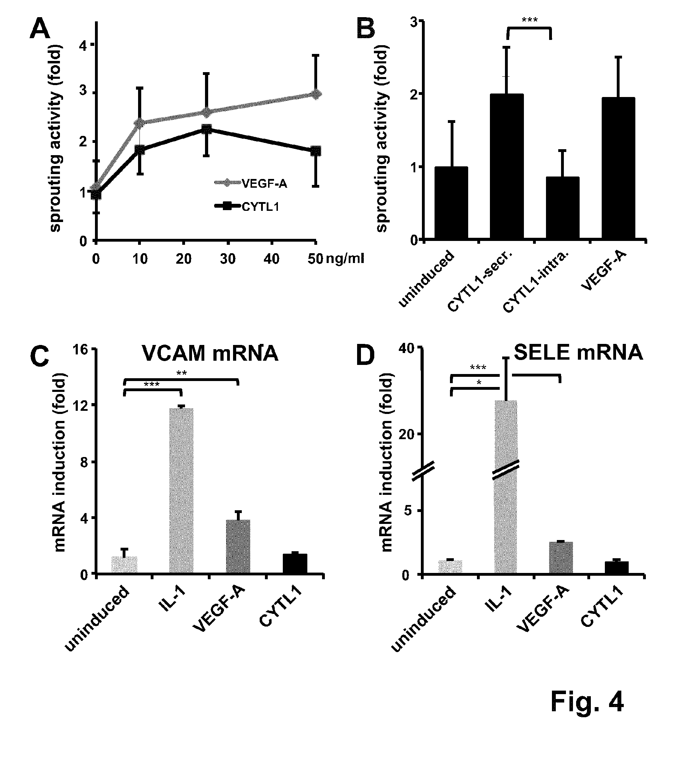

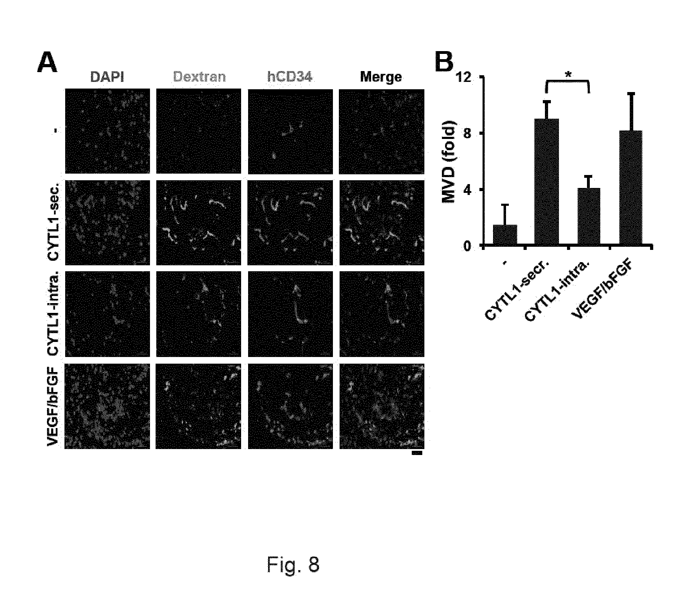

When it was noticed that secreted CYTL1 was modified by about 2 kDa (Figure 3A and B), but the CYTL1 sequence did not contain a recognizable N-linked glycosylation site or a unambiguously suggested or predicted O-glycosylation site, the precise nature of the modification was elucidated in the course of the present invention. By mass spectometry threonines 128 and/or 129 were identified to be both O-glycosylated with a specific carbohydrate structure composed of 1 HexNAc, 1 Hex and two Neu5Ac residues as displayed in Figure 3C. Unexpectedly, the not yet glycosylated form of CYTL1 isolated from cell lysates was much less active in stimulating sprouting in vitro or vessel formation in vivo (Figure 4B and Figure 8). This showed that in the case of CYTL1 surprisingly the correct specific modification is essential for full bioactivity presumably due to its role in receptor binding.

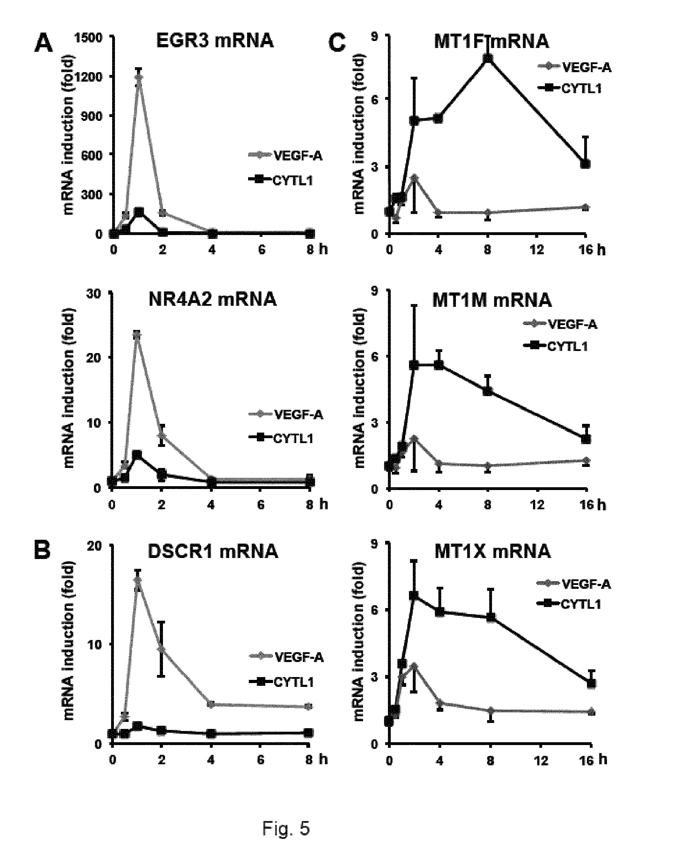

The global gene repertoire induced by VEGF-A in endothelial cells was previously investigated (Schweighofer et al, Thromb Haemost. 2009, PMID: 19718476). In this work it was found that VEGF-A induced a large gene repertoire, which displayed an astonishing overlap with the repertoire induced by the inflammatory cytokine IL-1. This showed that VEGF-A activity has an inflammatory component as many genes typically regarded as inflammatory were partially or fully induced also by VEGF-A. In addition, VEGF-A specifically triggered the upregulation of a small group of angiogenesis-related genes not induced by IL-1 or a more general growth factor, EGF. To gain information to which extent CYTL1 would parallel the activities observed for VEGF-A, selected genes from the inflammatory VEGF-A/IL-1 and the angiogenic VEGF-A specific cluster were tested for their inducibility by CYTL1. It was found that CYTL1 triggered the upregulation of two exemplary genes of the angiogenic cluster, namely EGR-3 and and NR4A3, although to a lower extent than VEGF-A (Figure 5A and B). As both of these transcription factors have been shown to contribute to the angiogenic response (Liu et al, 2008; Zhao et al, 2011) this shows the general induction of angiogenesis-related genes by CYTL1. However, unexpectedly CYTL1 did not induce genes from the inflammatory cluster. Neither VCAM1 nor E-selectin or DSCR1 were induced to a recognizable extent. This on the one side shows that the inflammatory VEGF-A/IL-1 cluster genes are not essential for the triggering of angiogenic sprouting and connected vessel formation, but rather is induced by VEGF-A in the context of a broader activity spectrum of the factor. It further showed that CYTL1 activity will be completely devoid of inflammatory activation.

To gain further insight into distinct mechanisms of action of CYTL1 gene profiling was used to define genes preferentially induced by CYTL1 but much less by VEGF-A. The list of genes obtained was headed by several isoforms of metallthionein genes, which were all at much higher levels and more sustained expressed after CYTL1 (Figure 5C). It is striking that metallothioneins are small cystein-rich proteins, which have been implicated in the scavenging of reactive oxygen species, the inhibition of inflammatory cytokines and the inhibition of apoptosis (Nielsen et al, 2007). Specifically in vascular cells they have been shown to facilitate proliferation, migration and network formation. Metallothioneins were also shown to protect from loss of capillaries in the diabetic heart (Xue et al, Am J Physiol Heart Circ Physiol 2012, PMID: 22523246) and to improve pathogenesis of acute lung injury (Wesselkamper et al, Am J Respir Cell Mol Biol 2006, PMID: 16166738). These proteins seem therefore to have unique roles in cytoprotection against degeneration and the stimulation of repair processes including revascularization and activation of stem/progenitor cells (Nielsen et al, 2007). It is therefore possible that ECFC-secreted CYTL1 via strong induction of metallothioneins is a natural protector from degenerative processes facilitating repair processes devoid of the adverse inflammatory side effects of VEGF-A.

Indeed, when CYTL1 was evaluated in an acute heart infarct model, it was observed that scar formation was significantly reduced four weeks after ligation of the left anterior descending coronary artery. In accordance, functional impairment as evaluated by left ventricular ejection fraction was improved (Figure 9). Whereas improved function four weeks after infarct could be attributed to better revascularization followed by muscle regeneration, unexpectedly a reduced functional impairment was already observed on day 2 after ligation. This is unlikely to be caused by neovessel formation, but rather shows a protective effect of CYTL1 on heart vessels and heart muscle still functional after ligation in the infarct border zone.

Taken together our data show a functioning of ECFCs which is in line with the proposal of a vascular progenitor cells shed in low number into the circulation to replenish the endothelial cell layer, to home to sites of hypoxia after wounding or other tissue damage and to support sprouting of capillaries to achieve neovascularization in combination with VEGF-A produced locally by hypoxic cells. This further shows the potential of ECFCs and of the factors produced by the cells for regenerative therapies. Herein it is shown for the first time that ECFCs are distinct from mature endothelial cells by their high capacity to produce CYTL1, which mediates at least in part the neovascularization functions of ECFCs previously observed in ischemia models. Furthermore, the novel data on CYTL1 demonstrate that it is a bona fide angiogenic factor with a distinct activity profile obviously devoid of inflammatory activity and rather protective from inflammatory damage.

All of this strongly shows that CYTL1 itself can be used alone or possibly in combination with ECFCs to achieve improved therapeutic effects on protection from tissue damage and regeneration of vascularized tissues such as the ischemic heart.

**Table 3 - CYTL1 sequences of the present invention and their UniProt sequence version (as of 5 May 2014 - database release 2014_04)**

| SEQ ID NO. | UniProt | species | seq. version |
|---|---|---|---|
| 1 | Q9NRR1 | Homo sapiens | 1 |
| 2 | G1S206 | Nomascus leucogenys | 1 |
| 3 | F7CJ05 | Macaca mulatta | 1 |
| 4 | H2QP59 | Pan troglodytes | 1 |
| 5 | G3QV27 | Gorilla gorilla gor. | 1 |
| 6 | H2PCW0 | Pongo abelii | 1 |
| 7 | G7MSJ5 | Macaca mulatta | 1 |
| 8 | G7P588 | Macaca fascicularis | 1 |
| 9 | S7MTB0 | Myotis brandtii | 1 |
| 10 | G3WH39 | Sarcophilus harrisii | 1 |
| 11 | B5DFG7 | Rattus norvegicus | 1 |
| 12 | U6D578 | Neovison vison | 1 |
| 13 | G3TD71 | Loxodonta africana | 1 |
| 14 | F1P7S3 | Canis familiaris | 2 |
| 15 | H0WPN0 | Otolemur garnettii | 1 |
| 16 | L5LQ03 | Myotis davidii | 1 |
| 17 | W5PBC3 | Ovis aries | 1 |
| 18 | E1BN89 | Bos taurus | 1 |
| 19 | L8IQ55 | Bos mutus | 1 |
| 20 | G3GWE1 | Cricetulus griseus | 1 |
| 21 | M3W6F7 | Felis catus | 1 |
| 22 | M3Y970 | Mustela putorius furo | 1 |
| 23 | D2HLS4 | Ailuropoda melanoleuca | 1 |
| 24 | G1M521 | Ailuropoda melanoleuca | 1 |
| 25 | G1T5G9 | Oryctolagus cuniculus | 2 |
| 26 | G5B313 | Heterocephalus glaber | 1 |
| 27 | I3LW41 | Ictidomys tridecemlin. | 1 |
| And the (putative) processed protein sequences thereof: | | | |
| (after cleavage of the respective signal peptide) | | | |
| | subsequence of | | |
| 28 | Q9NRR1 | Homo sapiens | |
| 29 | G1S206 | Nomascus leucogenys | |
| 30 | F7CJ05 | Macaca mulatta | |
| 31 | H2QP59 | Pan troglodytes | |
| 32 | G3QV27 | Gorilla gorilla gor. | |
| 33 | H2PCW0 | Pongo abelii | |
| 34 | G7MSJ5 | Macaca mulatta | |
| 35 | G7P588 | Macaca fascicularis | |
| 36 | S7MTB0 | Myotis brandtii | |
| 37 | G3WH39 | Sarcophilus harrisii | |
| 38 | B5DFG7 | Rattus norvegicus | |
| 39 | U6D578 | Neovison vison | |
| 40 | G3TD71 | Loxodonta africana | |
| 41 | F1P7S3 | Canis familiaris | |
| 42 | H0WPN0 | Otolemur garnettii | |
| 43 | L5LQ03 | Myotis davidii | |
| 44 | W5PBC3 | Ovis aries | |
| 45 | E1BN89 | Bos taurus | |
| 46 | L8IQ55 | Bos mutus | |
| 47 | G3GWE1 | Cricetulus griseus | |
| 48 | M3W6F7 | Felis catus | |
| 49 | M3Y970 | Mustela putorius furo | |
| 50 | D2HLS4 | Ailuropoda melanoleuca | |
| 51 | G1M521 | Ailuropoda melanoleuca | |
| 52 | G1T5G9 | Oryctolagus cuniculus | |
| 53 | I3LW41 | Ictidomys tridecemlineatus | |

As found in the course of the present invention, secreted human CYTL1 (UniProt Q9NRR1, SEQ ID NO: 1) is glycosylated at threonine 128 and/or 129 (threonine 106 and 107, respectively, when not taking the signal peptide into account) and this modification is essential for its bioactivity. These two consecutive threonines (also called "TT residues" herein) are located close to the C-terminus of CYTL1. This protein therefore is exceptionally eligible in the present invention.

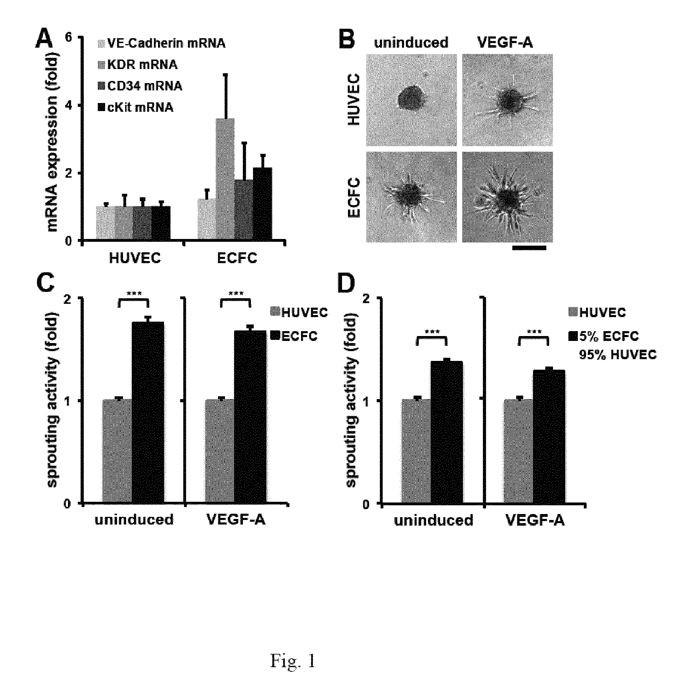

In WO 2000/63382 A1, in example 3, it is found that digestion with a panel of glycosidases failed to shift the protein migration of CYTL1. In contrast to this, Chao et al. (Chao et al., PloS One 2011, PMID: 21799806, especially Fig. 1) speculated that both N-terminal threonine 26 and C-terminal threonine 129 are glycosylated. They based their assumption solely on a comparison between human and murine CYTL1 sequences (and neither on any other bioinformatical analysis nor on experimental data), as there is no consensus sequence known for O-linked glycosylation. A third source, the Hirst Glycosylation predictor (http://comp.chem.nottingham.ac.uk/glyco/), rated threonine 128 as non-glycosylated and in turn many other sites of CYTL1 as O-glycosylated. Taken together, the opinion of the field in regard to CYTL1 glycosylation is under debate.

In fact, however, as shown above by mass spectrometric analysis, essentially only the C-terminal threonines (the "TT residues") are glycosylated, in 50% of the cases both of them. Moreover, Chao et al. were silent on whether the glycosylation is necessary for biological function of the protein - very often proteins are functional even without glycosylations (as it is common that glycosylation only serves to increase solubility of the protein or guide protein localisation). It was therefore most surprising, in the course of the present invention, that T128/T129-glycosylated CYTL1 strongly induces functional vessel formation in vivo, whereas the activity of the unglycosylated form is markedly reduced.

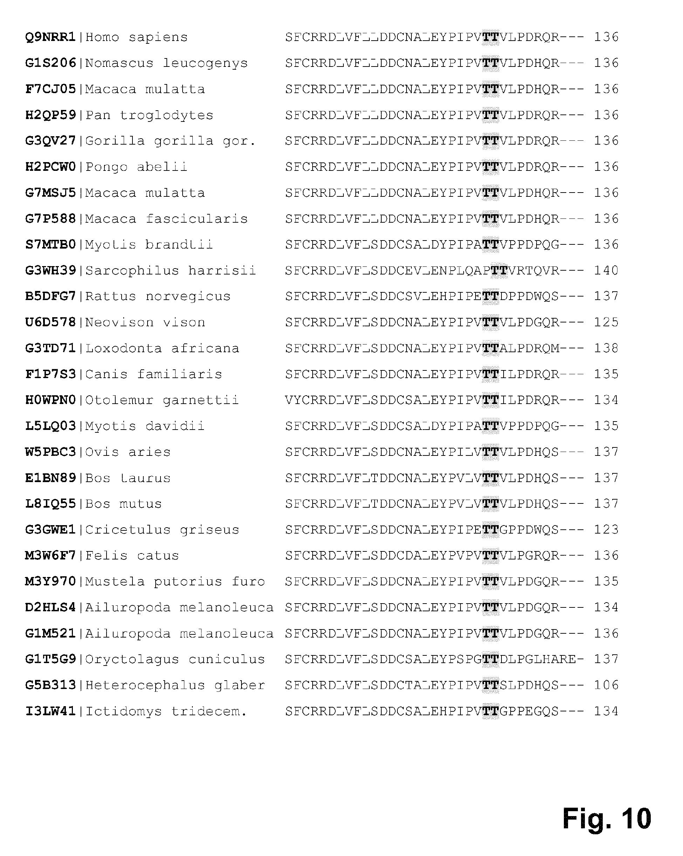

The TT residues are highly conserved among mammals (Fig. 10 shows the respective TT residues of proteins of the present invention). Thus it is very plausible that these TT residues are also modified according to what was found in the course of the present invention (i.e. by glycosylation). This is the reason why the homologue UniProt entries G1S206, F7CJ05, H2QP59, G3QV27, H2PCW0, G7MSJ5, G7P588, S7MTB0, G3WH39, B5DFG7, U6D578, G3TD71, F1P7S3, H0WPN0, L5LQ03, W5PBC3, E1BN89, L8IQ55, G3GWE1, M3W6F7, M3Y970, D2HLS4, G1M521, G1T5G9, G5B313, I3LW41 are also sequences of the present invention (SEQ ID NOS: 2-27, see Table 3 for sequences of the present invention). These proteins are very eligible in the present invention as well.

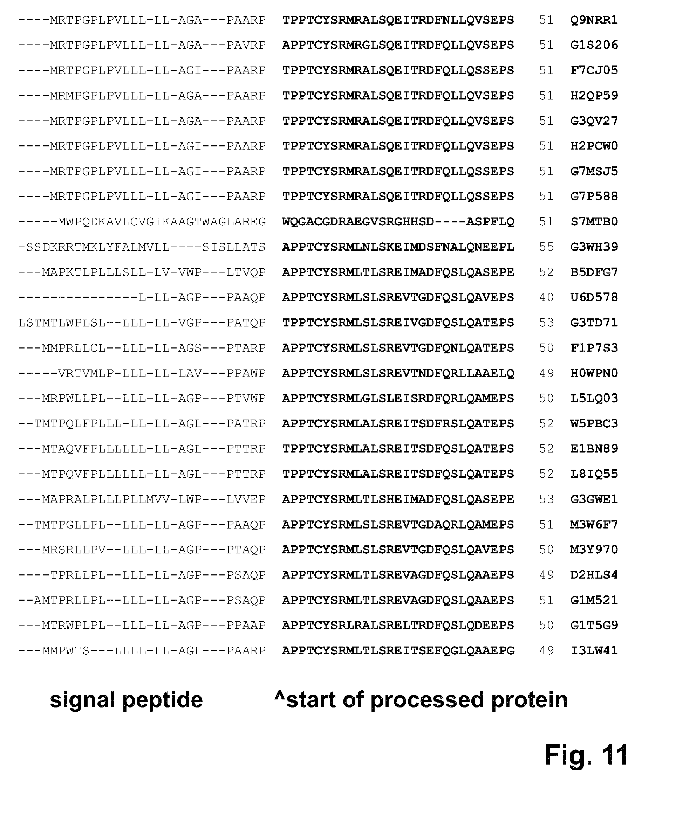

As mentioned, the first 22 amino acids of human CYTL1 (Q9NRR1) constitute a signal peptide that is cleaved upon secretion to yield a processed protein (SEQ ID NO: 28, Table 3). As found in the course of this invention, the signal peptide is not required for the newly characterised function of CYTL1, apart from the secretion process itself. According to sequence alignment, it is very plausible that the previously mentioned homologues also comprise a signal peptide (Fig. 11). Consequently, also the processed proteins of UniProt entries G1S206, F7CJ05, H2QP59, G3QV27, H2PCW0, G7MSJ5, G7P588, S7MTB0, G3WH39, B5DFG7, U6D578, G3TD71, F1P7S3, H0WPN0, L5LQ03, W5PBC3, E1BN89, L8IQ55, G3GWE1, M3W6F7, M3Y970, D2HLS4, G1M521, G1T5G9, G5B313, I3LW41 (SEQ. ID. NOS: 29-53, Table 3) are highly eligible in the present invention and SEQ ID NO: 28 is exceptionally eligible in the present invention. Most of them consist of 114 amino acids (like the processed human CYTL1), which means that for most of them the TT residues are at position 106-107 of their sequence (after processing).

The fact that protein function is not harmed by a certain amount of mutations is well known in the art. Therefore, also very eligible in the present invention is a protein with a protein sequence that is more than 80%, preferably more than 90%, more preferably more than 95%, especially more than 98% identical to the entire amino-acid sequence of the previously mentioned proteins (SEQ ID NOS: 1-53), as long as it maintains its function. The function is measured by whether the protein induces sprouting in a spheroid-based in-vitro angiogenesis assay compared to an uninduced control, which is well-known in the art (Korff & Augustin, JCB 1998, PMID: 9832561; T.Korff, in: Methods in Endothelial Cell Biology, ed. H.Augustin, Springer Verlag, 2004, chapter 12, p. 115; see also Example 1).

Therefore, in conclusion, an aspect of the present invention provides an isolated CYTL1 protein,
wherein the protein sequence is more than 80%, preferably more than 90%, more preferably more than 95%, even more preferably more than 98%, especially completely identical to the entire amino-acid sequence of an entry selected from the group of the following: UniProt entries Q9NRR1, G1S206, F7CJ05, H2QP59, G3QV27, H2PCW0, G7MSJ5, G7P588, S7MTB0, G3WH39, B5DFG7, U6D578, G3TD71, F1P7S3, H0WPN0, L5LQ03, W5PBC3, E1BN89, L8IQ55, G3GWE1, M3W6F7, M3Y970, D2HLS4, G1M521, G1T5G9, G5B313, I3LW41 (SEQ ID NOS: 1-27) and their respective processed proteins (SEQ ID NOS: 28-53); and
wherein the protein sequence comprises two consecutive threonine residues, and wherein the first of said residues is glycosylated and/or wherein the second of said residues is glycosylated, especially both; and
wherein the protein induces sprouting in a spheroid-based in-vitro angiogenesis assay compared to an uninduced control.

Typically, a CYTL1 protein of the invention induces sprouting (also called sprouting activity herein) in a spheroid-based in-vitro angiogenesis assay compared to an uninduced control, preferably at least 25% more, more preferably at least 50% more, especially at least 100% more or even at least 200% more.

The protein of the present invention is provided in isolated form, i.e. outside of the animal and/or human body. The protein of the present invention implicitly includes natural sequence variants of UniProt entries Q9NRR1, G1S206, F7CJ05, H2QP59, G3QV27, H2PCW0, G7MSJ5, G7P588, S7MTB0, G3WH39, B5DFG7, U6D578, G3TD71, F1P7S3, H0WPN0, L5LQ03, W5PBC3, E1BN89, L8IQ55, G3GWE1, M3W6F7, M3Y970, D2HLS4, G1M521, G1T5G9, G5B313, I3LW41, that are designated as such in the UniProt database as of the filing date of the present application. In the case of Q9NRR1, this comprises the single-nucleotide polymorphisms (SNPs) resulting in the mutations: S51L or R136C.

As stated above, the human CYTL1 (SEQ ID NO: 1) or its processed protein (SEQ ID NO: 28) are exceptionally eligible in the present invention, therefore another preferable embodiment of the present invention is an isolated CYTL1 protein with the protein sequence being more than 80%, preferably more than 90%, more preferably more than 95%, even more preferably more than 98%, especially completely identical to the entire sequence of UniProt entry Q9NRR1 (SEQ ID NO: 1) or of its processed protein (SEQ ID NO: 28).

Another preferred embodiment of the present invention is an isolated CYTL1 with the sequence of SEQ ID NO: 28,
wherein at most three, preferably at most two, more preferably at most one of the amino-acid residues, preferably except for the two consecutive threonine residues, are deleted or replaced by any other amino-acid residue independently of each other, and/or
wherein at most 10, preferably at most five, more preferably at most three, especially at most one other amino-acids are inserted into the sequence, except between the two consecutive threonine residues.

As well-known in the art, inserting or changing a few amino-acid residues is not necessarily harmful to protein function, so these proteins are still very suitable for achieving the biological effect, if the first of the consecutive two threonine residues is glycosylated and/or the second of the consecutive two threonine residues is glycosylated, especially both.

Another preferred embodiment of the present invention is the isolated protein comprising or consisting of the protein sequence SEQ ID NO: 28, preferably with no amino-acid residue replaced, deleted or inserted. This is the secreted human CYTL1 protein with which most of the experiments herein were performed, therefore it is exceptionally well-suited for the present invention, if the first of the consecutive two threonine residues is glycosylated and/or the second of the consecutive two threonine residues is glycosylated, especially both.

In another preferred embodiment, both of the TT residues are glycosylated. 50% of secreted human CYTL1 was found to have both TT residues glycosylated, therefore these proteins are very well-suited to perform their biological function.

In another preferred embodiment, one or each, especially each, of the TT residues are replaced by any other natural or unnatural amino acid residue that can be glycosylated, preferably tyrosine, asparagine, arginine, hydroxylysine, or hydroxyproline, especially serine since serine and threonine are very similar and act both as substrates of polypeptide:N-acetylgalactosaminyltransferases. Both serine and threonine are usually glycosylated by O-linked glycosylation. An overview about protein glycosylation in vertebrates such as mammals and its pharmacological significance is for instance given in Moremen et al. 2012 (Moremen et al., Nat Rev Mol Cell Biol. 2012, PMID: 22722607), and Li & d'Anjou 2009 (Li & d'Anjou, Curr Opin Biotechnol 2009, PMID: 19892545).

As found in the course of the present invention, the human CYTL1 glycosylation comprises a hexose (Hex), an N-acetylhexosamine (HexNAc) or a N-acetylneuraminic acid (Neu5Ac). Therefore, in a preferred embodiment of the invention, the glycosylation comprises a hexose (Hex), an N-acetylhexosamine (HexNAc) or a N-acetylneuraminic acid (Neu5Ac).

Hexoses are monosaccharides with six carbon atoms (total formula C₆H₁₂O₆), such as allose, altrose, glucose, mannose, gulose, idose, galactose, talose, psicose, fructose, sorbose, tagatose. Hexose deoxy sugars (total formula C₆H₁₂O₅) such as fucose shall also be included into the meaning of the term hexose herein. Mammalian glycans (in glycoproteins / glycosylated proteins) are usually assembled from only ten monosaccharides: fucose (Fuc), galactose (Gal), glucose (Glc), N-acetylgalactosamine (GalNAc), N-acetylglucosamine (GlcNAc), glucuronic acid (GlcA), iduronic acid (IdoA), mannose (Man), sialic acid (SA) and xylose (Xyl) (Moremen et al, 2012). Neu5Ac is the predominant SA found in mammalian cells. The HexNAc predominantly found in mammalian cells is GalNAc or GlcNAc.

Preferably, one or each, especially each, of said glycosylation(s) consists of 1 HexNAc, 1 Hex and 2 Neu5Ac.

More specifically, the following glycosylation pattern was found attached to the threonine:

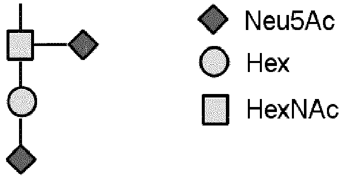

Therefore in a preferred embodiment of the invention, one or each, especially each, of said glycosylation(s) comprises or consists, especially consists, of this glycosylation pattern, preferably wherein the HexNAc is attached to the respective glycosylated residue of the protein. Also preferably the Hex is Gal and the HexNAc is GalNAc.

Within the glycan (glycosylation), the nature of the covalent linkage between the glycan monomers can vary, also due to so called micro-heterogeneity (Moremen et al, 2012). The linkage can be an S-, N-, C-, or O-glycosidic bond (either as a alpha or beta anomer) between the glycan monomers, positioned at a specific functional group of each monomer. Brockhausen et al., especially in Fig. 9.2, give examples for such linkages in O-linked GalNAc (a HexNAc) glycans (Brockhausen I, Schachter H, Stanley P. O-GalNAc Glycans. In: Varki A, Cummings RD, Esko JD, et al., editors. Essentials of Glycobiology. 2nd edition. Cold Spring Harbor (NY): Cold Spring Harbor Laboratory Press; 2009. Chapter 9).

In an especially preferred embodiment, said glycosylation or glycosylation pattern is **Neu5Acα2-3Gaβ1-3(Neu5Acα2-6)GalNAc:**

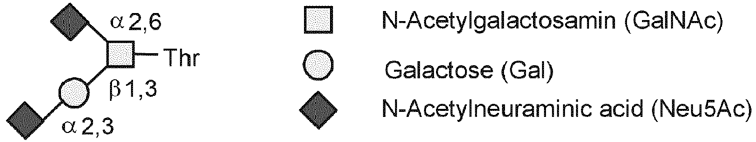
α 2,6
Thr
β 1,3
α 2,3

N-Acetylgalactosamin (GalNAc)
Galactose (Gal)
N-Acetylneuraminic acid (Neu5Ac)

As, in the course of the present invention, essentially no other glycosylation except on either one or both of the glycosylated residues was found, in a preferable embodiment of the present invention said glycosylation (s) of one or both of the glycosylated residues is/are the only glycosylation(s) of the protein.

It is well-known in the art that protein fragments can be as biologically active as the full-length protein (or sufficiently active), and sometimes protein fragments are preferable over full-length proteins due to considerations such as chemical stability, pharmaceutical half-life and dosing. Hence in another preferable embodiment the protein of the present invention consists of a fragment of the CYTL1 protein of the present invention, wherein the fragment comprises more than 20 amino-acids, preferably more than 40 amino-acids, more preferably more than 60 amino-acids, especially more than 100 amino-acids; and wherein the fragment induces sprouting in a spheroid-based in-vitro angiogenesis assay compared to an uninduced control (as it is important that the biological activity of the fragment is retained).

Similarly, since it is sometimes more preferable to provide a fusion protein instead of the original protein for the same reasons as mentioned above, another embodiment of the present invention provides a fusion protein comprising a CYTL1 protein of the present invention, further comprising at least one more amino-acid acid, preferably at least ten more amino-acids, more preferably at least 20 more amino-acids, especially at least 30 more amino-acids; and wherein the fusion protein induces sprouting in a spheroid-based in-vitro angiogenesis assay compared to an uninduced control.

In another preferred embodiment of the present, at least one amino-acid residue, the N-terminus and/or the C-terminus of the protein is chemically modified by a modification other than glycosylation: especially by pegylation, biotinylation, alkylation, hydroxylation, adenylation, phosphorylation, succinylation, oxidation, or acylation, in particular acetylation. This can improve the pharmaceutical properties (such as solubility, half-life, activity) of the protein of the present invention.

It is advantageous to provide the protein of the present invention in a pharmaceutical composition, for instance to increase the stability of the protein in storage. Consequently, another preferred embodiment provides a pharmaceutical composition comprising the protein of the invention, further comprising one or more excipients.

The term "pharmaceutical composition" refers to any composition or preparation that contains a protein, as defined above, which ameliorates, cures or prevents the conditions described herein. In particular, the expression "a pharmaceutical composition" refers to a composition comprising a protein according to the present invention and a pharmaceutically acceptable carrier or excipient (both terms are used interchangeably). Suitable carriers or excipients are known to the person skilled in the art, for example saline, Ringer's solution, dextrose solution, buffers, Hank solution, vesicle forming compounds (e.g. lipids), fixed oils, ethyl oleate, 5% dextrose in saline, substances that enhance isotonicity and chemical stability, buffers and preservatives. Other suitable carriers include any carrier that does not itself induce the production of antibodies in the patient that are harmful for the patient. Examples are well tolerable proteins, polysaccharides, polylactic acids, polyglycolic acid, polymeric amino acids and amino acid copolymers. This pharmaceutical composition or the protein of the present invention can (as a drug) be administered via appropriate procedures known to the skilled person to a patient in need thereof (i.e. a patient having or having the risk of developing the diseases or conditions mentioned herein). The patient is a vertebrate, preferably a mammal, especially a human.

A preferred route of administration is parenteral administration, in particular through intravenous administration. Other preferred routes are intracoronary application via catheter or intramyocardial injection. For these routes of administration, the pharmaceutical composition of the present invention is provided in injectable dosage unit form, eg as a solution, suspension or emulsion, formulated in conjunction with the above-defined pharmaceutically acceptable excipients. The dosage and method of administration, however, depends on the individual patient to be treated.

Another preferable mode of administration is via a slow release form utilising biomaterials. For instance, processed human CYTL1 (SEQ ID NO: 28) has a predicted pI of 8.22, hence presumably carrying a net positive charge at physiological pH. The same applies to other CYTL1 proteins. Therefore, CYTL1 protein can be provided conjugated to a biocompatible matrix itself having negative charge at physiological pH, such as a sulfated polysaccharide, in particular alginate sulfate or hyaluronan sulfate. General principles of such a release system is for instance disclosed in the WO 2001/066164 A1 or the WO 2007/043050 A2. Slow release forms can also use modified forms of CYTL1 protein, e.g. to provide an even stronger positively charged CYTL1 protein for slower release conjugated to said negatively charged matrix. Methods to render a protein more positively charged are known in the art. For instance, Asp and Glu amino acid residues can either be mutated to other amino-acids or chemically modified (e.g. by reactions involving a carboxyl-reactive compound such as a carbodiimide). Alternatively or additionally, positively charged amino-acid residues or modifications can be introduced. One of skill will modify non-conserved residues (as known from sequence alignments of CYTL1 sequences provided herein and/or known at the time of filing) and then test the activity of the modified CYTL1 protein in the spheroid-based in-vitro angiogenesis assay (or sprouting assay) disclosed herein in order to avoid reducing said activity.

In general, the protein according to the present invention is administered at a dose of between 1 µg/kg and 10 mg/kg, more preferably between 10 µg/kg and 5 mg/kg, most preferably between 0.1 and 2 mg/kg. Preferably, the composition will be administered as a bolus dosage intracoronarily via catheter or intramyocardial injection. Also continuous infusion can be applied. In this case, the protein is delivered at a dose of 5 to 20 µg/kg/minute, more preferably 7-15 µg/kg/minute infusion.

As the preferred mode of administration is parenteral administration, the pharmaceutical composition according to the present invention is preferably liquid or ready to be dissolved in liquid such sterile, de-ionised or distilled water or sterile isotonic phosphate-buffered saline (PBS). Preferably, 1000µg (dry-weight) of such a composition comprises or consists, especially consists, of 1-990µg, preferably 100-900µg, more preferably 500-800µg CYTL1 protein according to the present invention, and optionally 1-500µg, preferably 1-100µg, more preferably 5-15µg (buffer) salts (preferably to yield a isotonic buffer in the final volume), and optionally 1-999µg, preferably 100-800µg, more preferably 200-600µg other excipients. Preferably, 1mg of such a dry composition is dissolved in sterile, de-ionised/distilled water or sterile isotonic phosphate-buffered saline (PBS) to yield a final volume of 0.1-100ml, preferably 0.5-20ml, more preferably 1-10ml. Preferably, in this pharmaceutical composition, the CYTL1 protein of the present invention is conjugated to a biocompatible matrix, such as a polysaccharide, in particular a sulfated polysaccharide, especially alginate sulfate or hyaluronan sulfate; preferably in a slow release form (this is also described in detail a few paragraphs above; see also the WO 2001/066164 A1 or the WO 2007/043050 A2 for such biocompatible materials for protein delivery).

In another aspect of the present invention, the protein or composition of the present invention is used in therapy, especially in the treatment of myocardial infarction, as it was surprisingly found that the protein improves recovery after myocardial infarct in a rat model (Fig. 9).

Furthermore, in another embodiment, the protein or composition of the present invention is preferably used in treatment or prevention of ischemia, especially ischemic cardiomyopathy preferably related to myocardial infarction, as it was found that the protein of the present invention induces vessel formation in vivo (Fig. 8).

This is also the reason why the protein or composition of the present invention is preferably used in treatment or prevention of tissue damage treatable by administration of CYTL1, in another embodiment. For instance, such tissue damage can occur, in the heart, e.g. due to loss of capillaries in the diabetic heart or after myocardial infarction, in ischemic cardiomyopathy, or in non-ischemic cardiomyopathy (e.g. caused by viral infection) or, in the lung, e.g. due to conditions such as acute lung injury (ACL) or in peripheral ischemia due to loss of vascularization or in retinopathy including diabetic retinopathy.

In another preferred embodiment of the present invention, the therapy of the present invention is conducted in combination with administration of endothelial colony forming cells (ECFCs) or other cells that promote vascularisation of tissues. This could increase the beneficial effect of the therapy.

In yet another aspect of the present invention, a method for manufacturing a CYTL1 protein is provided (as exemplified in Example 2). This method comprises
- recombinant expression of the protein, preferably in 293 HEK cells; and
- purification of the protein, preferably by affinity purification.

Recombinant protein expression and protein purification methods are well-known in the art.

Preferably, the method further comprises cleavage of a signal peptide from the protein and, preferably, secretion of the protein from the cells. This simplifies the purification of the protein, as the cells would not have to be lysed.

In another preferred embodiment of the method of the present invention, the protein further comprises a cleavable or non-cleavable affinity tag, preferably a FLAG tag; and preferably the tag is cleaved from the protein. An affinity tag aids in purification of the protein and allows for affinity purification such as affinity (column) chromatography. Affinity tags are known in the art, and comprise polyhistidine-tags, maltose-binding protein tags, glutathione-S-transferase tags and the FLAG octapeptide (or FLAG tag). The FLAG tag is disclosed in US patent No. 4,703,004.

Despite the protein of the present invention being exceptionally well-suited for specific therapy according to the invention, any CYTL1 protein is suitable for such therapy (yet to a significantly lesser extent), which was previously unknown.

Therefore, another preferred embodiment of the present invention provides a CYTL1 protein, or a functional fragment protein thereof or a functional fusion protein thereof, for use in treatment of myocardial infarction, wherein said protein, fragment protein or fusion protein induces sprouting in a spheroid-based in-vitro angiogenesis assay compared to an uninduced control.

Such CYTL1 proteins *for use* comprise SEQ ID Nos.: 1-53, especially SEQ ID Nos.: 1 and 28, and the following UniProt entries: Q9NRR1, G1S206, F7CJ05, UPI00027F32F6, H2QP59, G3QV27, H2PCW0, G7MSJ5, G7P588, S7MTB0, UPI0002C2DFE8, UPI0002BCE03B, G3WH39, UPI00027FA17A, A1E5L0, Q6P536, Q0VG51, B5DFG7, UPI00033447B9, UPI00033193AE, U6D578, G3TD71, UPI00022346AE, H0ZI47, UPI000194C372, UPI00032AE38E, UPI00038EFAD7, F1P7S3, UPI000359D860, UPI0003C488C0, UPI000388B5C7, K7GER1, UPI00042BCFB6, H0WPN0, UPI00027423DC, L5LQ03, UPI000333DDDA, H0VBW2, UPI00022B6D40, UPI000328A678, UPI000328E1D4, UPI00038C4288, W5PBC3, UPI00029D7CC2, E1BN89, L8IQ55, UPI0003AF8B30, UPI0003C10549, UPI00022F3975, G3GWE1, UPI000333186A, M3W6F7, UPI0000F5A458, UPI00042BCEBF, M3Y970, D2HLS4, G1M521, UPI0001DEB975, UPI0002C55126, UPI0003EE2687, UPI000399217F, UPI0003833174, UPI000386FA6F, F6RIT6, G1T5G9, G5B313, UPI0003500720, U3I8P9, R0JVB5, UPI0003509675, UPI0003C259FF, F1NSV3, G1NJB0, UPI0002035275, I3LW41, in addition to proteins with a sequence identity thereto of more than 80%, preferably more than 90%, more preferably more than 95%, even more preferably more than 98%.

Along similar lines, another preferred embodiment of the present invention provides a CYTL1 protein, or a functional fragment protein thereof or a functional fusion protein thereof, for use in treatment or prevention of ischemia, especially ischemic cardiomyopathy preferably related to myocardial infarction, wherein said protein, fragment protein or fusion protein induces sprouting in a spheroid-based in-vitro angiogenesis assay compared to an uninduced control. For instance, such tissue damage can occur, in the heart, e.g. due to loss of capillaries in the diabetic heart or, in the lung, e.g. due to conditions such as acute lung injury (ACL).

Further along similar lines, yet another preferred embodiment of the present invention provides a CYTL1 protein, or a functional fragment protein thereof or a functional fusion protein thereof, for use in treatment or prevention of tissue damage, preferably non-ischemic cardiomyopathy, wherein said protein, fragment protein or fusion protein induces sprouting in a spheroid-based in-vitro angiogenesis assay compared to an uninduced control.

Further along those lines, yet another preferred embodiment of the present invention provides a CYTL1 protein, or a functional fragment protein thereof or a functional fusion protein thereof, for use in therapy, wherein the therapy is conducted in combination with administration of endothelial colony forming cells (ECFCs) and/or other cells that promote vascularisation of tissues, and wherein said protein, fragment protein or fusion protein induces sprouting in a spheroid-based in-vitro angiogenesis assay compared to an uninduced control.

In the context of the present invention, a CYTL1 protein (or a fusion protein thereof or fragment protein thereof) of the present invention, or a CYTL1 protein (or a fusion protein thereof or fragment protein thereof) for inventive use, induces sprouting (also called sprouting activity herein) in a spheroid-based in-vitro angiogenesis assay (Korff & Augustin, JCB 1998, PMID: 9832561; T.Korff, in: Methods in Endothelial Cell Biology, ed. H.Augustin, Springer Verlag, 2004, chapter 12, p. 115; see also Example 1) compared to an uninduced control, preferably at least 25% more, more preferably at least 50% more, especially at least 100% more or even at least 200% more.

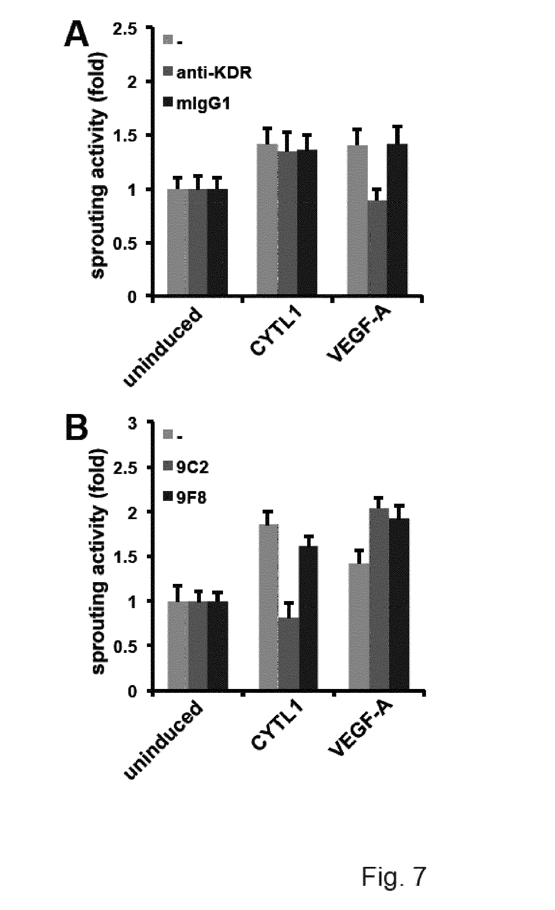

Many tumours depend on angiogenesis for their growth. Monoclonal antibodies against VEGF such as Bevacizumab (Avastin®) have been shown to be successful against cancers such as colon cancer, lung cancer and breast cancer in clinical trials and are approved for cancer therapy by the US-FDA and the EMA. In the course of the present invention, it was shown that CYTL1 is induced in endothelial cells by hypoxia and mediates sprouting *independently* of VEGF-A / VEGFR2 signaling (Fig. 7). Conditions inside tumours are often hypoxic. Therefore, a therapy with an anti-CYTL1 antibody or a CYTL1 competitor also promises success in the treatment of cancer, in particular cancer of the bladder, breast, colon, endometrium, kidney, lung, skin, pancreas, prostate, thyroid or central nervous system (such as neuroblastoma or glioma), either alone or in combination with a VEGF inhibitor or anti-VEGF antibody such as Bevacizumab or with another compound suitable for the treatment of cancer. Therapy with a anti-CYTL1 antibody or a CYTL1 competitor will be especially useful in cases where the cancer is resistant (or becomes resistant) to VEGF-inhibition, as CYTL1-induced angiogenesis is independent of VEGF-A / VEGFR2 signaling, which was found during the present invention.

Consequently, another preferred embodiment of the present invention provides an anti-CYTL1 antibody, or a functional fragment thereof, for use in treatment of cancer, in particular cancer of the bladder, breast, colon, endometrium, kidney, lung, skin, pancreas, prostate, thyroid or central nervous system (such as neuroblastoma or glioma), wherein tumour angiogenesis is inhibited, preferably administered in combination with other angiogenesis inhibitors such as Bevacizumab, and wherein said antibody inhibits CYTL1-induced sprouting in a spheroid-based in-vitro angiogenesis assay in the presence of CYTL1, compared to an uninhibited control. Preferably the antibody CYTL1 antibody prevents binding of CYTL1 to the CYTL1 receptor. Methods to raise antibodies and manufacture monoclonal antibodies are well-known in the art. Functional fragments of antibodies are fragments that are also able to bind the respective antigen. They may offer advantages over the full-size antibody due to considerations such as chemical stability, pharmaceutical half-life, dosing or simpler production. Such fragments are for instance Fab fragments, single-domain antibodies and binding domains derived from the constant region of an antibody (such as an Fcab™). An overview over such engineered antibody fragments that are suitable in the present invention is given in Hollinger & Hudson (Nat Biotechnol. 2005, PMID: 16151406).

In general, screening of chemical compound libraries is also known in the art. In this particular case, however, the present invention provides a novel screening method: One screens a chemical library for CYTL1 competitors (or to verify antibodies raised against CYTL1 or functional fragments thereof) in a spheroid-based in-vitro angiogenesis assay, in the presence of CYTL1. If the sprouting is decreased compared to an uninduced control (addition of CYTL1 without antibodies/competitors), one may have a viable clinical candidate for cancer therapy. Preferably the CYTL1 competitor directly binds CYTL1 or the CYTL1 receptor, thereby preventing CYTL1-mediated angiogenesis. Thus, another embodiment of the present invention provides a method to screen for CYTL1 competitors, comprising:
- providing a chemical compound library; and
- providing CYTL1, preferably the protein of the invention, especially glycosylated protein with SEQ ID NO: 28; and
- providing cells, preferably HUVECs; and
- individually for each compound of the library: bringing the compound into contact with cells and CYTL1 ("a sample"); and
- performing a spheroid-based in-vitro angiogenesis assay for each sample and compare it to a negative control, preferably the cells in the presence of CYTL1 without any of the compounds; and
- identifying as positive hit (and hence possible preclinical or clinical candidate) the compound of a sample, which sample has reduced sprouting compared to the negative control, preferably more than 10% reduction, more preferably more than 25% reduction, especially more than 50% reduction.

Yet another preferred embodiment of the present invention provides a CYTL1 competitor (which can be a product of the screening method above) for use in treatment of cancer, in particular cancer of the bladder, breast, colon, endometrium, kidney, lung, skin, pancreas, prostate, thyroid or central nervous system (such as neuroblastoma or glioma), wherein tumour angiogenesis is inhibited, preferably administered in combination with other angiogenesis inhibitors such as Bevacizumab, and wherein said competitor inhibits CYTL1-induced sprouting in a spheroid-based in-vitro angiogenesis assay in the presence of CYTL1, compared to an uninhibited control. Preferably the CYTL1 competitor directly binds CYTL1 or the CYTL1 receptor, thereby preventing CYTL1-mediated angiogenesis.

In yet another aspect of the present invention, a method to identify ECFCs, and a method of purifying or enriching for ECFCs, is provided.

As CYTL1 was found herein to be the first reliable marker for ECFCs, one preferred embodiment provides a method to identify endothelial colony-forming cells (ECFCs), comprising:
- providing or isolating one or more cells, preferably from cultured cells or from a biological sample (preferably outside of the human body) such as peripheral blood.
- measuring levels of one or more selected from the group of CYTL1 mRNA inside the cells, CYTL1 protein inside the cells and secreted CYTL1 protein. Methods for measuring mRNA/protein levels are known in the art. In the case of mRNA, reverse transcription polymerase chain reaction (RT-PCR) or Northern blotting is a widely used method. In the case of protein, enzyme-linked immunosorbent assay (ELISA) or Western blotting is widely used.
- comparing the measured levels to levels of a control cell population. Such a control population could for instance consist of HUVECs.
- identifying the cells as ECFCs if the measured levels are higher than the control levels, preferably at least 2 times higher, more preferably at least 5 times higher, even more preferably at least 10 times higher, in particular at least 20 times higher.

In the context of this method, it is preferred that the CYTL1 mRNA codes for a protein sequence or the CYTL1 protein comprises a protein sequence identical to at least 20, preferably at least 50, more preferably at least 100, especially all, of the amino-acids of the entire sequence of an entry selected from the group of the following UniProt entries:
Q9NRR1, G1S206, F7CJ05, UPI00027F32F6, H2QP59, G3QV27, H2PCW0, G7MSJ5, G7P588, S7MTB0, UPI0002C2DFE8, UPI0002BCE03B, G3WH39, UPI00027FA17A, A1E5L0, Q6P536, Q0VG51, B5DFG7, UPI00033447B9, UPI00033193AE, U6D578, G3TD71, UPI00022346AE, H0ZI47, UPI000194C372, UPI00032AE38E, UPI00038EFAD7, F1P7S3, UPI000359D860, UPI0003C488C0, UPI000388B5C7, K7GER1, UPI00042BCFB6, H0WPN0, UPI00027423DC, L5LQ03, UPI000333DDDA, H0VBW2, UPI00022B6D40, UPI000328A678, UPI000328E1D4, UPI00038C4288, W5PBC3, UPI00029D7CC2, E1BN89, L8IQ55, UPI0003AF8B30, UPI0003C10549, UPI00022F3975, G3GWE1, UPI000333186A, M3W6F7, UPI0000F5A458, UPI00042BCEBF, M3Y970, D2HLS4, G1M521, UPI0001DEB975, UPI0002C55126, UPI0003EE2687, UPI000399217F, UPI0003833174, UPI000386FA6F, F6RIT6, G1T5G9, G5B313, UPI0003500720, U3I8P9, R0JVB5, UPI0003509675, UPI0003C259FF, F1NSV3, G1NJB0, UPI0002035275, I3LW41. These entries cluster with 50% sequence identity around Q9NRR1 (UniRef entry UniRef50_Q9NRR1) and are therefore extremely likely to be CYTL1 proteins as well.

In the context of this method, it is especially preferred that the CYTL1 mRNA codes for a protein sequence or the CYTL1 protein comprises a protein sequence identical to at least 20, preferably at least 50, more preferably at least 100, especially all, of the amino-acids of the entire sequence to UniProt entry Q9NRR1, and that the cells are human. It is particularly preferred that he CYTL1 mRNA codes for the protein sequence SEQ ID NO: 1 or the CYTL1 protein sequence comprises or consists, especially consists, of the protein sequence SEQ ID NOs: 1 or 28.

In the context of this method, it is further preferred that the cells are also classified in regard to whether they are CD31-positive and/or CD34-positive, in addition to being CYTL1 positive (as described above). CD31 and CD34 are markers of endothelial cells (see for instance Pusztaszeri et al, JHC, 2006, PMID: 16234507). Therefore, if the cells are positive for one or both of these markers, this increases the reliability of the cells being identified as ECFCs. How to test for these markers is well known in the art, for instance see Pusztaszeri et al. As monoclonal antibodies for both markers are commercially available (for instance from Thermo Scientific, Sigma-Aldrich or Millipore), Western Blot or ELISA are also suitable to identify CD31 and/or CD34 positive cells.

In another preferred embodiment of the present invention, the protein level is measured by means of an anti-CYTL1 antibody or fragment thereof or a molecule capable of essentially selectively binding the CYTL1 protein, preferably in a Western blot or an ELISA.

In another preferred embodiment of the present invention, the mRNA level is measured by means of a RNA molecule or DNA molecule capable of essentially selectively binding the RNA, preferably in a Northern Blot, a micro-array or an RT-PCR.

As CYTL1 was found herein to be the first reliable marker for ECFCs, another preferred embodiment provides a method of purifying or enriching for endothelial colony-forming cells (ECFCs), comprising:
- using the method of the present invention to identify ECFCs from a cell population
- purifying or enriching for the identified ECFCs.

In the context of this method, it is further preferred that the cells are also enriched for being CD31-positive and/or CD34-positive, in addition to being CYTL1 positive (as described above). CD31 and CD34 are markers of endothelial cells (see for instance Pusztaszeri et al, JHC, 2006, PMID: 16234507). Therefore, if the cells are positive for one or both of these markers, this increases the reliability of the cells being enriched are ECFCs. How to test for these markers is well known in the art, for instance see Pusztaszeri et al. As monoclonal antibodies for both markers are commercially available (for instance from Thermo Scientific, Sigma-Aldrich or Millipore), FASC or MACS are also suitable to enrich for CD31 and/or CD34 positive cells.

As CYTL1 was found herein to be the first reliable marker for ECFCs, another preferred embodiment provides a method of purifying or enriching for endothelial colony-forming cells (ECFCs), comprising:
- introducing into cells a reporter gene construct operatively linked to the endogenous CYTL1 gene, preferably by replacing the endogenous CYTL1 gene by a CYTL1-reporter gene fusion under the endogenous CYTL1 promoter or by having a reporter gene under the control of the endogenous CYTL1 promoter, preferably by transient transfection of total monocnuclear cells from peripheral blood (PBMCs) or cord blood (CBMCs) or CD34+ cells from blood or bone marrow
- providing or isolating the cells
- purifying or enriching for the cells having reporter gene construct expression or an increased reporter gene construct expression level compared to a control level.

Preferably, the endogenous CYTL1 gene codes for a protein selected from the group of the following UniProt entries: Q9NRR1, G1S206, F7CJ05, UPI00027F32F6, H2QP59, G3QV27, H2PCW0, G7MSJ5, G7P588, S7MTB0, UPI0002C2DFE8, UPI0002BCE03B, G3WH39, UPI00027FA17A, A1E5L0, Q6P536, Q0VG51, B5DFG7, UPI00033447B9, UPI00033193AE, U6D578, G3TD71, UPI00022346AE, H0ZI47, UPI000194C372, UPI00032AE38E, UPI00038EFAD7, F1P7S3, UPI000359D860, UPI0003C488C0, UPI000388B5C7, K7GER1, UPI00042BCFB6, H0WPN0, UPI00027423DC, L5LQ03, UPI000333DDDA, H0VBW2, UPI00022B6D40, UPI000328A678, UPI000328E1D4, UPI00038C4288, W5PBC3, UPI00029D7CC2, E1BN89, L8IQ55, UPI0003AF8B30, UPI0003C10549, UPI00022F3975, G3GWE1, UPI000333186A, M3W6F7, UPI0000F5A458, UPI00042BCEBF, M3Y970, D2HLS4, G1M521, UPI0001DEB975, UPI0002C55126, UPI0003EE2687, UPI000399217F, UPI0003833174, UPI000386FA6F, F6RIT6, G1T5G9, G5B313, UPI0003500720, U3I8P9, R0JVB5, UPI0003509675, UPI0003C259FF, F1NSV3, G1NJB0, UPI0002035275, I3LW41, especially for the protein of UniProt entry Q9NRR1 and the cells are preferably human.

Also preferably, the reporter gene construct comprises a DNA sequence coding for a fluorescent protein, preferably GFP, RFP or YFP, or for a receptor or any other protein (partially) localised on the cell surface which could then by bound by a fluorescent or magnetic nanoparticle-conjugated antibody specific for said receptor or protein.

Furthermore, the cells having reporter gene construct expression or an increased reporter gene construct expression level compared to a control level, preferably at least 2 times higher, more preferably at least 5 times higher, even more preferably at least 10 times higher, in particular at least 20 times higher, are preferably purified or enriched for by flow cytometry, especially by fluorescence-activated cell sorting (FACS) or magnetic-activated cell sorting (MACS).

The product of such a method (i.e. reliably identified ECFCs) is preferably used in therapy, optionally in combination with the administration of the protein or composition of the present invention, preferably for treatment of myocardial infarction or treatment or prevention of ischemia, especially ischemic cardiomyopathy preferably related to myocardial infarction, or tissue damage.

Herein, "sprouting" (also called "sprouting activity"), especially in the context of the expression "induces sprouting", refers to sprouting activity as measurable by applying the protocol detailed in Example 1 (spheroid-based in-vitro angiogenesis assay).

Herein, "UniProt entry" refers to entries in the Universal Protein Resource. Entries are identified by their accession codes, usually a code of six alphanumeric letters (e.g. "Q9NRR1"). UniProt accession codes that start with "UPI" and are longer than six alphanumeric letters are UniProt sequence archive (UniParc) entries (e.g. "UPI00027F32F6").

UniProt is a comprehensive resource for protein sequence and annotation data. UniProt is a collaboration between the European Bioinformatics Institute (EMBL-EBI), the SIB Swiss Institute of Bioinformatics and the Protein Information Resource (PIR). Across the three institutes more than 100 people are involved through different tasks such as database curation, software development and support. Website: http://www.uniprot.org/

If not specified otherwise, "UniProt entry" or "UniProt protein entry" means an entry in the Protein Knowledgebase (UniProtKB) of UniProt or Sequence Archive of UniProt (UniParc). Sequence cluster entries (UniRef) are referred to as "UniProt UniRef entry" or "UniProt sequence cluster entry". If not stated otherwise, the UniProt database state for all entries referenced herein is of 5 May 2014 (UniProtKB Release 2014_04 and UniParc Release 2014_04, respectively).

In the context of the present application, sequence variants (designated as "natural variant" in UniProt) are expressedly included when referring to a UniProt entry. In the case of Q9NRR1, this comprises the SNPs resulting in the mutations: S51L or R136C.

"Percent (%) amino acid sequence identity" with respect to a reference polypeptide or protein sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN, ALIGN-2, Megalign (DNASTAR) or the "needle" pairwise sequence alignment application of the EMBOSS software package. Those skilled in the art can determine appropriate parameters for aligning sequences, including any algorithms needed to achieve maximal alignment over the full length of the sequences being compared. For purposes herein, however, % amino acid sequence identity values are calculated using the sequence alignment of the computer programme "needle" of the EMBOSS software package (publicly available from European Molecular Biology Laboratory; Rice et al., EMBOSS: the European Molecular Biology Open Software Suite, Trends Genet. 2000 Jun;16(6):276-7, PMID: 10827456).

The needle programme can be accessed under the web site http://www.ebi.ac.uk/Tools/psa/emboss_needle/ or downloaded for local installation as part of the EMBOSS package from http://emboss.sourceforge.net/. It runs on many widely-used UNIX operating systems, such as Linux.

To align two protein sequences, the needle programme is preferably run with the following parameters:
Commandline: needle -auto -stdout -asequence SEQUENCE_FILE_A -bsequence SEQUENCE_FILE_B -datafile EBLOSUM62 -gapopen 10.0 -gapextend 0.5 -endopen 10.0 -endextend 0.5 -aformat3 pair -sprotein1 -sprotein2 (Align_format: pair Report_file: stdout)

The % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program needle in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. In cases where "the sequence of A is more than N% identical to the *entire* sequence of B", Y is the *entire* sequence length of B (i.e. the entire number of amino acid residues in B). Unless specifically stated otherwise, all % amino acid sequence identity values used herein are obtained as described in the immediately preceding paragraph using the needle computer program.

The invention is further described by the following examples and the drawing figures, yet without being limited thereto.
Figure 1: ECFCs display a high sprouting capacity.
   A. Differential expression of mRNAs for surface markers of ECFCs and HUVECs. ECFCs and HUVECs were grown to density, total RNA was isolated and subjected to realtime RT-PCR as described in Example 7. Obtained values were normalized to beta-actin mRNA levels and the data displayed as fold difference in comparison to HUVECs. HUVEC values were arbitrarily set to 1. Results were calculated using different cell batches isolated from a minimum of six donors and are displayed as mean +/-SEM.
   B-D. Analysis of the sprouting activities of ECFCs and HUVECs. Spheroids were generated from ECFCs and HUVECs, embedded in collagen gels, overlayed with medium without or containing VEGF-A and incubated overnight. Then pictures were taken for analysis. B. Examples of the spheroid sprouting assay obtained with ECFCs and HUVECs. Bar indicates 200 µm. C. Increased sprouting capacity of ECFCs in comparison to HUVECs. Cumulative sprout length was determined for a minimum of 20 spheroids per condition. Data are displayed as relative difference in sprout length of ECFCs compared to HUVECs. Data for HUVECs were arbitrarily set to 1. Results shown are calculated from four independent experiments using cells from different donors and displayed as mean +/- SEM. D. ECFCs induce sprouting of HUVECs. Spheroids were formed from mixtures of 5% of ECFCs and 95% of HUVECs and analyzed as described above. Data are displayed as relative difference in sprout length of spheroids composed of 5% ECFCs / 95% HUVECs compared to HUVECs without addition of ECFCs. Data for HUVECs without addition of ECFCs were arbitrarily set to 1. Results are calculated from four independent experiments using cells from different donors and displayed as mean +/- SEM. *** p<0.005
Figure 2: CYTL1 is preferentially expressed in ECFCs and mediates increased sprouting activity.
   A. Increased expression of CYTL1 mRNA in ECFCs in comparison to HUVECs. Total RNA isolated from ECFCs and HUVECs were subjected to realtime RT-PCR, values normalized to beta-actin mRNA and the data displayed as fold difference in comparison to HUVECs. HUVEC values were arbitrarily set to 1. Results of cell batches isolated from five different donors are displayed as mean +/- SD. ** p<0.01
   B-D. Reduction of sprouting activities by knockdown of CYTL1. ECFCs were transduced with lentiviruses expressing either shRNA for CYTL1 (shCYTL1) or a control shRNA (shneg). Transduced cells were selected with puromycin and used for analysis 72 h after transduction. B. Reduction of sprouting activity of ECFCs. Spheroids were formed from the transduced ECFCs and analyzed as described in figure 1. Control shRNA transduced samples were arbitrarily set to 1. Data are calculated from cell samples obtained from three different donors and displayed as mean +/-SEM. C. Reduction of induced sprouting activity in mosaic spheroids. Spheroids generated from 5% transduced ECFCs and 95% HUVECs were analyzed. Data for control HUVECs without addition of ECFCs were arbitrarily set to 1. Results for three independent experiments using cells from different donors are displayed as mean +/- SEM. D. Knockdown of CYTL1 mRNA in ECFCs. For control purposes the knockdown of CYTL1 mRNA was confirmed for transduced cells used in parallel for the spheroid assay. Realtime RT-PCR was performed using total RNA and the values obtained for control shRNA transduced cells set to 1. Data are calculated from three independent experiments and displayed as mean +/-SD. ** p<0.01, *** p<0.005. Scale bar is 200µm.

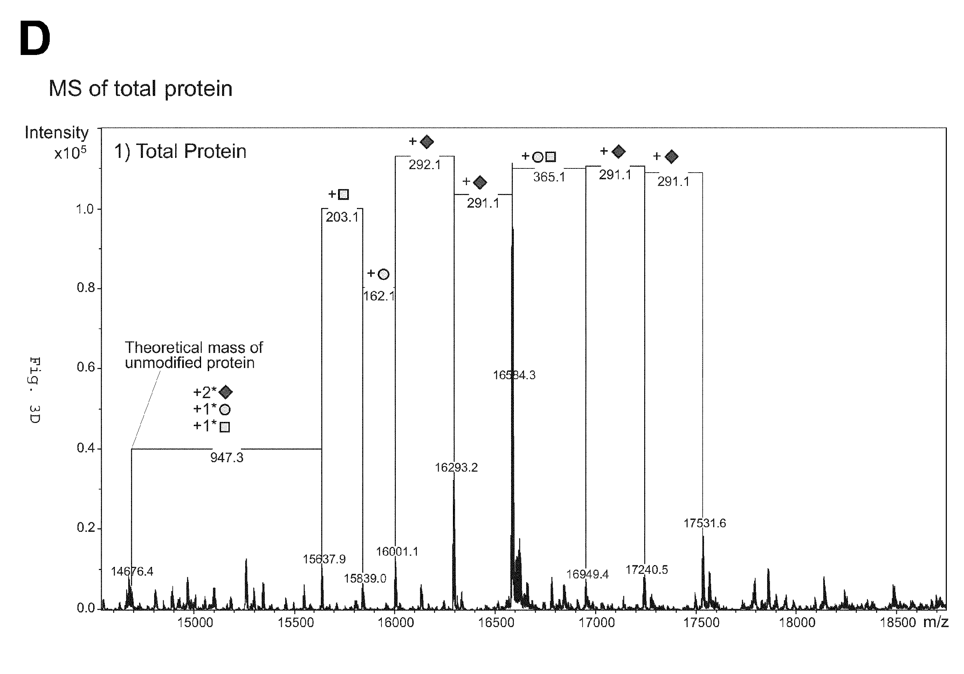

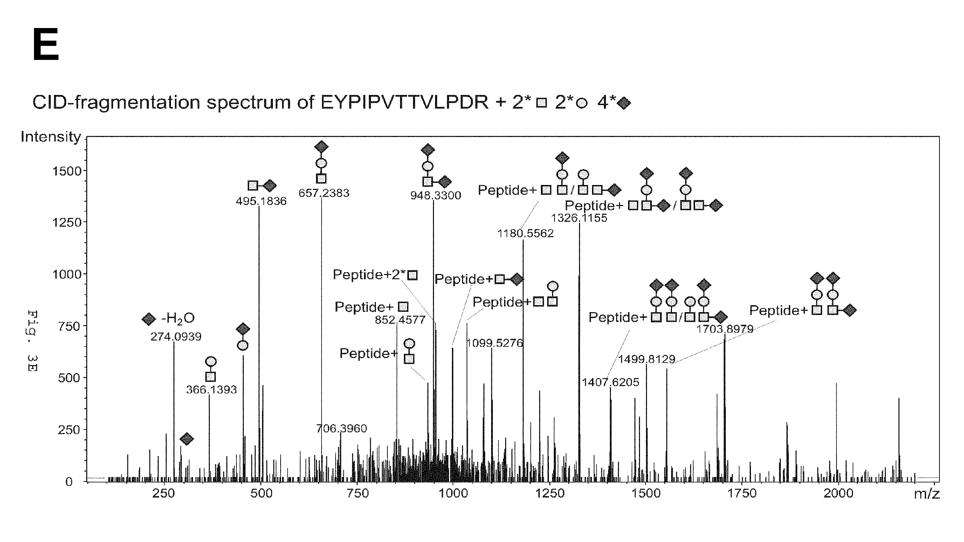

Figure 3. Modification of secreted CYTL1.
   A. Molecular weight of modified and non-modified CYTL1. HEK293 cells were transfected with an expression construct containing the complete coding region of CYTL1 cDNA fused with a three Flag-tags encoding sequence. Stably expressing recombinant HEK293 cells were selected with G418. Supernatants were produced from dense cell cultures in serum-free Opti-MEM for 48 h. Then supernatants were collected and the pelleted cells lysed in Laemmli sample buffer. SDS-PAGE of samples of the supernatants or cell lysates was performed and subjected to Western blot analysis using an antibody against the Flag-tag. A molecular weight difference of approximately 2 kDa was observed showing that the protein is modified upon secretion.
   B. Comparison of molecular weights of CYTL1 produced by HUVECs, ECFCs or HEK293. HUVECs, ECFCs and HEK293 were infected with an adenovirus encoding Flag-tagged CYTL1, supernatants and cell lysates were produced as described above and subjected to SDS-PAGE. Western blot analysis of lysates (intracellular CYTL1) and supernatants (secreted CYTL1) was performed detecting CYTL1 with an antibody against the Flag-tag.
   C. Identification of the molecular structure of the modification of secreted CYTL1 by mass spectroscopy. For larger scale CYTL1 production a variant HEK293 cell stably transfected with a construct providing for six C-terminal His-tags was generated and cell supernatant produced. Secreted CYTL1 was isolated via the His-Tag using a NiNTA column and the purified protein subjected to proteolytic (tryptic) digestion. The amino acid sequence was confirmed and the partially cleaved C-terminal peptide EYPIPVTTVLPDR (boxed in dark grey; SEQ ID NO: 82) was identified as O-linked glycopeptide. The spectrum clearly showed the presence of 1 or 2 carbohydrate moieties each consisting of 1 HexNAc + 1 Hex + 2 Neu5Ac for the peptide with the indicated amino acid sequence (identification by MSMS), indicating (V)TT(V) as the sites of glycosylation. In addition, the N-terminal fragment TPPTCYSR (SEQ ID NO: 83) was found to be glycosylated too, but only to a negligible extent. The glycoforms with 1 HexNAc + 1 Hex + 1 Neu5Ac as well as 1 HexNAc + 1 Hex + 2 Neu5Ac were detected with 1:100 and 1:200 intensities compared to the unmodified TPPTCYSR. The exact glycosylation sites of this fragment could not be identified as the signals were too weak to perform ETD fragmentation. The depicted protein sequence is SEQ ID NO: 28.
   D. MS of total protein identifying the exact molecular weights of the modified total protein.
   E. CID-fragmentation spectrum of the glycopeptide EYPIPVTTVLPDR (SEQ ID NO: 82) identifying the exact location of the carbohydrate moieties.
Figure 4. Recombinant CYTL1 induces sprouting comparable to VEGF-A, but does not display an inflammatory response.
   A. Dose response curve for sprouting stimulated by CYTL1 in comparison to VEGF-A. HUVECs were used in the spheroid sprouting assay and induced with increasing concentrations of recombinant CYTL1 and VEGF-A. Cumulative sprout length was determined and is displayed mean +/- SD (relative units, 0ng/ml CYTL1 is set to 1). Data of one representative experiment out of three performed are shown.
   B. Comparison of recombinant CYTL1 forms isolated from the cell culture supernatant or from cell lysates. HUVEC spheroids were induced with the secreted or intracellular forms of recombinant CYTL1 or VEGF-A (25 ng/ml each). Obtained cumulative sprout lengths are displayed as mean +/-SD calculated from one representative experiment out of three performed (relative units, uninduced is set to 1). *** p<0.005
   C-D. Lack of induction of upregulation of inflammatory VCAM1 and E-selectin expression. HUVECs were grown to density, starved overnight and treated with IL-1 (100 ng/ml), VEGF-A (30 ng/ml) or recombinant CYTL1 (20 ng/ml) for one hour. Then RNA was isolated and realtime RT-PCR performed. Data were normalized to beta-actin mRNA and displayed as mean +/- SD from one experiment performed in triplicates. The value obtained for uninduced cells were arbitrarily set to one. ** p<0.01, *** p<0.005
Figure 5. Induction of target genes by CYTL1.
   HUVECs were grown to density, starved overnight in serum-free EBM-2 medium and induced with CYTL1 or VEGF-A. Samples were taken at the indicated time points, total RNA isolated and subjected to realtime RT-PCR. Values were normalized to beta-actin mRNA levels. Fold induction is displayed as mean +/-SD calculated from one exemplary experiment of two performed in triplicates.
   A-B. Induction of mRNAs for early response genes by CYTL1 or VEGF-A.
   C. Induction of metallothionein isoforms by CYTL1 or VEGF-A.
Figure 6. Lack of induction of VEGF-A by CYTL1 and response of VEGF-A and CYTL1 to hypoxia.
   HUVECs were grown to density, starved overnight in serum-free EBM-2 medium and induced with CYTL1 or VEGF-A or subjected to hypoxia (2% oxygen). Samples were taken at the indicated time points, total RNA isolated and subjected to realtime RT-PCR. Values were normalized to beta-actin mRNA levels. Fold induction is displayed as mean +/-SD calculated from one exemplary experiment of two performed in triplicates.
   A. Effects of CYTL1 and VEGF-A on CYTL1 and VEGF-A mRNAs.
   B. Upregulation of CYTL1 and VEGF-A mRNAs by hypoxia. * p<0.05, ** p<0.01
Figure 7. CYTL1 induces sprouting independent of VEGF-A/VEGFR-2 signaling.
   HUVECs were used to generate spheroids and the spheroid sprouting assay was performed. Sprouting was induced by CYTL1 or VEGF-A in the presence or without neutralizing antibodies to VEGFR-2 (anti-KDR) or CYTL1. The cumulative sprout lengths obtained is depicted as mean of fold induction +/-SEM.
Figure 8. CYTL1 induces vessel formation in vivo.
   Spheroids formed from HUVECs were mixed into a Matrigel/fibrin solution containing CYTL1 or VEGF-A/bFGF or without growth factors and injected subcutaneously in the flank of SCID mice. After 21 days FITC-dextran was injected via the tail vein, perfusion was allowed for 10 min and the mice sacrificed. The Matrigel/fibrin plugs were excised, fixed and embedded. Sections were generated and stained with an antibody to human CD34 as described in Example 9.
   A. Exemplary sections are depicted for nuclear staining with DAPI, for the injected FITC-dextran and for staining with human CD34. The last column displays merged images. Bar 50 µm.
   B. Statistic evaluation of microvessel density (MVD). Data are displayed as mean MVD +/-SEM calculated from three sections per plug using three plugs per condition. Shown is one exemplary experiment of two performed. * p<0.05
Figure 9. CYTL1 improves recovery after myocardial infarct in a rat model.
   Myocardial infarct was induced in male immunodeficient Nude rats by ligation of the left anterior descending artery. A CYTL1 solution (2µg/100µl/rat) or PBS as control was then injected intramyocardially within the infarcted area at several sites. One week before surgery and 2 and 28 days after surgery heart function was thoroughly investigated by sonography. After the last sonography rats were injected with heparin and custadiol and rats sacrificed. Hearts were excised, fixed and embedded in paraffin. Masson Trichrome staining was performed of sections across the whole heart.
   A. Diminished impairment of ejection fraction (EF). EF was determined by sonography. Impairment of EF monitored 2 days and 28 days after ligation was calculated as percent reduction of the baseline level following deduction of control rats undergoing sham surgery. The values obtained with rats which received PBS injection after ligation were arbitrarily set to 100 %.
   B. Scar area of left ventricle. Statistic analysis is based on six sections across the whole scar area from 4 to 5 rats displaying strong infarcts and depicted as mean+/-SD. * p < 0.05.
   C. Exemplary picture of Trichrome staining of 4 hearts each for CYTL1 or PBS injected animals.
Figure 10. In CYTL1 proteins, the two consecutive threonines close to the C-terminus are well-conserved between mammalian species. 6-letter code is the corresponding UniProt accession number. Numbers indicate position of the respective last amino-acid. All depicted sequences correspond to a C-terminal fragment of the respective protein as identified by the respective accession number, retrievable from UniProt (database release 2014_04).
Figure 11. Probable start of the processed CYTL1 protein, after cleavage of the signal peptide, in various mammalian species. 6-letter code is the corresponding UniProt accession number. Numbers indicate position of the respective last amino-acid shown. All depicted sequences correspond to a N-terminal fragment of the respective protein as identified by the respective accession number, retrievable from UniProt (database release 2014_04).

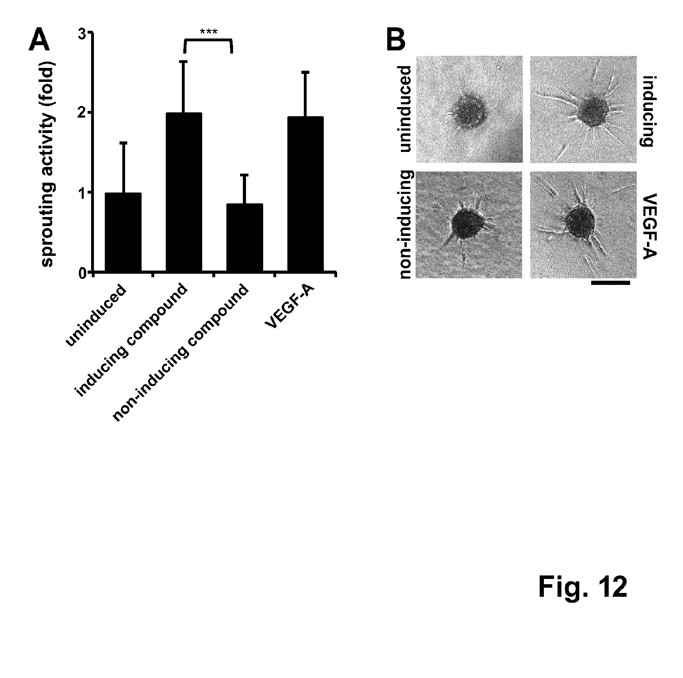

Figure 12. Exemplary expected results of a spheroid-based in-vitro angiogenesis assay as described in Example 1. A compound (e.g. the CYTL1 protein of the present invention) is tested and compared to uninduced cells (negative control) and VEGF-A-induced cells (positive control).
   A. If the compound is a sprouting-inducing ("inducing") compound, its sprouting activity will be similar to the positive control. If the compound is not a sprouting-inducing ("non-inducing") compound, its sprouting activity will be similar to the negative control. (relative units, uninduced is set to 1)
   B. To classify a compound as inducing or non-inducing, the visual examination of cells in the microscope is sufficient. If the compound is a sprouting-inducing ("inducing") compound, the number and lengths of the sprouts will be similar to the positive control. If the compound is not a sprouting-inducing ("non-inducing") compound, the number and lengths of the sprouts will be similar to the negative control. Scale bar is 200µm.

### Examples

### General Results and Discussion

### Endothelial progenitor cells display a high angiogenic sprouting capacity and stimulate sprouting of mature endothelial cells

Initially the phenotypic and functional properties of ECFCs in comparison to terminally differentiated endothelial cells of the vessel wall were characterised. For this purpose, ECFCs from human cord blood and human umbilical vein endothelial cells (HUVECs) from umbilical cords were isolated. When the mononuclear cell fraction of cord blood was plated onto collagen-coated plates, progenitor cells present in the fraction gave rise to ECFC colonies, which became first visible between day five and 22. Typically, two to five colonies were obtained from 50 ml of cord blood, with some variability depending on the donor. Cells reseeded from the colonies displayed high proliferation rates and could be expanded to large cell numbers.

Cells at low passage numbers (passage three to five) were analyzed for markers of progenitor cells and of mature endothelial cells by flow cytometry. ECFCs displayed staining for surface markers of the endothelial cell lineage such as VE-cadherin (Figure 1A) and CD31 undistinguishable from HUVEC. However, expression of c-Kit and KDR (VEGFR-2) was significantly more pronounced than for HUVEC in line with a progenitor status of ECFCs and the high proliferation rate (Figure 1A).

Functionally it was found that ECFCs to be distinguished from HUVECs by their higher potential to form tubular sprouts in vitro (Figure 1B and C). Furthermore, when small numbers of ECFCs (5%) were mixed to HUVECs and spheroids were formed of the mixture, the basic as well as the VEGF-A-induced sprouting capacity of HUVECs was surprisingly found to be significantly increased (by about 30 to 40%) to levels intermediary between ECFCs and HUVEC (Figure 1D). This showed that ECFCs trigger increased sprouting of HUVECs by secretion of molecules or cell-cell interactions.

### CYTL1 is preferentially produced by endothelial progenitor cells and mediates the increased sprouting capability

To identify the reponsible factor(s) in endothelial progenitors of the ECFC type were comparatively analyzed the gene expression profiles of ECFCs isolated from human cord blood or adult peripheral blood and vessel wall endothelial cells from human umbilical cords or adult veins. When focusing our analysis on genes for secreted proteins, mRNA for CYTL1 was more than 30-fold overrepresented in ECFCs of both sources when compared to the vessel wall endothelial types (Table 1). First the gene profiling data was reassessed by investigating the mRNA levels of CYTL1 by realtime RT-PCR using multiple isolates of ECFCs (n=6) and HUVECs (n=6) derived from different donors. Consistent with the microarray analysis CYTL1 mRNA was regularly more than 10-fold higher in ECFCs when compared to HUVECs (Figure 2A).

Table 1: ECFCs and HUVECs were cultured to density, total RNA isolated and subjected to Affymetrix microarray analysis as described in detail in Example 4. (A) Relative differences in random gene expression intensities for individual genes in ECFCs from cord blood (ECFCs CB) versus HUVECs are given. (B) displays a corresponding analysis using adult peripheral blood (ECFCs PB) and adult human venous endothelial cells (HVECs). The 20 most differentially expressed genes encoding secreted proteins are displayed.

**Table 1A**

| **UniGene ID** | **Entrez Gene** | **Gene Title** | **ECFCs CB vs. HUVECs** |
|---|---|---|---|
| Hs.13872 | 54360 | cytokine-like 1 | 33.88 |
| Hs.145586 | 1288 | collagen, type IV, alpha 6 | 14.88 |
| Hs.369397 | 7045 | transforming growth factor, beta-induced, 68kDa | 8.27 |
| Hs.25590 | 6781 | Stanniocalcin 1 | 8.20 |
| Hs.164021 | 6372 | chemokine (C-X-C motif) ligand 6 (granulocyte chemotactic protein 2) | 6.55 |
| Hs.789 | 2919 | chemokine (C-X-C motif) ligand 1 (melanoma growth stimulating activity, alpha) | 6.37 |
| Hs.474270 | 3053 | serpin peptidase inhibitor, clade D (heparin cofactor), member 1 | 4.46 |
| Hs.252820 | 5228 | placental growth factor, vascular endothelial growth factor-related protein | 3.98 |
| Hs.77274 | 5328 | plasminogen activator, urokinase | 3.80 |
| Hs.621232 | 1906 | endothelin 1 | 3.59 |
| Hs.522019 | 92949 | ADAMTS-like 1 | 3.09 |
| Hs.414795 | 5054 | serpin peptidase inhibitor, clade E (nexin, plasminogen activator inhibitor type 1), member 1 | 3.00 |
| Hs.695934 | 1305 | collagen, type XIII, alpha 1 | 2.87 |
| Hs.616962 | 9518 | growth differentiation factor 15 | 2.81 |
| Hs.943 | 9235 | interleukin 32 | 2.78 |
| Hs.624 | 3576 | interleukin 8 | 2.78 |
| Hs.387057 | 79875 | thrombospondin, type I, domain containing 4 | 2.71 |
| Hs.491582 | 5327 | plasminogen activator, tissue | 2.62 |
| Hs.656071 | 56999 | ADAM metallopeptidase with | 2.46 |
| Hs.387057 | 79875 | thrombospondin type 1 motif, 9 thrombospondin, type I, domain containing 4 | 2.33 |

**Table 1B**

| **UniGene ID** | **Entrez Gene** | **Gene Title** | **ECFCs PB vs. HVECs** |
|---|---|---|---|
| Hs.13872 | 54360 | cytokine-like 1 | 45.98 |
| Hs.145586 | 1288 | collagen, type IV, alpha 6 | 38.17 |
| Hs.248197 | 8995 | tumor necrosis factor (ligand) superfamily, member 18 | 11.85 |
| Hs.491582 | 5327 | plasminogen activator, tissue | 8.54 |
| Hs.77274 | 5328 | plasminogen activator, urokinase | 6.25 |
| Hs.695934 | 1305 | collagen, type XIII, alpha 1 | 5.58 |
| Hs.624 | 3576 | interleukin 8 transforming growth factor, | 4.75 |
| Hs.369397 | 7045 | beta-induced, 68kDa | 4.31 |
| Hs.369089 | 1287 | collagen, type IV, alpha 5 (Alport syndrome) | 4.02 |
| Hs.453951 | 3084 | neuregulin 1 | 3.90 |
| Hs.1735 | 3625 | inhibin, beta B | 3.77 |
| Hs.418367 | 10874 | neuromedin U | 3.68 |
| Hs.522019 | 92949 | ADAMTS-like 1 | 3.43 |
| Hs.624 | 3576 | interleukin 8 | 3.06 |
| Hs.1722 | 3552 | interleukin 1, alpha | 2.98 |
| Hs.9613 | 51129 | angiopoietin-like 4 | 2.88 |
| Hs.210283 | 1289 | collagen, type V, alpha 1 | 2.80 |
| Hs.656071 | 56999 | ADAM metallopeptidase with thrombospondin type 1 motif, 9 | 2.76 |
| Hs.37055 | 2250 | fibroblast growth factor 5 | 2.73 |
| Hs.34333 | 147372 | collagen and calcium binding EGF domains 1 | 2.71 |

To investigate the contribution of CYTL1 to angiogenic sprouting of endothelial cells, loss-of-function studies were performed. For this purpose, lentiviruses expressing short hairpin RNAs (shRNAs) were used to downregulate endogenous CYTL1 expression levels (cf. Examples 5 and 6). Up to 70% decrease in CYTL1 mRNA expression was achieved 48 h after infection (Figure 2D). When endogenous CYTL1 was knocked down, a strongly reduced sprouting activity was observed for ECFCs in control as well as VEGF-A induced assays (Figure 2B). These data are in line with an important role of CYTL1 for basal as well as VEGF-A-induced sprouting of ECFCs.

It was further investigated whether the influence of reducing CYTL1 expression on the capacity of ECFCs to stimulate sprouting of mature endothelial cells in a mosaic spheroid sprouting experiment. 5% of ECFCs infected with lentiviruses expressing shRNA for CYTL1 (shCYTL1) or control shRNA (shneg) were mixed to HUVECs and then the spheroid sprouting assay was performed. Indeed, addition of shCYTL1 expressing cells had a strongly diminished effect on sprouting of HUVECs when compared to shneg expressing cells (Figure 2C).

Taken together, these findings show that CYTL1 secreted by ECFCs is capable to stimulate sprouting of ECFCs in an autocrine and of mature endothelial cells in a paracrine manner. Furthermore, it shows that ECFC-secreted CYTL1 can act additively to VEGF-A.

### Secreted recombinant CYTL1 is O-glycosylated at threonine 128 and/or 129 and this modification is essential for bioactivity

Based on the evidence that CYTL1 is an important factor mediating proangiogenic functions of endothelial progenitors and working in cooperation with VEGF-A, recombinant CYTL1 was generated for further evaluation. That CYTL1 is indeed a secreted protein is shown by the fact that the sequence of murine and human CYTL1 predicts a clear signal peptide (Liu et al, 2000; Tomczak & Pisabarro, Proteins 2011, PMID: 21322034). Therefore, several expression constructs were generated, containing the full-length coding region of the cDNA fused to sequences encoding either three Flag peptides or six His-tags to be used for stable transfections and for recombinant adenovirus generation (cf. Examples 4 and 6). First HEK293 cells were stably transfected with the flag construct and the expression of the C-terminally Flag-tagged protein analyzed. High amounts of recombinant CYTL1 was detected in the supernatant as well as in cell lysates (Figure 3A). The protein secreted into the supernatant displayed a size of 17 kDa, whereas in cell lysates a 15 kDa form was detected corresponding to the size predicted for the unmodified protein. This shows that the secreted form of CYTL1 is posttranslationally modified.

To demonstrate that CYTL1 is similarly modified when synthesized in endothelial cells and their progenitors, recombinant human CYTL1 was expressed in ECFCs and HUVECs following transduction of the cells with an adenovirus encoding the Flag-tagged form of CYTL1. Again the major form in the supernatant displayed a molecular mass of approximately 17 kDa indistinguishable from the product obtained from 293 cells (Figure 3B), whereas in the cell extract only the 15 kD form was present. This showed that also in progenitors and endothelial cells an immature unmodified form of CYTL1 is present in cell lysates, whereas the mature secreted form in the supernatant is presumably modified in an identical way to the protein produced from 293 cells.

To gain structural information on this modification, recombinant His-tagged CYTL1 was produced by stably transfected 293 cells, purified from the supernatants by affinity chromatography and subjected to mass spectrometry analysis (cf. Example 12). For this purpose, purified CYTL1 was either used to establish a mass spectrum of the total protein, which determined the exact molecular weight of the modified CYTL1 form. Alternatively, the protein was subjected to proteolytic (tryptic plus chymotryptic) digestion and the cleavage products were individually identified in the spectrum. In this way major parts of the amino acid sequence were confirmed and the peptide EYPIPVTTVLPDR was detected to be O-glycosylated on threonines 128 and/or 129. The specific carbohydrate structure identified is composed of 1 HexNAc, 1 Hex and 2 Neu5Ac as displayed (Figure 3C, D, E). No glycosylation was detected on the 15 kD CYTL1 form. This is a novel unpredictable finding as the sites and structures of O-glycosylation cannot be predicted from the protein sequence.

To evaluate the bioactivities of recombinant CYTL1 the O-glycosylated and unglycosylated forms of CYTL1 were purified and tested in the spheroid sprouting assay (see Example 1). Recombinant glycosylated CYTL1 induced angiogenic sprouting of HUVECs in vitro comparable to VEGF-A up to a concentration of 25 ng/ml (Figure 4A). However, surprisingly the non-glycosylated 15 kDa form did not induce sprouting (Figure 4B). This demonstrates an unexpected role of the glycosylation, as the specific modification is apparently essential for CYTL1 bioactivity and probably for binding to a not yet identified specific receptor. This is surprising as in the majority of cases unglycosylated forms of cytokines or growth factors possess comparable bioactivities with their modified forms. For example, a large number of recombinant factors used for research or therapeutic purposes is produced in bacteria, yeast or insect cells which cannot add the authentic glycosylation.

### The transcriptional response to CYTL1 is distinct from VEGF-A and does not comprise pro-inflammatory but rather anti-inflammatory components

To evaluate whether the signaling response to CYTL1 might just mimic the response to VEGF-A, or would induce unique signaling pathways in the cell, transcriptional profiling was used to compare the gene repertoire induced by CYTL1 to the repertoire triggered by VEGF-A.

In this regard, it was previously investigated whether the transcriptional response of endothelial cells to VEGF-A and observed a surprisingly large overlap of the gene repertoire induced by VEGF-A and the inflammatory cytokine IL-1. These findings demonstrated a significant inflammatory component in VEGF-A bioactivity (Schweighofer et al, Thromb Haemost. 2009, PMID: 19718476). It was therefore investigated here if CYTL1 would induce a similar inflammatory response in HUVECs. First the upregulation of VCAM and E-selectin mRNAs was tested, which encode two surface proteins highly important for the inflammatory response by their essential involvement in immune cell diapedesis (Vestweber, Ann N Y Acad Sci 2012, PMID: 22671605). Whereas VEGF-A, as previously shown (Schweighofer et al, 2009), reproducibly caused a low but significant upregulation of VCAM and E-selectin mRNAs, CYTL1 was completely devoid of this activity (Figure 4C). Furthermore, it was shown in the previous work that a number of genes thought to be important for the inflammatory response are actually upregulated equally strong by VEGF-A and IL-1. This group of genes includes for example DSCR-1 and tissue factor and the transcription factor NFAT is important for their VEGF-A-mediated upregulation (Schabbauer et al, Thromb Haemost. 2007, PMID: 17549302). It is shown here for DSCR1 that, when compared to VEGF-A, CYTL1 cannot upregulate DCSR1 mRNA to a significant extent (Figure 5B).

In contrast to the inflammatory genes, a small group of genes was shown to be specifically upregulated by VEGF-A and not by inflammatory cytokines or general growth factors, which was proposed to be angiogenesis-related genes (Schweighofer et al, 2009). Two members of this group most highly upregulated by VEGF-A were the transcription factors EGR-3 and NR4A2, which have been shown meanwhile to play indeed a role for angiogenesis (Liu et al, Oncogene 2008, PMID: 18059339; Zhao et al, Int J Cancer 2011, PMID: 20715116).CYTL1 was able to strongly induce EGR-3 and NR4A2 mRNAs about 150- and 5-fold, respectively, although these induction rates were lower than observed for VEGF-A (Figure 5A).

Finally, the transcriptomic data for genes that are uniquely upregulated by CYTL1 were screened. In this regard we made the striking detection of a group of genes encoding several isomeric forms of metallothioneins, which were strongly and sustained induced by CYTL1 for a period of over 10 h. In contrast, VEGF-A only led to a transient much lower induction after 2 h which returned to baseline at 4h post induction (Figure 5C). This finding is important since metallothioneins have been reported to possess anti-apoptotic and anti-inflammatory functions (Nielsen et al, Biomark Insights 2007, PMID 19690641).

Taken together, these data show that the response of endothelial cells to CYTL1 is clearly distinct from VEGF-A in a number of important directions. Whereas proangiogenic genes induced by VEGF-A appear to be significantly expressed after CYTL1 treatment, especially genes with inflammatory functions, which in part can be induced by VEGF-A, are not induced by CYTL1 at all. In contrast, a group of genes such as the metallothioneins, which have been reported to fulfil roles to prevent inflammation and apoptosis, are highly and sustained upregulated by CYTL1 and much less so by VEGF-A. This showed that CYTL1, when compared to VEGF-A, has a much more favourable range of efficacy for regenerative therapies.

### CYTL1 is induced in endothelial cells by hypoxia and mediates sprouting independently of VEGF-A / VEGFR2 signaling

Hypoxia, the deficiency of oxygen, is the initial trigger to produce VEGF-A in any cell type, which consecutively leads to new blood vessel formation by sprouting angiogenesis. It is therefore a hallmark of all neovascularization (Hickey & Simon, Curr Top Dev Biol 2006, PMID: 17118268). To investigate if upregulation of CYTL1 could also be triggered by hypoxia, HUVECs were subjected to low oxygen (2%) conditions. Indeed, CYTL1 mRNA was significantly induced, although this induction was lower than for VEGF-A (Figure 6B).

Principally it would be possible that CYTL1 effects could be mediated at least in part via induction of VEGF-A or that VEGF-A would induce CYTL1. Therefore we analyzed whether CYTL1 would upregulate VEGF-A or vice versa. However, neither CYTL1 was capable to induce VEGF-A nor VEGF-A could upregulate CYTL1, demonstrating that the effects of CYTL1 on angiogenic sprouting are clearly not mediated via VEGF-A. This was the case under normoxic as well as hypoxic conditions (Figure 6A).

This independence of CYTL1 and VEGF-A activities was further shown by separately inhibiting VEGF-A or CYTL1 effects on sprouting using a neutralizing anti-KDR (anti-VEGFR-2) antibody or a neutralizing anti-CYTL1 antibody, which has recently been generated by us. The anti-KDR antibody inhibited VEGF-A-induced sprouting but left CYTL1-induced sprouting unaffected (Figure 7A), whereas the anti-CYTL1 antibody inhibited CYTL1-induced sprouting, but did not affect VEGF-A-induced sprouting (Figure 7B). This demonstrates that CYTL1 mediates sprouting independent from VEGF-A/VEGFR-2 signaling.

### Glycosylated recombinant CYTL1 strongly induces functional vessel formation in vivo, whereas the activity of the unglycosylated form is strongly reduced

To test whether recombinant CYTL1 would also function to trigger vessel formation in vivo, we further used an in vivo angiogenesis assay including human endothelial cells in mice (Alajati et al, Nature Methods 2008, PMID: 18391960). In this assay, spheroids formed from HUVECs were suspended in a mixture of Matrigel and fibrinogen supplemented with the glycoslylated or unglycoslyated forms of CYTL1 or a mixture of VEGF-A and bFGF and implanted subcutaneously into SCID mice. It has been shown previously, that in the presence of VEGF-A and bFGF the grafted human endothelial cells efficiently vascularize the plug and anastomose with the mouse vasculature to give rise to a perfused vascular network, whereas in the absence of the angiogenic factors no or very little vessel formation is obtained (Alajati et al, 2008). In this assay, recombinant glycosylated CYTL1 strongly stimulated the formation of perfused vessels comparable to VEGF-A/bFGF (Figure 8A and B and Examples 9/10). However, only a strongly reduced activity was observed for the unglycosylated form. Notably, it is possible that the residual activity of the unglycosylated CYTL1 could be due to contamination with some active glycosylated CYTL1 due to the isolation procedure from cell lysates.

### In a rat acute infarct model CYTL1 attenuates scar formation and functional impairment of heart muscle (see also Example 11)

ECFCs/BOECs have previously been shown to incorporate into heart vessels and to be beneficial when injected intramyocardially in a porcine infarct model (Dubois et al, 2010). Due to our findings that CYTL1, which is prominently secreted by these cells, can stimulate angiogenic sprouting of endothelial cells in vitro and strongly induces functional vessel formation in vivo, it is possible that the beneficial effects observed for ECFCs in the infarct model is caused by CYTL1. Therefore, we tested the recombinant protein in an acute infarction model in rats. Myocardial infarct was induced by ligation of the left anterior descending artery (LAD). Recombinant CYTL1 was then injected intramyocardially into the infarcted area at several sites. Impairment of heart function was monitored two days and 28 days after ligation by measuring left ventricular ejection fractions using sonography. Indeed, rats that had received CYTL1 showed a diminished functional impairment of the EF by about 30% compared to control rats at day 28 (Figure 9A). Furthermore, after day 28 hearts were excised, paraffin sections prepared and subjected to Masson Trichrome staining. This showed that CYTL1-treated hearts displayed significantly reduced scar formation four weeks after LAD ligation (Figure 9B and C).

### Example 1 - Spheroid-based in-vitro angiogenesis assay (e.g. for testing whether a compound, in particular a CYTL1 protein of the invention, induces sprouting)

HUVECs or ECFCs were used to generate spheroids of defined cell numbers as described (Korff & Augustin, JCB 1998, PMID: 9832561; T.Korff, in: Methods in Endothelial Cell Biology, ed. H.Augustin, Springer Verlag, 2004, chapter 12, p. 115), with minor modifications as described below. When indicated, cells were used 48 hours after infection with the lentiviruses.

### Generation and harvesting of spheroids

Endothelial cells were washed, trypsinized and suspended in culture medium containing 20% (wt/vol) methylcellulose (Sigma) at a concentration of 2,7x105 per 15ml. For the generation of mosaic spheroids 5% ECFCs were mixed with 95% HUVECs. Drops of 25 µl of the suspension were pipetted onto non-adhesive square plates (Greiner Bio-One) and incubated over night hanging upside-down at 37°C to form spheroids of about 450 cells. Spheroids were harvested by washing them off the plate with PBS containing 10% FCS followed by centrifugation (5 min / 150g / without brake / RT). The supernatant was removed and the tube was shortly scratched over a rough underground to loosen the pellet.

The outer wells of a 48-well plate was filled with 0.5 ml PBS and placed at 37°C. 0.5 ml 10x M199 (Invitrogen LT), 90 µl HEPS and 4 ml collagen stock solution were carefully mixed and put on ice. The harvested spheroids were overlayed with 2.2 ml FCS-containing Methocel solution (20% FCS / 80% Methocel). Immediately prior to use, the collagen medium solution was neutralized using ice cold 0.2 N NaOH. 2.2 ml neutralized collagen medium were then mixed with the 2.2 ml spheroid containing Methocel solution. The spheroid suspension with a final concentration of 40% Methocel / 10% FCS / 40% collagen / 10% 10xM199 was rapidly transferred into prewarmed 48-well plates (0.5 ml per well) and put at 37°C. After 30 min of gel formation, EBM-2 MV (basal medium without bullet Kit) medium without VEGF-A and the compound (as a negative control), or containing VEGF-A (as a positive control) or the CYTL1 protein (or in general, the compound) to be tested at the indicated concentrations was layered onto the top of the gel. Typically, the medium contains 25 ng/ml of either VEGF-A or the CYTL1 protein to be tested. For unknown compounds, or to adapt the assay, one of skill can easily find a suitable concentration of the compound, for instance by testing several magnitudes of compound concentration in the assay (typically testing a range from 10pM to 1mM). Commonly, such an approach will result in a dose dependent response curve, as known in the art.

Optionally, as an additional test, to block KDR or CYTL1 mediated sprouting activity, spheroids were incubated with 10 µg/ml of the neutralizing monoclonal anti-human VEGFR2/KDR antibody (R&D Systems) or with hybridoma supernatant containing neutralizing anti-human CYTL1 antibody (9C2).

### Quantitative analysis of endothelial cell sprouting

Following incubation at 37°C overnight, in vitro angiogenesis was assessed. Pictures were taken on a Nikon eclipse 80i microscope equipped with a CCD camera (Nikon) (see Fig. 12B for exemplary micrographs). Sprouts were quantified by measuring the number of sprouts per spheroid, the mean length per sprout and the total cumulative length of sprouts grown out of each spheroid with the ImageJ software. The proangiogenic potency of a compound is reflected by its capacity to increase the number and the length of the sprouts originating from the spheroids. For this reason, the cumulative length of all sprouts originating from one spheroid is a sensitive parameter ("cumulative sprout length"). All experiments were performed at least three times with HUVECs and ECFCs of different donors, 20 spheroids were analyzed for each analysis point in all experiments.
"Sprouting activity" of a sample is calculated in the following way:
N is the number of spheroids in the respective sample. Furthermore, the (sum of all cumulative sprout lengths) = (cumulative sprout length of spheroid 1) + (cumulative sprout length of spheroid 2) + (cumulative sprout length of spheroid 3) + ... + (cumulative sprout length of spheroid N); wherein all spheroids are part of the respective sample. And finally, sprouting activity = (sum of all cumulative sprout lengths) divided by N. (see Fig. 12A for exemplary results) Preferably, sprouting activity is defined in relative terms, by expressing the measured sprouting activity of a sample as percentage, percentage increase/decrease, "fold" or "fold increase/decrease" in reference to the measured sprouting activity of the control.

A CYTL1 protein of the invention or for inventive use induces sprouting (also called sprouting activity herein) in a spheroid-based in-vitro angiogenesis assay compared to an uninduced control, typically at least 25% more, more typically at least 50% more, especially at least 100% more or even at least 200% more.

Exemplary expected results are given in Fig. 12. See also Figs. 1, 2, 4, 7 and the description text relating thereto for exemplary results of the spheroid-based in-vitro angiogenesis assay.

### Example 2 - Production of recombinant glycosylated CYTL1

Expression constructs for CYTL1 were generated by insertion of the cDNA for CYTL1 into the pIRES2-AcGFP1 vector (Clontech, Takara Bio-Europe, Saint-Germain-en-Laye, France). Several different constructs in which the 3'-end of the CytL1 coding region was fused to DNA sequences encoding for a 3xFlag-tag, a 6xHis-tag or a combined 3xFlag-6xHis-tag were obtained. Human embryonic kidney cells (HEK293T/17, ATCC-No. CRL-11268) were stably transfected with these expression constructs and transfected cells overexpressing the fusion proteins isolated by selection with G418 (Carl Roth, Karlsruhe, Germany). Cells were subcloned and expanded in Dulbecco's Modified Eagle's Medium (DMEM, Invitrogen) with 10% fetal calf serum (FCS, Sigma-Aldrich), 2 mM glutamine, 100 U/ml penicillin and 1 mg/ml streptomycin. For protein production dense cultures of the cells were kept in serum-free OptiMEM medium (Life Technologies) for 24 to 48h. Then the supernatants were collected and the recombinant tagged protein affinity purified using anti-Flag M2 affinity gel (Sigma) or subjected to Ni-NTA (Life technologies) purification under native conditions according to the protocols provided by the manufacturers. Briefly, binding to anti-Flag agarose beads was performed in TBS (50mM Tris/HCl, pH=7.4, 150mM NaCl), followed by extensive washing with TBS and elution with 0.1M Glycin pH=3.5 followed by immediate neutralizing by adjusting the solution to 0.05M Tris/HCl pH=7.5, 150mM NaCl. Binding to Ni-NTA Agarose was carried out in Native Binding Buffer (50mM NaH2PO4, pH 8.0), washing with Native Wash buffer (50mM NaH2PO4, pH 8.0 containing 10mM imidazole) and bound protein was eluted with Native Elution Buffer (50mM NaH2PO4, pH 8.0 with 250mM imidazole). The eluted protein was then concentrated by Vivaspin columns (GE Healthcare, Uppsala, Sweden) to a concentration of about 1⑥g/ml and dialysed against PBS pH=7.2. The purity of the protein and its molecular weight was determined by SDS polyacrylamide gel electrophoresis followed by Western blot analysis using antibodies directed against the Flag- or His-Tag. Biological activity was evaluated by performing the spheroid-based in-vitro angiogenesis assay described herein.

### Example 3 - Cell culture

Human umbilical vein endothelial cells (HUVECs) were isolated as described previously (Wojta et al, JBC 1989, PMID: 2492525) and cultured on 1% gelatine coated cell culture dishes in endothelial cell growth medium-2 (EGM-2 bullet kit, Lonza). HUVECs of passage 3 to 5 were used for experiments.

Endothelial colony forming cells (ECFCs) were isolated as described previously (Untergasser et al, Exp Gerontol 2006, PMID: 16698211; Yoder et al, 2007) with minor modifications. Briefly, human cord blood samples were obtained from the Department of Obstetrics and Gynecology, Medical University of Vienna, according to an approval of the respective ethical committee including written informed consent of the donors. Samples were collected in cord blood collection bags (MacoPharma) and stored at room temperature until mononuclear cells (MNCs) were isolated. MNC isolation was carried out with Lymphocyte Separation Medium LSM 1077 (PAA) as recommended by the manufacturer within 24 h after collecting the blood. Total MNCs were resuspended in EGM-2 medium (Lonza) and seeded onto 0.01% kangaroo collagen (Sigma-Aldrich) coated culture dishes. 24 h after seeding the floating cells were removed and fresh medium was added to the adherent cells. Clonal outgrowth was observed after 2-4 weeks. ECFCs were expanded and used for further analysis. ECFCs of passage 3 to 5 were used for experiments.

Human embryonic kidney 293 cells (HEK293 cells, ATCC No. CRL-1573) were cultured in Minimum Essential Medium α (MEM-α, Invitrogen) with 10% newborn calf serum (NCS, Invitrogen), 2 mM glutamine (Sigma-Aldrich), 100 U/ml penicillin and 1 mg/ml streptomycin (Bio Whittacker).

HEK293T/17 cells (ATCC No. CRL-11268) were cultured in Dulbecco's Modified Eagle's Medium (DMEM, Invitrogen) with 10% fetal calf serum (FCS, Sigma-Aldrich), 2 mM glutamine, 100 U/ml penicillin and 1 mg/ml streptomycin.

Cells were grown under normoxic conditions at 37°C, 5% CO2 and 21% 02 in a Napco cell culture incubator equipped with an oxygen measuring device (Thermo Fisher, Vienna, Austria). "Hypoxia" conditions were applied by flow through of nitrogen reducing oxygen concentration to 2%.

### Example 4 - Affymetrix Microarray hybridization

Total RNA was prepared from ECFCs and HUVECs as described under RNA preparation. cRNA was synthesized from 200 ng of RNA, hybridized to the GeneChip® PrimeView™ Human Gene Expression Array (Affymetrix) according to the manufacturer's protocols (Affymetrix support site, http://www.affymetrix.com/support/index.affx) and the arrays scanned and analyzed as described in detail in Tauber et al. (Tauber et al, 2010). RMA (robust multiarray average) signal extraction and normalization were performed as described (http://www.bioconductor.org/).

### Example 5 - Generation of replication-defective adenoviruses encoding CYTL1

A full length open reading frame complementary DNA (cDNA) clone (IRATp970D0645D) encoding for the human CYTL1 was ordered and obtained in the plasmid pCMV-SPORT6 from ImaGenes (Berlin, Germany). The plasmid pShuttle-CYTL1-flag-IRES-hrGFP-1 was constructed by inserting the CYTL1 ORF sequence, which was amplified from the CYTL1 cDNA clone by PCR using primers containing restriction sites for SpeI and XhoI, into corresponding restriction sites of the pShutlle-IRES-hrGFP-1 plasmid (Stratagene, LaJolla, CA). BJ5183 bacteria were transformed with the pShuttle constructs in combination with the Adeasy-1 adenoviral plasmid (Stratagene), which encodes for an E1/E3 depleted adenovirus type 5 vector, and clones with homologous recombination selected by kanamycin. Recombinant plasmids (Ad.CYTL1-flag-IRES-GFP and empty Ad.IRES-GFP vector) were then amplified in XL10-Gold cells (Stratagene) and, after restriction digest of the vector DNA with PacI (New England Biolabs, Frankfurt am Main, Germany), transfected into HEK 293 cells using a mammalian transfection kit (Stratagene) to generate primary adenoviruses. Viruses containing the CYTL1 cDNA (Ad.CYTL1) and control viruses containing only the GFP gene (Ad.GFP) were subcloned by limiting dilution, amplified again and finally purified by CsCl2 ultracentrifugation. The viral titer was determined using the Adeno-X Rapid Titer kit (Clontech) according to the recommended protocol.

### Example 6 - Generation of replication defective lentiviruses and viral titer determination

Lentiviral vector DNA encoding shRNA directed against CYTL1 (Mission pLKO.1-shRNA-CYTL1-puro) or scrambled shRNA (Mission pLKO.1-shRNA-neg-puro) were obtained from Sigma-Aldrich. The packaging construct psPAX2 for the lentiviral protein production and pMD2.G, the plasmid encoding for the VSV-G envelope, were purchased from Addgene (Cambridge, UK). HEK 293T/17 cells were used for the generation of lentiviruses following cotransfection with the above mentioned plasmids. For transfection cells were seeded at a density of 2x10⁶ cells per 10 cm dish. 24 h later transfection was performed by the calcium phosphate method (Stratagene) according to the recommendations of the manufacturer. 8 h after transfection, medium was exchanged and viral supernatant was harvested 24 h and 36 h later. To determine virus titers viral RNA was extracted with QiaAmp Viral RNA Mini Kit (Qiagen, Germantown, MD, USA) according to the recommendations of the manufacturer. RNA was primed with hexamer primers (Invitrogen) and reverse transcribed with Superscript II Reverse Transcriptase (Invitrogen). Viral cDNA was quantified using primers specific for the puromycin resistance gene by real-time RT-PCR (qRT-PCR) detecting Rotor Gene SYBR Green (Qiagen) in a Rotor-Gene⁽Q LightCycler (Qiagen).

### Example 7 - Adenoviral and lentiviral infection of cells

For the infection 1x105 cells were seeded per well of 6-well plates. 24 h later medium was removed and fresh medium including 10 MOI adenoviruses or 0.2 to 1 ml lentivirus supernatant added per well. 24 h to 72 h after infection cells were harvested and used for further analysis.

### Example 8 - RNA preparation and real-time RT-PCR analysis

For the extraction of RNA, cells were incubated with RNAlater (Ambion) for 1 min, washed with DEPC-treated water and lysed with QIAzol (Qiagen). RNA was extracted using RNeasy Mini Kit (Qiagen) according to manufacturer's instructions. 1 µg of total RNA was used to synthesize cDNA with Superscript II Reverse Transcriptase (Invitrogen) and oligo(dT) primers (Invitrogen) according to the recommended protocol.

For quantitative real-time PCR a Rotor-Gene(Q LightCycler (Qiagen) detecting Rotor Gene SYBR Green (Qiagen) was used. All values were normalized to β-actin mRNA as internal standard. Primer sequences were designed using the Clone Manager Basic program (Sci-Ed Software, http://www.scied.com). All primers used for gene amplification are listed in Table 2.

**Table 2**

| **Primer** | **Sequence** | **SEQ ID NO** |
|---|---|---|
| betaActin-forward | TTTGAATGATGAGCCTTCGTCCCC GGTCTCAAGTCAGTGTACAGGTAAGC | 54 |
| betaActin-reverse | | 55 |
| | | |
| CD34-forward | TGGACCGCGCTTTGCT CCCTGGGTAGGTAACTCTGGG | 56 |
| CD34-reverse | | 57 |
| | | |
| cKit-forward | ATTCCCAGCCCATGAGTCCTTGA ACACGTGGAACACCAACATCCT | 58 |
| cKit-reverse | | 59 |
| | | |
| CYTL1-forward | ACTGTGTGCTGGACAAGCTG CATTGCAGTCATCCAACAGG | 60 |
| CYTL1-reverse | | 61 |
| | | |
| DSCR1-forward | TAGCTCCCTGATTGCCTGT GGAGAAGGGGTTGCTGAAGT | 62 |
| DSCR1-reverse | | 63 |
| | | |
| EGR3-forward | GACAATCTGTACCCCGAGGA TCCCAAGTAGGTCACGGTCT | 64 |
| EGR3-reverse | | 65 |
| | | |
| KDR-forward | CCTCTACTCCAGTAAACCTGATTGGG | 66 |
| KDR-reverse | TGTTCCCAGCATTTCACACTATGG | 67 |
| | | |
| MT1F-forward | GATGCCAGGACAACCTTTCT AGCAAATGGGTCAAGGTGGT | 68 |
| MT1F-reverse | | 69 |
| | | |
| MT1M-forward | TGTGTCTGCAAAGGACGTT CCAGGTTGTGCAGCTTGTTC | 70 |
| MT1M-reverse | | 71 |
| | | |
| MT1X-forward | GCTTTTCATCTGTCCCGCTG AGTTGGGGTCCATTTCGAGG | 72 |
| MT1X-reverse | | 73 |
| | | |
| NR4A2-forward | TTTCTGCCTTCTCCTGCATT GTGGCACCAAGTCTTCCAAT | 74 |
| NR4A2-reverse | | 75 |
| | | |
| VE-Cadherin-forward | CTTCTCTGCCTCACCTGGTC TCTCCAAGGTGTGTTTGCAG | 76 |
| VE-Cadherin-reverse | | 77 |
| | | |
| VCAM-forward | AAAAGCGGAGACAGGAGACA CCCTTCATGTTGGCTTTTCT | 78 |
| VCAM-reverse | | 79 |
| | | |
| VEGF-A-forward | CTACCTCCACCATGCCAAGT GCAGTAGCTGCGCTGATAGA | 80 |
| VEGF-A-reverse | | 81 |

### Example 9 - SDS PAGE and Western blot analysis

Cell pellets were directly lysed in Laemmli buffer and supernatants of CYTL1 overexpressing cells, all containing the Flag-tagged CYTL1, were mixed 1:1 with Laemmli buffer. Equal amounts of protein extracts were separated under reducing conditions on 15% sodium dodecyl sulfate -polyacrylamide gels and transferred onto Immobilon-P membranes (Millipore) by semidry blotting (Peqlab). Membranes were blocked for 1h in 5% organic skimmed milk powder (Sigma-Aldrich) in PBS containing 0.1% Tween-20 (Bio-Rad). For protein detection membranes were incubated in PBS/0.1% Tween-20/5% milk powder containing a 1:2.000 diluted mouse anti-Flag antibody (Sigma) for exogenous CYTL1 expression. Bound antibodies were detected by applying a species-specific horseradish peroxidase-conjugated secondary antibody (GE Healthcare; diluted 1:5.000) followed by enhanced chemiluminescence (GE Healthcare). Cf. Figure 3 for exemplary results.

### Example 10 - In vivo spheroid implantation angiogenesis assay

HUVECs or ECFCs were used to generate spheroids of defined cell numbers as described HUVECs or ECFCs were used to generate spheroids of defined cell numbers as described (Laib et al., Nature protocols 2009, 4, 1202-1215, PMID: 19644460).

### Generation and harvesting of spheroids

Endothelial cells were washed, trypsinized and suspended in culture medium containing 20% (wt/vol) methylcellulose (Sigma) at a concentration of 4x 106 per 100 ml (sufficient to generate 4 plugs of 1000 spheroids). Drops of 25 µl of the suspension were pipetted onto non-adhesive square plates (Greiner Bio-One) and incubated over night hanging up-side-down at 37°C to form spheroids of about 100 cells. Spheroids were harvested by washing them off the plate with PBS containing 10% FCS followed by centrifugation (5 min / 150g / without brake / RT). The supernatant was removed and the tube was shortly scratched over a rough underground to loosen the pellet. 4x 300 µl of methocel/fibrinogen (endconcentration: 2 mg/ml; Calbiochem, Merck)/ECBM (1:1:1) mixture for injecting two mice were prepared at RT. The spheroids were washed once with EBM-2 MV (basal medium without bullet Kit) and again sedimented by centrifugation (5 min / 150g / without brake / RT). After resuspension in EBM-2 (volume: 1 ml per plug) the spheroids were evenly divided into 2 ml reaction tubes (1 reaction tube per plug) and spinned down for 5 min at 1300g, RT. VEGF-A (165) and FGF-2 (500 ng/ml each) or CYTL1 (25ng/ml) were added to the methocel/fibrinogen/EBM mixture and 300 µl immediately added to each spheroid sediment.

### Plug implantation

300 µl of Matrigel (growth factor reduced; BD Biosciences) was pipetted on top of the spheroid/methocel/fibrinogen/ EBM mixture on ice. 4 µl of thrombin (1 U/µl; Calbiochem) and the solution was carefully mixed the by gently using a syringe. Spheroid suspensions containing approximately 1000 spheroids of 100 cells each were then injected subcutaneously on each side lateral to the abdominal midline region into 4- to 6-week-old SCID mice. Two plugs were implanted per mouse, each experimental group consisted of 5 mice.

### Dissection and of implanted plugs

After 21 days, mice were killed, the harvested plugs were fixed overnight (0.5 g/l calcium acetate, 5 g/l zinc acetate, 5 g/l zinc chloride in 0.1M Tris, pH 7.4) and analyzed by immunohistochemistry. For perfusion studies, FITC-dextran (100 ml; 70,000 MV, 25 mg/ml, anionic, lysine fixable; Molecular Probes) was injected into the tail veins of mice. Circulation was allowed to continue for 10 min before sacrificing the animals. The Matrigel-fibrinogen plugs were surgically removed.

All animal procedures were carried out in accordance to guidelines of the local committee for animal experimentation (DKFZ Heidelberg and RP Karlsruhe).

### Quantification of the human grafted vasculature and statistical analysis

Zinc-fixed Matrigel plugs were embedded in paraffin. Sections at 8 µm were prepared across the entire implant size and 3 sections were selected from the beginning, the center, and the end. Microvessel density (MVD) analysis was performed using immunohistochemistry for human CD34. Briefly, after antigen retrieval by digestion with Proteinase K (Sigma-Aldrich) at 37°C for 15 minutes plugs were incubated with mouse-anti-human CD34 (QBEND10; Novocastra) followed by incubation with goat-anti-mouse Alexa 546 (Invitrogen). Slides were mounted with DAKO fluorescent mounting medium (DAKO). Images of the complete plug area were taken using an Axioimager microscope (Zeiss) with a 10-fold objective and the inbuilt camera. Pictures were assembled by multiple alignment and fluorescent signals of the complete plug matrix were calculated as vessel number per mm2 (Image J). Cf. Fig. 8 for exemplary results.

### Generation and harvesting of spheroids

Endothelial cells were washed, trypsinized and suspended in culture medium containing 20% (wt/vol) methylcellulose (Sigma) at a concentration of 4x 106 per 100 ml (sufficient to generate 4 plugs of 1000 spheroids). Drops of 25 ul of the suspension were pipetted onto non-adhesive square plates (Greiner Bio-One) and incubated over night hanging up-side-down at 37°C to form spheroids of about 100 cells. Spheroids were harvested by washing them off the plate with PBS containing 10% FCS followed by centrifugation (5 min / 150g / without brake / RT). The supernatant was removed and the tube was shortly scratched over a rough underground to loosen the pellet. 4x 300 µl of methocel/fibrinogen (endconcentration: 2 mg/ml; Calbiochem, Merck)/ECBM (1:1:1) mixture for injecting two mice were prepared at RT. The spheroids were washed once with EBM-2 MV (basal medium without bullet Kit) and again sedimented by centrifugation (5 min / 150g / without brake / RT). After resuspension in EBM-2 (volume: 1 ml per plug) the spheroids were evenly divided into 2 ml reaction tubes (1 reaction tube per plug) and spinned down for 5 min at 1300g, RT. VEGF-A (165) and FGF-2 (500 ng/ml each) or CYTL1 (25ng/ml) were added to the methocel/fibrinogen/EBM mixture and 300 µl immediately added to each spheroid sediment.

### Plug implantation

300 µl of Matrigel (growth factor reduced; BD Biosciences) was pipetted on top of the spheroid/methocel/fibrinogen/ EBM mixture on ice. 4 µl of thrombin (1 U/µl; Calbiochem) and the solution was carefully mixed the by gently using a syringe. Spheroid suspensions containing approximately 1000 spheroids of 100 cells each were then injected subcutaneously on each side lateral to the abdominal midline region into 4- to 6-week-old SCID mice. Two plugs were implanted per mouse, each experimental group consisted of 5 mice.

### Dissection and of implanted plugs

After 21 days, mice were killed, the harvested plugs were fixed overnight (0.5 g/l calcium acetate, 5 g/l zinc acetate, 5 g/l zinc chloride in 0.1M Tris, pH 7.4) and analyzed by immunohistochemistry. For perfusion studies, FITC-dextran (100 ml; 70,000 MV, 25 mg/ml, anionic, lysine fixable; Molecular Probes) was injected into the tail veins of mice. Circulation was allowed to continue for 10 min before sacrificing the animals. The Matrigel-fibrinogen plugs were surgically removed.

All animal procedures were carried out in accordance to guidelines of the local committee for animal experimentation (DKFZ Heidelberg and RP Karlsruhe).

### Quantification of the human grafted vasculature and statistical analysis

Zinc-fixed Matrigel plugs were embedded in paraffin. Sections at 8 µm were prepared across the entire implant size and 3 sections were selected from the beginning, the center, and the end. Microvessel density (MVD) analysis was performed using immunohistochemistry for human CD34. Briefly, after antigen retrieval by digestion with Proteinase K (Sigma-Aldrich) at 37°C for 15 minutes plugs were incubated with mouse-anti-human CD34 (QBEND10; Novocastra) followed by incubation with goat-anti-mouse Alexa 546 (Invitrogen). Slides were mounted with DAKO fluorescent mounting medium (DAKO). Images of the complete plug area were taken using an Axioimager microscope (Zeiss) with a 10-fold objective and the inbuilt camera. Pictures were assembled by multiple alignment and fluorescent signals of the complete plug matrix were calculated as vessel number per mm2 (Image J). Cf. Fig. 8 for exemplary results.

### Example 11 - Acute myocardial infarction model

Myocardial infarct (MI) was induced in 8- to 10-week old male rnu/rnu rats (Harlan Winkelmann, Borchen, Germany) through left anterior descending coronary artery (LAD) ligation (Kocher et al, 2001). Animal procedures were carried out in accordance to guidelines of the local committee for animal experimentation (Medical University of Vienna). A CYTL1 solution (2⑥g/100⑥l/rat) or PBS as control was then injected intramyocardially within the infarcted area at several sites. Transthoracic echocardiography was performed before, 2 days and 4 weeks after MI with a Vivid 7 Echocardiography system (GE Healthcare), using a commercially available high-frequency linear-array transducer system.

Tissue Preparation and histochemistry of rat hearts: After the last sonography rats were injected intravenously with 5000 I.E. heparin (Gilvasan) and with 5 ⑥l custodiol (Sandor) via the aortic arch. Hearts were excised and fixed in 4% paraformaldehyde. Three parts of equal thickness representing the base, middle, and apex of the heart were embedded in paraffin. From each part 4 µm slides were prepared for Masson Trichrome staining (Sigma) according to the manufacturer's instructions.

See Fig. 9 for exemplary results.

### Example 12 - Mass spectrometry

Basic chemicals were obtained from Sigma Aldrich (Vienna, Austria), sequencing grade modified trypsin was purchased from Promega (Madison, WI, USA). Protein digestion: CYTL1 (∼20 µg) was reduced in 15 mM dithiothreitol, 0,1 M ammonium bicarbonate at 56°C for 45 min, followed by cystein-alkylation with 55 mM iodoacetamide in 0,1 M ammonium bicarbonate at room temperature for 30 min in the dark. Proteins were precipitated with a fivefold volume of ice cold acetone (-20 °C, 20 min) and vacuum-dried. Digestion was performed with 0.1 µg trypsin in the mentioned bicarbonate buffer at 37°C over night. Peptide analysis: 0.5 µg digested CYTL1 was subjected to LC-ESI-MS/MS analysis. Samples were run on a Dionex Ultimate 3000 system with a BioBasic C-18 column (Thermo, 150 x 0.35 mm, 5 µm particle size) and a standard gradient form 5% B to 65 % B in 50 minutes (solvent A: 0,3 % formate buffered to pH 3 with ammonia, solvent B: acetonitrile). Eluted peptides were detected using a Bruker MaXis 4G in Auto MS/MS mode with collision induced dissociation (CID). Specific values were set to: low mass 300 m/z, transfer time 100 µs, pre-pulse storage 10 µs, spectra rate 1,0 Hz, 10 precursor ions with active exclusion after 3 spectra and a threshold of 5000 counts. Prior to sample measurement, the MS was calibrated with Agilent's ESI-L low concentration tune mix in positive ion mode. A Chip Cube high mass reference ("lock mass", Agilent, 1221.991 m/z) was used for recalibrating the data set after measurement. Evaluation of the results was done manually with Bruker's Data Analysis 4.0 as well as automatically with Protein Scape 3.0 and Mascot search algorithm. Total protein analysis: 1 µg per sample was introduced to the ESI-source via a C5 reversed-phase column (50 x 0.32 mm, 3 µm, Discovery Biowidepore, Supelco) using 0.1 % formic acid in water (Eluent A) and 0.1 % formic acid in acetonitrile (Eluent B) with the said Ultimate 3000 system. After an 8 min sample loading, a gradient from 10 to 50 % Eluent B in 20 min at a flow rate of 6 µl/min was run. Mass spectrometreic detection was done using a maXis 4G from Bruker with specific settings adapted to spectra rate 1 Hz, low mass 600, transfer time 120 µs and pre pulse storage 15 µs. The data were analyzed using Bruker's Data Analysis 4.0 with the deconvolution algorithm max ent.

The present invention is further exemplified by (but not limited to) the following embodiments:
1. An isolated CYTL1 protein,
   wherein the protein sequence is more than 80%, preferably more than 90%, more preferably more than 95%, even more preferably more than 98%, especially completely identical to the entire amino-acid sequence of an entry selected from the group of the following: UniProt entries Q9NRR1, G1S206, F7CJ05, H2QP59, G3QV27, H2PCW0, G7MSJ5, G7P588, S7MTB0, G3WH39, B5DFG7, U6D578, G3TD71, F1P7S3, H0WPN0, L5LQ03, W5PBC3, E1BN89, L8IQ55, G3GWE1, M3W6F7, M3Y970, D2HLS4, G1M521, G1T5G9, G5B313, I3LW41 (SEQ ID NOS: 1-27) and their respective processed proteins (SEQ ID NOS: 28-53); and
   wherein the protein sequence comprises two consecutive threonine residues, and wherein the first of said residues is glycosylated and/or wherein the second of said residues is glycosylated; and
   wherein the protein induces sprouting in a spheroid-based in-vitro angiogenesis assay compared to an uninduced control, preferably at least 25% more, more preferably at least 50% more, especially at least 100% more or even at least 200% more.
2. The protein of embodiment 1, wherein the protein sequence is more than 80%, preferably more than 90%, more preferably more than 95%, even more preferably more than 98%, especially completely identical to the entire sequence of UniProt entry Q9NRR1 (SEQ ID NO: 1) or of its processed protein (SEQ ID NO: 28).
3. The protein of embodiment 2,
   wherein the protein sequence comprises the following sequence: 
TPPTCYSRMRALSQEITRDFNLLQVSEPSEPCVRYLPRLYLDIHNYCVLD
KLRDFVASPPCWKVAQVDSLKDKARKLYTIMNSFCRRDLVFLLDDCNALE
YPIPVTTVLPDRQR,
 wherein at most three, preferably at most two, more preferably at most one of the amino-acid residues, are deleted or replaced by any other amino-acid residue independently of each other, and/or wherein at most 10, preferably at most five, more preferably at most three, especially at most one other amino-acids are inserted into the sequence, except between the two consecutive threonine residues.
4. The protein of embodiment 2,
   wherein the protein sequence comprises the following sequence: 
TPPTCYSRMRALSQEITRDFNLLQVSEPSEPCVRYLPRLYLDIHNYCVLD
KLRDFVASPPCWKVAQVDSLKDKARKLYTIMNSFCRRDLVFLLDDCNALE
YPIPVTTVLPDRQR.
5. The protein of embodiment 4, wherein the protein sequence is 
TPPTCYSRMRALSQEITRDFNLLQVSEPSEPCVRYLPRLYLDIHNYCVLD
KLRDFVASPPCWKVAQVDSLKDKARKLYTIMNSFCRRDLVFLLDDCNALE
YPIPVTTVLPDRQR.
6. An isolated CYTL1 protein,
   wherein the protein sequence comprises the following sequence: 
TPPTCYSRMRALSQEITRDFNLLQVSEPSEPCVRYLPRLYLDIHNYCVLD
KLRDFVASPPCWKVAQVDSLKDKARKLYTIMNSFCRRDLVFLLDDCNALE
YPIPVTTVLPDRQR,
 wherein at most three, preferably at most two, more preferably at most one of the amino-acid residues, are deleted or replaced by any other amino-acid residue independently of each other, and/or
   wherein at most 10, preferably at most five, more preferably at most three, especially at most one other amino-acids are inserted into the sequence, except between the two consecutive threonine residues;
   and
   wherein the first of the consecutive two threonine residues is glycosylated and/or wherein the second of the consecutive two threonine residues is glycosylated.
7. The protein of embodiment 6,
   wherein the protein sequence comprises the following sequence: 
TPPTCYSRMRALSQEITRDFNLLQVSEPSEPCVRYLPRLYLDIHNYCVLD
KLRDFVASPPCWKVAQVDSLKDKARKLYTIMNSFCRRDLVFLLDDCNALE
YPIPVTTVLPDRQR.
8. The protein of embodiment 7, wherein the protein sequence is 
TPPTCYSRMRALSQEITRDFNLLQVSEPSEPCVRYLPRLYLDIHNYCVLD
KLRDFVASPPCWKVAQVDSLKDKARKLYTIMNSFCRRDLVFLLDDCNALE
YPIPVTTVLPDRQR.
9. The protein of any one of embodiments 1 to 8, wherein both of said threonine residues are glycosylated.
10. The protein of any one of embodiments 1 to 9, wherein one or each, especially each, of the two consecutive threonine residues are replaced by any other natural or unnatural amino acid residue that can be glycosylated, preferably tyrosine, asparagine, arginine, hydroxylysine, or hydroxyproline, especially serine.
11. The protein of any one of embodiments 1 to 10, wherein one or each, especially each, of said glycosylation(s) comprises a hexose (Hex), an N-acetylhexosamine (HexNAc) or a N-acetylneuraminic acid (Neu5Ac).
12. The protein of any one of embodiments 1 to 11, wherein one or each, especially each, of said glycosylation(s) consists of 1 HexNAc, 1 Hex and 2 Neu5Ac.
13. The protein of any one of embodiments 1 to 12, wherein one or each, especially each, of said glycosylation(s) comprises or consists, especially consists, of the following glycosylation pattern: 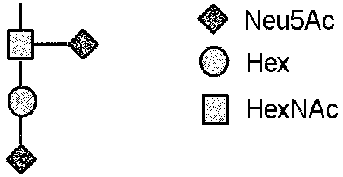 and wherein the HexNAc is attached to the respective glycosylated residue of the protein, preferably wherein said glycosylation pattern is **Neu5Acα2-3Galβ1-3(Neu5Acα2-6)GalNAc.**
14. The protein of any one of embodiments 1 to 13, wherein said glycosylation(s) is/are the only glycosylation(s) of the protein.
15. A protein consisting of a fragment of the protein of any one of embodiments 1 to 14,
   wherein the fragment comprises more than 20 amino-acids, preferably more than 40 amino-acids, more preferably more than 60 amino-acids, especially more than 100 amino-acids;
   and
   wherein the fragment induces sprouting in a spheroid-based in-vitro angiogenesis assay compared to an uninduced control, preferably at least 25% more, more preferably at least 50% more, especially at least 100% more or even at least 200% more..
16. A fusion protein comprising the protein of any one of embodiments 1 to 15,
   further comprising at least one more amino-acid acid, preferably at least ten more amino-acids, more preferably at least 20 more amino-acids, especially at least 30 more amino-acids; and
   wherein the fusion protein induces sprouting in a spheroid-based in-vitro angiogenesis assay compared to an uninduced control, preferably at least 25% more, more preferably at least 50% more, especially at least 100% more or even at least 200% more..
17. The protein of any one of embodiments 1 to 16, wherein at least one amino-acid residue, the N-terminus and/or the C-terminus is chemically modified by a modification other than glycosylation: especially by pegylation, biotinylation, alkylation, hydroxylation, adenylation, phosphorylation, succinylation, oxidation, or acylation, in particular acetylation.
18. A pharmaceutical composition comprising the protein of any one of embodiments 1 to 17, further comprising one or more excipients, preferably wherein the protein is conjugated to a biocompatible matrix selected from polysaccharides, in particular sulfated polysaccharides, especially alginate sulfate or hyaluronan sulfate.
19. The protein of any one of embodiments 1 to 17 or the composition of embodiment 18, for use in therapy.
20. The protein or composition according to embodiment 19, for use in treatment of myocardial infarction.
21. The protein or composition according to embodiment 19, for use in treatment or prevention of ischemia, especially ischemic cardiomyopathy preferably related to myocardial infarction.
22. The protein or composition according to embodiment 19, for use in treatment or prevention of tissue damage, preferably non-ischemic cardiomyopathy.
23. The protein or composition for use according to any one of embodiments 19 to 22, wherein the therapy is conducted in combination with administration of endothelial colony forming cells (ECFCs) or other cells that promote vascularisation of tissues.
24. A method for manufacturing the protein of any one of embodiments 1 to 17, comprising:
   - recombinant expression of the protein, preferably in 293 HEK cells; and
   - purification of the protein, preferably by affinity purification.
25. The method of embodiment 24, further comprising cleavage of a signal peptide from the protein and, preferably, secretion of the protein from the cells.
26. The method of embodiment 24 or 25, wherein the protein further comprises a cleavable or non-cleavable affinity tag, preferably a FLAG tag; and preferably wherein the tag is cleaved from the protein.
27. A CYTL1 protein, or a functional fragment protein thereof or a functional fusion protein thereof, for use in treatment of myocardial infarction, wherein said protein, fragment protein or fusion protein induces sprouting in a spheroid-based in-vitro angiogenesis assay compared to an uninduced control, preferably at least 25% more, more preferably at least 50% more, especially at least 100% more or even at least 200% more..
28. A CYTL1 protein, or a functional fragment protein thereof or a functional fusion protein thereof, for use in treatment or prevention of ischemia, especially ischemic cardiomyopathy preferably related to myocardial infarction, wherein said protein, fragment protein or fusion protein induces sprouting in a spheroid-based in-vitro angiogenesis assay compared to an uninduced control, preferably at least 25% more, more preferably at least 50% more, especially at least 100% more or even at least 200% more.
29. A CYTL1 protein, or a functional fragment protein thereof or a functional fusion protein thereof, for use in treatment or prevention of tissue damage, preferably non-ischemic cardiomyopathy, wherein said protein, fragment protein or fusion protein induces sprouting in a spheroid-based in-vitro angiogenesis assay compared to an uninduced control, preferably at least 25% more, more preferably at least 50% more, especially at least 100% more or even at least 200% more.
30. A CYTL1 protein, or a functional fragment protein thereof or a functional fusion protein thereof, for use in therapy, wherein the therapy is conducted in combination with administration of endothelial colony forming cells (ECFCs) and/or other cells that promote vascularisation of tissues, and wherein said protein, fragment protein or fusion protein induces sprouting in a spheroid-based in-vitro angiogenesis assay compared to an uninduced control, preferably at least 25% more, more preferably at least 50% more, especially at least 100% more or even at least 200% more.
31. A anti-CYTL1 antibody, or a functional fragment thereof, for use in treatment of cancer, wherein tumour angiogenesis is inhibited, preferably administered in combination with other angiogenesis inhibitors such as Bevacizumab, and wherein said antibody inhibits CYTL1-induced sprouting in a spheroid-based in-vitro angiogenesis assay in the presence of CYTL1, compared to an uninhibited control.
32. A method to screen for CYTL1 competitors, comprising:
   - providing a chemical compound library; and
   - providing CYTL1, preferably the protein of any one of embodiments 1 to 17, especially the protein of embodiment 8; and
   - providing cells, preferably human umbilical vein endothelial cells (HUVECs); and
   - individually for each compound of the library: bringing the compound into contact with cells and CYTL1 ("a sample"); and
   - performing a spheroid-based in-vitro angiogenesis assay for each sample and compare it to a negative control, preferably the cells in the presence of CYTL1 without any of the compounds; and
   - identifying as positive hit the compound of a sample, which sample has reduced sprouting compared to the negative control, preferably more than 10% reduction, more preferably more than 25% reduction, especially more than 50% reduction.
33. A CYTL1 competitor, optionally the positive hit according to the method of embodiment 32, for use in treatment of cancer, wherein tumour angiogenesis is inhibited, preferably administered in combination with other angiogenesis inhibitors such as Bevacizumab, and wherein said competitor inhibits CYTL1-induced sprouting in a spheroid-based in-vitro angiogenesis assay in the presence of CYTL1, compared to an uninhibited control.
34. A method to identify endothelial colony-forming cells (ECFCs), comprising:
   - providing or isolating one or more cells; and
   - measuring levels of one or more selected from the group of
      CYTL1 mRNA inside the cells,
      CYTL1 protein inside the cells,
      secreted CYTL1 protein; and
   - comparing the measured levels to levels of a control cell population; and
   - identifying the cells as ECFCs if the measured levels are higher than the control levels.
35. The method of embodiment 34, wherein the CYTL1 mRNA codes for a protein sequence or the CYTL1 protein comprises a protein sequence identical to at least 20, preferably at least 50, more preferably at least 100, especially all, of the amino-acids of the entire sequence of an entry selected from the group of the following UniProt entries:
   Q9NRR1, G1S206, F7CJ05, UPI00027F32F6, H2QP59, G3QV27, H2PCW0, G7MSJ5, G7P588, S7MTB0, UPI0002C2DFE8, UPI0002BCE03B, G3WH39, UPI00027FA17A, A1E5L0, Q6P536, Q0VG51, B5DFG7, UPI00033447B9, UPI00033193AE, U6D578, G3TD71, UPI00022346AE, H0ZI47, UPI000194C372, UPI00032AE38E, UPI00038EFAD7, F1P7S3, UPI000359D860, UPI0003C488C0, UPI000388B5C7, K7GER1, UPI00042BCFB6, H0WPN0, UPI00027423DC, L5LQ03, UPI000333DDDA, H0VBW2, UPI00022B6D40, UPI000328A678, UPI000328E1D4, UPI00038C4288, W5PBC3, UPI00029D7CC2, E1BN89, L8IQ55, UPI0003AF8B30, UPI0003C10549, UPI00022F3975, G3GWE1, UPI000333186A, M3W6F7, UPI0000F5A458, UPI00042BCEBF, M3Y970, D2HLS4, G1M521, UPI0001DEB975, UPI0002C55126, UPI0003EE2687, UPI000399217F, UPI0003833174, UPI000386FA6F, F6RIT6, G1T5G9, G5B313, UPI0003500720, U3I8P9, R0JVB5, UPI0003509675, UPI0003C259FF, F1NSV3, G1NJB0, UPI0002035275, I3LW41.
36. The method of embodiment 35, wherein the CYTL1 mRNA codes for a protein sequence or the CYTL1 protein comprises a protein sequence identical to at least 20, preferably at least 50, more preferably at least 100, especially all, of the amino-acids of the entire sequence to UniProt entry Q9NRR1, preferably wherein the cells are human.
37. The method of embodiment 36, wherein the CYTL1 mRNA codes for the protein sequence SEQ ID NO: 1.
38. The method of embodiment 36, wherein the CYTL1 protein sequence comprises or consists, especially consists, of the protein sequence SEQ ID NO: 1 or SEQ ID NO: 28.
39. The method of any one of embodiments 33 to 38, wherein the protein level is measured by means of an anti-CYTL1 antibody or fragment thereof or a molecule capable of essentially selectively binding the CYTL1 protein, preferably in a Western blot or an ELISA.
40. The method of any one of embodiments 33 to 38, wherein the mRNA level is measured by means of a RNA molecule or DNA molecule capable of essentially selectively binding the RNA, preferably in a Northern Blot, a micro-array or an RT-PCR; and/or wherein it is further determined whether the cells are CD31 and/or CD34 positive.
41. A method of purifying or enriching for endothelial colony-forming cells (ECFCs), comprising
   - using the method of any one of embodiments 33 to 40 to identify ECFCs from a cell population; and
   - purifying or enriching for the identified ECFCs.
42. A method of purifying or enriching for endothelial colony-forming cells (ECFCs), comprising:
   - introducing into cells a reporter gene construct operatively linked to the endogenous CYTL1 gene, preferably by replacing the endogenous CYTL1 gene by a CYTL1-reporter gene fusion under the endogenous CYTL1 promoter or by having a reporter gene under the control of the endogenous CYTL1 promoter; and
   - providing or isolating the cells; and
   - purifying or enriching for the cells having reporter gene construct expression or an increased reporter gene construct expression level compared to a control level.
43. The method of embodiment 41, wherein the endogenous CYTL1 gene codes for a protein selected from the group of the following UniProt entries:
   Q9NRR1, G1S206, F7CJ05, UPI00027F32F6, H2QP59, G3QV27, H2PCW0, G7MSJ5, G7P588, S7MTB0, UPI0002C2DFE8, UPI0002BCE03B, G3WH39, UPI00027FA17A, A1E5L0, Q6P536, Q0VG51, B5DFG7, UPI00033447B9, UPI00033193AE, U6D578, G3TD71, UPI00022346AE, H0ZI47, UPI000194C372, UPI00032AE38E, UPI00038EFAD7, F1P7S3, UPI000359D860, UPI0003C488C0, UPI000388B5C7, K7GER1, UPI00042BCFB6, H0WPN0, UPI00027423DC, L5LQ03, UPI000333DDDA, H0VBW2, UPI00022B6D40, UPI000328A678, UPI000328E1D4, UPI00038C4288, W5PBC3, UPI00029D7CC2, E1BN89, L8IQ55, UPI0003AF8B30, UPI0003C10549, UPI00022F3975, G3GWE1, UPI000333186A, M3W6F7, UPI0000F5A458, UPI00042BCEBF, M3Y970, D2HLS4, G1M521, UPI0001DEB975, UPI0002C55126, UPI0003EE2687, UPI000399217F, UPI0003833174, UPI000386FA6F, F6RIT6, G1T5G9, G5B313, UPI0003500720, U3I8P9, R0JVB5, UPI0003509675, UPI0003C259FF, F1NSV3, G1NJB0, UPI0002035275, I3LW41.
44. The method of embodiment 43, wherein the endogenous CYTL1 gene codes for the protein of UniProt entry Q9NRR1 and the cells are preferably human.
45. The method of any one of embodiments 42 to 45, wherein the reporter gene construct comprises a DNA sequence coding for a fluorescent protein, preferably GFP, RFP or YFP, or for a receptor localised on the cell surface.
46. The method of embodiment 45, wherein the cells having reporter gene construct expression or an increased reporter gene construct expression level compared to a control level are purified or enriched for by flow cytometry, especially by fluorescence-activated cell sorting or magnetic-activated cell sorting.
47. The product of the method of any one of embodiments 41 to 46, for use in therapy, optionally in combination with the administration of the protein of any one of embodiments 1 to 17 or the composition of embodiment 18.
48. The product of embodiment 47, for use in treatment of myocardial infarction.
49. The product of embodiment 47, for use in treatment or prevention of ischemia, especially ischemic cardiomyopathy preferably related to myocardial infarction.
50. The product of embodiment 47, for use in treatment or prevention of tissue damage, preferably non-ischemic cardiomyopathy.

## Claims

1. An isolated CYTL1 protein,
wherein the protein sequence is more than 80%, preferably more than 90%, more preferably more than 95%, even more preferably more than 98%, especially completely identical to the entire amino-acid sequence of an entry selected from the group of the following: UniProt entries Q9NRR1, G1S206, F7CJ05, H2QP59, G3QV27, H2PCW0, G7MSJ5, G7P588, S7MTB0, G3WH39, B5DFG7, U6D578, G3TD71, F1P7S3, H0WPN0, L5LQ03, W5PBC3, E1BN89, L8IQ55, G3GWE1, M3W6F7, M3Y970, D2HLS4, G1M521, G1T5G9, G5B313, I3LW41 (SEQ ID NOS: 1-27) and their respective processed proteins (SEQ ID NOS: 28-53); and
wherein the protein sequence comprises two consecutive threonine residues, and wherein the first of said residues is glycosylated and/or wherein the second of said residues is glycosylated; and
wherein the protein induces sprouting in a spheroid-based in-vitro angiogenesis assay compared to an uninduced control, preferably at least 25% more, more preferably at least 50% more, especially at least 100% more or even at least 200% more.

2. The protein of claim 1, wherein:
A) the protein sequence is more than 80%, preferably more than 90%, more preferably more than 95%, even more preferably more than 98%, especially completely identical to the entire sequence of UniProt entry Q9NRR1 (SEQ ID NO: 1) or of its processed protein (SEQ ID NO: 28); or
B) the protein sequence comprises the following sequence: 
TPPTCYSRMRALSQEITRDFNLLQVSEPSEPCVRYLPRLYLDIHNYCVLD
KLRDFVASPPCWKVAQVDSLKDKARKLYTIMNSFCRRDLVFLLDDCNALE
YPIPVTTVLPDRQR,
 wherein at most three, preferably at most two, more preferably at most one of the amino-acid residues, are deleted or replaced by any other amino-acid residue independently of each other, and/or
wherein at most 10, preferably at most five, more preferably at most three, especially at most one other amino-acids are inserted into the sequence, except between the two consecutive threonine residues; or
C) wherein the protein sequence comprises or, especially, is the following sequence:

TPPTCYSRMRALSQEITRDFNLLQVSEPSEPCVRYLPRLYLDIHNYCVLD
KLRDFVASPPCWKVAQVDSLKDKARKLYTIMNSFCRRDLVFLLDDCNALE
YPIPVTTVLPDRQR.

3. The protein of claims 1 or 2, wherein:
A) both of said threonine residues are glycosylated; and/or
B) one or each, especially each, of said glycosylation(s) comprises a hexose (Hex), an N-acetylhexosamine (HexNAc) or a N-acetylneuraminic acid (Neu5Ac);
preferably wherein one or each, especially each, of said glycosylation(s) consists of 1 HexNAc, 1 Hex and 2 Neu5Ac; more preferably wherein one or each, especially each, of said glycosylation(s) comprises or, especially, consists of the following glycosylation pattern: 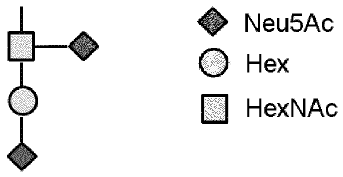 and wherein the HexNAc is attached to the respective glycosylated residue of the protein; and/or
C) said glycosylation(s) is/are the only glycosylation(s) of the protein.

4. A protein consisting of a fragment of the protein of any one of claims 1 to 3,
wherein the fragment comprises more than 20 amino-acids, preferably more than 40 amino-acids, more preferably more than 60 amino-acids, especially more than 100 amino-acids; and
wherein the fragment induces sprouting in a spheroid-based in-vitro angiogenesis assay compared to an uninduced control, preferably at least 25% more, more preferably at least 50% more, especially at least 100% more or even at least 200% more.

5. A fusion protein comprising the protein of any one of claims 1 to 4,
further comprising at least one more amino-acid acid, preferably at least ten more amino-acids, more preferably at least 20 more amino-acids, especially at least 30 more amino-acids; and
wherein the fusion protein induces sprouting in a spheroid-based in-vitro angiogenesis assay compared to an uninduced control, preferably at least 25% more, more preferably at least 50% more, especially at least 100% more or even at least 200% more.

6. The protein of any one of claims 1 to 5, wherein at least one amino-acid residue, the N-terminus and/or the C-terminus is chemically modified by a modification other than glycosylation: especially by pegylation, biotinylation, alkylation, hydroxylation, adenylation, phosphorylation, succinylation, oxidation, or acylation, in particular acetylation.

7. A pharmaceutical composition comprising the protein of any one of claims 1 to 6, further comprising one or more excipients, preferably wherein the protein is conjugated to a biocompatible matrix selected from polysaccharides, in particular sulfated polysaccharides, especially alginate sulfate or hyaluronan sulfate.

8. The protein of any one of claims 1 to 6 or the composition of claim 7, for use in therapy, preferably for use in:
A) treatment of myocardial infarction; or
B) treatment or prevention of ischemia, especially ischemic cardiomyopathy preferably related to myocardial infarction; or
C) treatment or prevention of tissue damage, preferably non-ischemic cardiomyopathy; or
D) therapy, especially according to A), B) or C), wherein the therapy is conducted in combination with administration of endothelial colony forming cells (ECFCs) or other cells that promote vascularisation of tissues.

9. A method for manufacturing the protein of any one of claims 1 to 6, comprising:
- recombinant expression of the protein, preferably in 293 HEK cells; and
- purification of the protein, preferably by affinity purification;
and preferably further comprising cleavage of a signal peptide from the protein and, especially, secretion of the protein from the cells, and/or wherein the protein further comprises a cleavable or non-cleavable affinity tag, especially a FLAG tag, in particular wherein the tag is cleaved from the protein.

10. A CYTL1 protein, or a functional fragment protein thereof or a functional fusion protein thereof, for use in
A) treatment of myocardial infarction; or
B) treatment or prevention of ischemia, especially ischemic cardiomyopathy preferably related to myocardial infarction; or
C) treatment or prevention of tissue damage, preferably non-ischemic cardiomyopathy; or
D) therapy, especially according to A), B) or C), wherein the therapy is conducted in combination with administration of endothelial colony forming cells (ECFCs) or other cells that promote vascularisation of tissues;
and, for each of A) or B) or C) or D), wherein said protein, fragment protein or fusion protein induces sprouting in a spheroid-based in-vitro angiogenesis assay compared to an uninduced control, preferably at least 25% more, more preferably at least 50% more, especially at least 100% more or even at least 200% more;
and preferably, for each of A) or B) or C) or D), wherein the CYTL1 protein is selected from the group of proteins with the sequence of SEQ ID Nos.: 1-53, especially SEQ ID Nos.: 1 and 28, or from the group of proteins with the following UniProt entries: Q9NRR1, G1S206, F7CJ05, UPI00027F32F6, H2QP59, G3QV27, H2PCW0, G7MSJ5, G7P588, S7MTB0, UPI0002C2DFE8, UPI0002BCE03B, G3WH39, UPI00027FA17A, A1E5L0, Q6P536, Q0VG51, B5DFG7, UPI00033447B9, UPI00033193AE, U6D578, G3TD71, UPI00022346AE, H0ZI47, UPI000194C372, UPI00032AE38E, UPI00038EFAD7, F1P7S3, UPI000359D860, UPI0003C488C0, UPI000388B5C7, K7GER1, UPI00042BCFB6, H0WPN0, UPI00027423DC, L5LQ03, UPI000333DDDA, H0VBW2, UPI00022B6D40, UPI000328A678, UPI000328E1D4, UPI00038C4288, W5PBC3, UPI00029D7CC2, E1BN89, L8IQ55, UPI0003AF8B30, UPI0003C10549, UPI00022F3975, G3GWE1, UPI000333186A, M3W6F7, UPI0000F5A458, UPI00042BCEBF, M3Y970, D2HLS4, G1M521, UPI0001DEB975, UPI0002C55126, UPI0003EE2687, UPI000399217F, UPI0003833174, UPI000386FA6F, F6RIT6, G1T5G9, G5B313, UPI0003500720, U3I8P9, R0JVB5, UPI0003509675, UPI0003C259FF, F1NSV3, G1NJB0, UPI0002035275, I3LW41, or from the group of proteins with a sequence identity to the entire sequence of a protein of one of the previous groups of more than 80%, preferably more than 90%, more preferably more than 95%, even more preferably more than 98%.

11. A anti-CYTL1 antibody, or a functional fragment thereof, for use in treatment of cancer, wherein tumour angiogenesis is inhibited, preferably administered in combination with other angiogenesis inhibitors such as Bevacizumab, and wherein said antibody inhibits CYTL1-induced sprouting in a spheroid-based in-vitro angiogenesis assay in the presence of CYTL1, compared to an uninhibited control.

12. A method to screen for CYTL1 competitors, comprising:
- providing a chemical compound library; and
- providing CYTL1, preferably the protein of any one of claims 1 to 6; and
- providing cells, preferably human umbilical vein endothelial cells (HUVECs); and
- individually for each compound of the library: bringing the compound into contact with cells and CYTL1 ("a sample"); and
- performing a spheroid-based in-vitro angiogenesis assay for each sample and compare it to a negative control, preferably the cells in the presence of CYTL1 without any of the compounds; and
- identifying as positive hit the compound of a sample, which sample has reduced sprouting compared to the negative control, preferably more than 10% reduction, more preferably more than 25% reduction, especially more than 50% reduction.

13. A method to identify endothelial colony-forming cells (ECFCs), comprising:
- providing or isolating one or more cells; and
- measuring levels of one or more selected from the group of
CYTL1 mRNA inside the cells,
CYTL1 protein inside the cells,
secreted CYTL1 protein; and
- comparing the measured levels to levels of a control cell population; and
- identifying the cells as ECFCs if the measured levels are higher than the control levels.

14. The method of claim 13, wherein the CYTL1 mRNA codes for a protein sequence or the CYTL1 protein comprises a protein sequence identical to at least 20, preferably at least 50, more preferably at least 100, especially all, of the amino-acids of the entire sequence of an entry selected from the group of the following UniProt entries:
Q9NRR1, G1S206, F7CJ05, UPI00027F32F6, H2QP59, G3QV27, H2PCW0, G7MSJ5, G7P588, S7MTB0, UPI0002C2DFE8, UPI0002BCE03B, G3WH39, UPI00027FA17A, A1E5L0, Q6P536, Q0VG51, B5DFG7, UPI00033447B9, UPI00033193AE, U6D578, G3TD71, UPI00022346AE, H0ZI47, UPI000194C372, UPI00032AE38E, UPI00038EFAD7, F1P7S3, UPI000359D860, UPI0003C488C0, UPI000388B5C7, K7GER1, UPI00042BCFB6, H0WPN0, UPI00027423DC, L5LQ03, UPI000333DDDA, H0VBW2, UPI00022B6D40, UPI000328A678, UPI000328E1D4, UPI00038C4288, W5PBC3, UPI00029D7CC2, E1BN89, L8IQ55, UPI0003AF8B30, UPI0003C10549, UPI00022F3975, G3GWE1, UPI000333186A, M3W6F7, UPI0000F5A458, UPI00042BCEBF, M3Y970, D2HLS4, G1M521, UPI0001DEB975, UPI0002C55126, UPI0003EE2687, UPI000399217F, UPI0003833174, UPI000386FA6F, F6RIT6, G1T5G9, G5B313, UPI0003500720, U3I8P9, R0JVB5, UPI0003509675, UPI0003C259FF, F1NSV3, G1NJB0, UPI0002035275, I3LW41.

15. A method of purifying or enriching for endothelial colony-forming cells (ECFCs), comprising:
- introducing into cells a reporter gene construct operatively linked to the endogenous CYTL1 gene, preferably by replacing the endogenous CYTL1 gene by a CYTL1-reporter gene fusion under the endogenous CYTL1 promoter or by having a reporter gene under the control of the endogenous CYTL1 promoter; and
- providing or isolating the cells; and
- purifying or enriching for the cells having reporter gene construct expression or an increased reporter gene construct expression level compared to a control level.
